# EUROPEAN PATENT APPLICATION

(11) **EP 2 474 540 A1**
(43) Date of publication of application: **11.07.2012**
(21) Application number: 10812052.8
(22) Date of filing: 30.08.2010
(51) Int. Cl.: C07D 401/14, A61K 31/4545, A61P 3/10, A61P 43/00, C07D 405/14, C07D 409/14, C07D 413/14, C07D 471/04

(54) **GPR119 AGONIST**

(30) Priority: 31.08.2009 JP 2009200626; 01.02.2010 JP 2010019857
(71) Applicant: Nippon Chemiphar Co., Ltd., Tokyo 101-0032 (JP)
(72) Inventor: ENDO, Tsuyoshi, Misato-shi Saitama 341-0005 (JP); TANAKA, Hiroto, Misato-shi Saitama 341-0005 (JP); TAKAHASHI, Toshihiro, Misato-shi Saitama 341-0005 (JP); KUNIGAMI, Toshihiro, Misato-shi Saitama 341-0005 (JP)
(74) Representative: Hiebl, Inge Elisabeth
(86) International application number: PCT/JP2010/064730
(87) International publication number: WO 2011/025006

(57) **Abstract**

A compound represented by the formula (II) is a GPR119 agonist, and is used as an agent for treating diabetes: 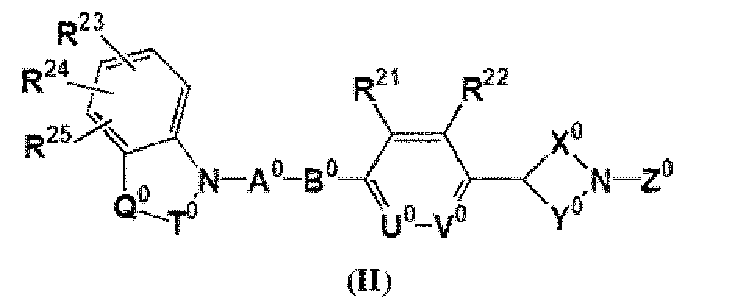 wherein each of R²³, R²⁴, and R²⁵ is hydrogen, halogen, C₁₋₈ alkyl, C₁₋₈ alkoxy, C₁₋₈ alkylsulfonyl, or the like;
each of Q⁰ and T⁰ is CH₂ or the like, or Q⁰ and T⁰ are combined to form CH=CH or the like; A⁰ is (CH₂)p, C(O), or a bond;
B⁰ is a bond or the like;
one of U⁰ and V⁰ is N, and the other is CR³¹ or the like;
each of X⁰ and Y⁰ is CH₂CH₂ or the like;
Z⁰ is C(O)OR³² or the like; and
each of R²¹ and R²² is hydrogen, a halogen atom, hydroxyl, C₁₋₈ alkyl, or the like.

## Description

### Field of the invention

The present invention relates to a GPR119 agonist.

### Background of the invention

Diabetes is a life-style related disease and the number of patients increases all over the world. The treatments for diabetes are classified into diet, exercise and drug therapy (injectable insulin and an oral anti-diabetic drug). Some oral anti-diabetic drugs, for example, α-glucosidase inhibitors (acarbose, voglibose), insulin-sensitizing agents (pioglitazone hydrochloride), biguanides (metformin hydrochloride), sulfonylureas (glibenclamide, glimepiride), and short-acting insulin secretagogues (mitiglinide calcium hydrate) are commercially available.

Recently, an incretin mimetics (excenatide) and a DPP IV inhibitor (sitagliptin), which accelerates secretion of insulin, are also commercially available. The incretin is a gastrointestinal hormone, which accelerates secretion of insulin. Further, SGLT inhibitors have been developed abroad.

GPR119 has been reported as a G protein-coupled-receptor (GPCR) whose endogenous ligand is N-oleoylethanolamide and which stimulate insulin secretion from pancreatic β-cells (Non-patent Document 1). It has been reported that GPR119 agonist increases the plasma concentration of Glucagon like peptide-1 (GLP-1), one of incretins (Non-patent Document 2), which may indirectly relate to stimulation of insulin secretion. It has been further reported that GPR119 agonist suppresses a weight increase in rats fed a high-fat diet (Non-patent Document 1), which may relate to energy metabolism. For the reasons mentioned above, the GPR119 agonist has been expected as a drug not only for diabetes but also for life-style related diseases such as obesity and metabolic syndrome.

Compounds such as (A) are described in Patent Document 1 as the GPR119 agonist.

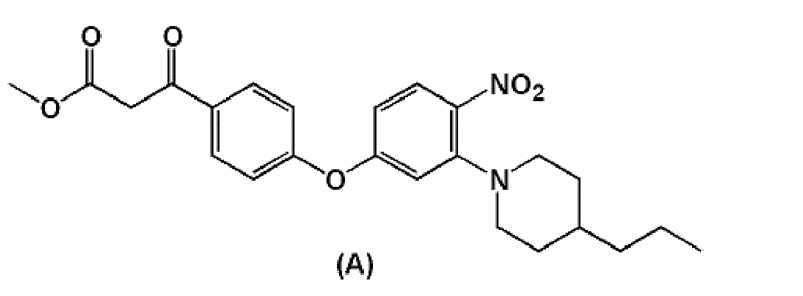

Compounds such as (B) are described in Patent Document 2 as the GPR119 agonist.

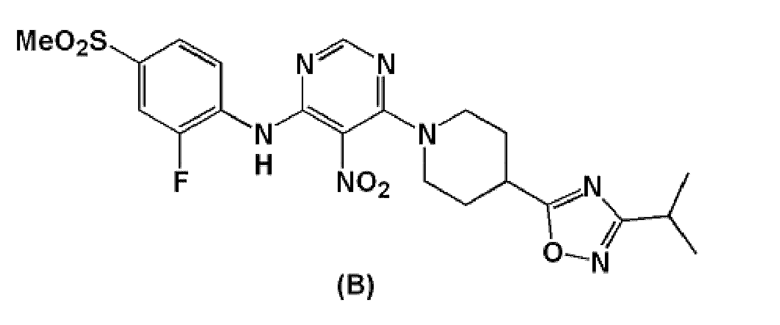

Compounds such as (C) are described in Patent Document 3 as the GPR119 agonist.

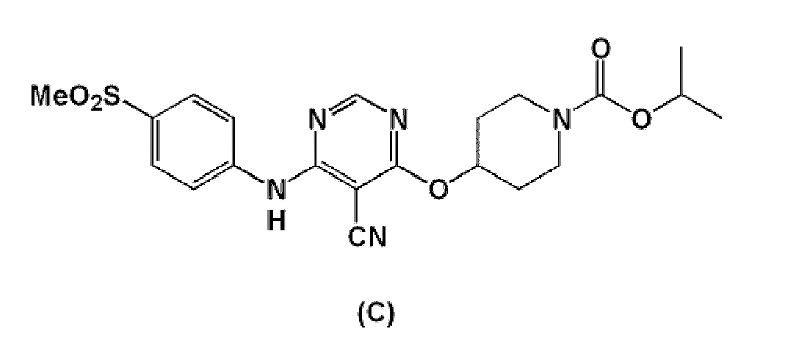

Compounds such as (D) are described in Patent Document 4 as the GPR119 agonist.

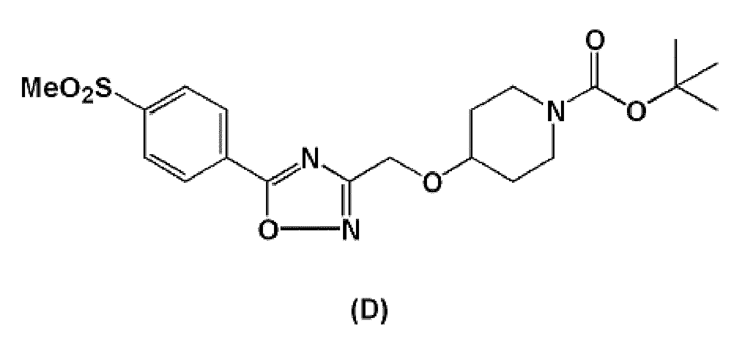

Compounds such as (E) are described in Patent Document 5 as the GPR119 agonist.

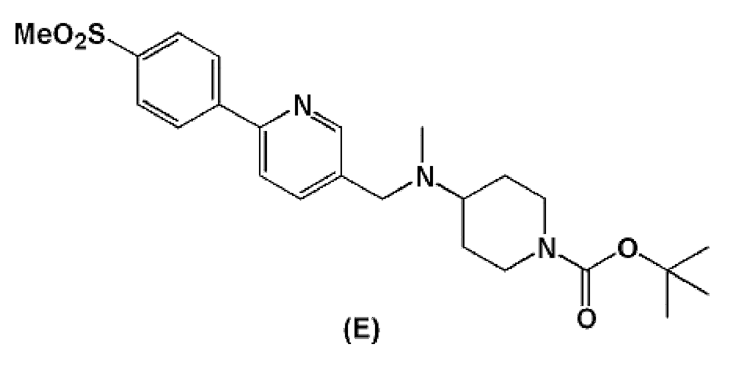

Compounds such as (F) are described in Patent Document 6 as the GPR119 agonist.

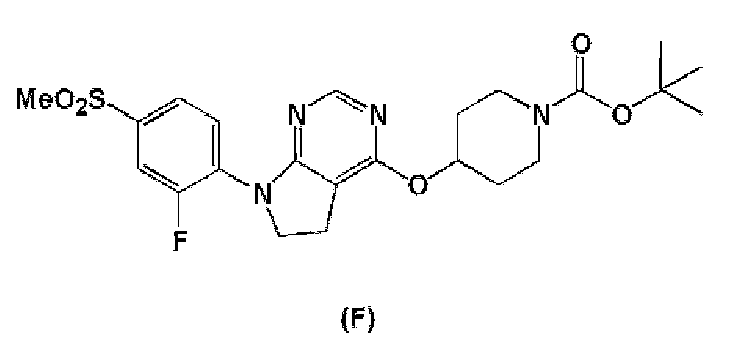

The compounds of the present invention represented by the below-described formulas (I), (II), and (III) are different from the compounds (A) to (F) because the carbon atom of a cyclic amine such as a pipreridine ring is directly combined with a pyridyl group or the like in the compounds of the present invention. There are other differences that Ar in the below-described formula (I) is a bicyclic heterocyclic ring, and corresponds to indole or the like in the below-described formula (II) or (III).

The present inventors have studied the GPR119 agonist, and filed Patent Document 7. Further, Patent Document 8 has recently been published.

The compounds disclosed in the Patent Documents 7 and 8 are different from the compounds of the present invention represented by the below-described formula (I), (II), or (III) because the heterocyclic ring of Ar is monocyclic in the former compounds, while it is bicyclic in the latter compounds.

Compound (G) represented by the following formula is described in Patent Document 9.

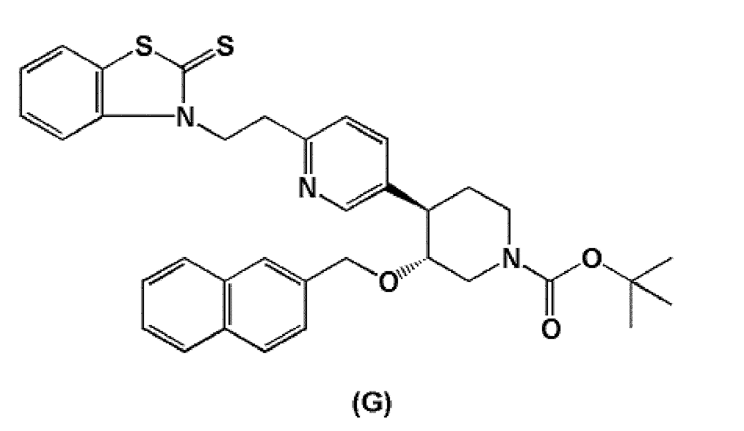

The Patent Document 9 uses the compound (G) as an intermediate in preparation of an agent for treating Alzheimer's disease. There is no description in the Document that the compound has a function of a GPR119 agonist.

### Prior art documents

### Patent Documents

Patent Document 1: WO 2004/076413
Patent Document 2: WO 2004/065380
Patent Document 3: WO 2005/007647
Patent Document 4: WO 2007/003960
Patent Document 5: WO 2008/025798
Patent Document 6: WO 2008/008887
Patent Document 7: WO 2010/013849
Patent Document 8: WO 2010/008739
Patent Document 9: WO 2002/076440

### Non-patent Documents

Non-patent Document 1: Overton HA et al., Cell Metab., 2006, 3, 167-75
Non-patent Document 2: Chu ZL et al., Endocrinology, 2008, 149, 2038-47

### Summary of the invention

### Problems to be solved by the invention

The object of the invention is to provide a compound represented by the formula (I), (II), or (III), or a pharmaceutically acceptable salt thereof, and an agent for treating diabetes containing it as an active ingredient.

### Means for solving the problems

The present invention relates to a compound having the following formula (I) or a pharmaceutically acceptable salt thereof: 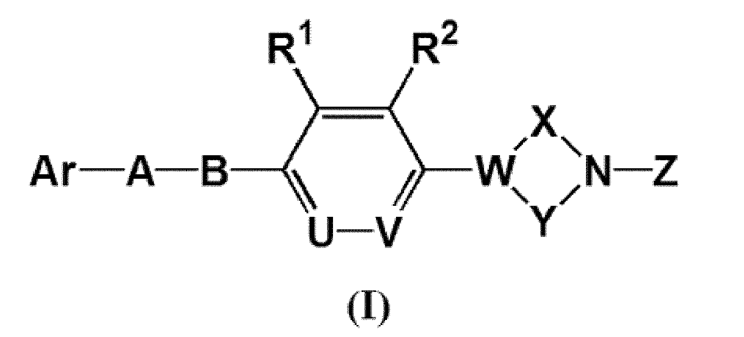 wherein Ar is a bicyclic heterocyclic ring comprising a benzene or pyridine ring condensed with a five-membered or six-membered heterocyclic ring, which optionally has a substituent or substituents selected from the group consisting of a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group having a C₁₋₈ alkoxy group, carboxyl, carbamoyl, an acyl group having a C₁₋₈ alkyl group, an alkylaminocarbonyl group having a C₁₋₈ alkyl group, a dialkylaminocarbonyl group having C₂₋₁₂ alkyl groups, an alkoxycarbonylmethylcarbonyl group having a C₁₋₈ alkoxy group, an alkylsulfonylmethyl group having a C₁₋₈ alkyl group, amino, a C₁₋₈ alkylamino group, a C₂₋₁₂ dialkylamino group, a C₁₋₈ alkylsulfonylamino group, an acylamino group having a C₁₋₈ alkyl group, a C₁₋₈ alkylsulfinyl group, a C₁₋₈ alkylsulfonyl group, sulfamoyl, a C₁₋₈ alkylaminosulfonyl group, a C₂₋₁₂ dialkylaminosulfonyl group, phenylsulfonyl, and a five-membered or six-membered heteroaryl group;
A is (CH₂)ₘ, C(O), S, O, NR³, or a bond, wherein m is an integer of 1 to 3, and R³ is hydrogen or a C₁₋₈ alkyl group;
B is (C(R⁴)H)ₙ, S, O, CH=CH, NR⁵, or a bond, wherein n is an integer of 1 to 3, and each of R⁴ and R⁵ independently is hydrogen or a C₁₋₈ alkyl group, provided that B is neither S, O, nor NR⁵ when A is S, O, or NR³;
one of U and V is N and the other is CR⁶, or each of U and V is N, wherein R⁶ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
W is C or CR⁷, wherein R⁷ is hydrogen, a halogen atom, hydroxyl, a C₁₋₈ alkyl, a C₁₋₈ alkoxy group, a C₁₋₈ alkyl group having one to three halogen atoms, or a C₁₋₈ alkoxy group having one to three halogen atoms;
X is a C₁₋₃ alkylene group, which optionally has a substituent or substituents selected from the group consisting of a halogen atom, hydroxyl, a C₁₋₈ alkyl group, a C₁₋₈ alkoxy group, a C₁₋ₛ alkyl group having one to three halogen atoms, and a C₁₋₈ alkoxy group having one to three halogen atoms;
when W is C, X may combine to W with a double bond;
Y is a C₁₋₃ alkylene group, which optionally has a substituent or substituents selected from the group consisting of a halogen atom, hydroxyl, a C₁-₈ alkyl group, a C₁₋₈ alkoxy group, a C₁₋₈ alkyl group having one to three halogen atoms, and a C₁₋₈ alkoxy group having one to three halogen atoms;
Z is C(O)OR⁸, C(O)R⁹, S0₂R¹⁰, C(O)NR¹¹R¹², CH₂C(O)N(R¹³) (R¹⁴), or a five-membered or six-membered heteroaryl group comprising carbon and nitrogen atoms, said carbon atom combining to the nitrogen atom of the neighboring cγyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R⁸, R⁹, R¹⁰, _{R}¹¹, R¹², R¹³, and R¹⁴ independently is a C₁₋₈ alkyl group, a C₂₋₈ alkenyl group, a C₃₋₈ cycloalkyl group, phenyl, or a C₁₋₈ alkyl group having phenyl; and
each of R¹ and R² independently is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms.

The invention also relates to a compound having the following formula (II) or a pharmaceutically acceptable salt thereof: 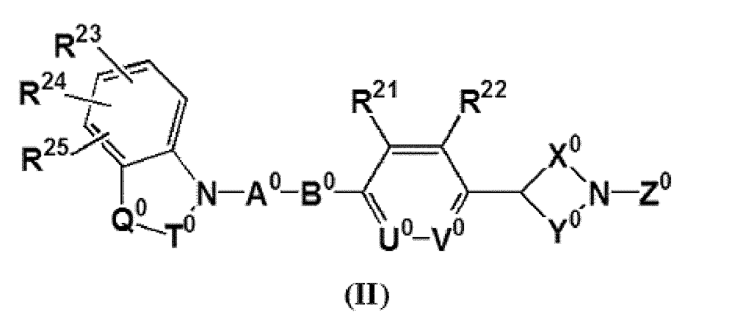 wherein each of R²³, R²⁴, and R²⁵ independently is hydrogen, a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group having a C₁₋₈ alkoxy group, carboxyl, carbamoyl, an acyl group having a C₁₋₈ alkyl group, an alkylaminocarbonyl group having a C₁₋₈ alkyl group, a dialkylaminocarbonyl group having C₂₋₁₂ alkyl groups, an alkoxycarbonylmethylcarbonyl group having a C₁₋₈ alkoxy group, an alkylsulfonylmethyl group having a C₁₋₈ alkyl group, amino, a C₁₋₈ alkylamino group, a C₂₋₁₂ dialkylamino group, a C₁₋₈ alkylsulfonylamino group, an acylamino group having a C₁₋₈ alkyl group, a C₁₋₈ alkylsulfinyl group, a C₁₋₈ alkylsulfonyl group, sulfamoyl, a C₁₋₈ alkylaminosulfonyl group, a C₂₋₁₂ dialkylaminosulfonyl group, phenylsulfonyl, or a five-membered or six-membered heteroaryl group;
each of Q⁰ and T⁰ independently is C(R²⁶) (R²⁷), or Q⁰ and T⁰ are combined to form CR²⁸=CR²⁹ or CR³⁰=N, wherein each of R²⁶ and R²⁷ independently is hydrogen or a C₁₋₈ alkyl group, each of R²⁸ and R²⁹ independently is hydrogen or a C₁₋₈ alkyl group, and R³⁰ is hydrogen or a C₁₋₈ alkyl group;
A° is (CH₂)ₚ, C(O), or a bond, wherein p is an integer of 1 to 3;
B° is (C(R³¹)H)_{q}, S, O, CH=CH, NR³², or a bond, wherein q is an integer of 1 to 3, and each of R³¹ and R³² is hydrogen or a C₁₋₈ alkyl group, provided that B⁰ is neither S, O, nor NR³² when A⁰ is CH₂ or a bond;
one of U⁰ and V⁰ is N and the other is CR³³, or each of U⁰ and V⁰ is N, wherein R³³ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
each of X⁰ and Y⁰ independently is a C₁₋₃ alkylene group, which optionally has a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms;
Z⁰ is C(O)OR³⁴, C(O)R³⁵, SO₂R³⁶, C(O)NR³⁷R³⁸, CH₂C(O)N(R³⁹) (R⁴⁰), or a five-membered or six-membered heteroaryl group comprising carbon and nitrogen atoms, said carbon atom combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R³⁴, R³⁵, R³⁶, R³⁷, R³⁸, R³⁹, and R⁴⁰ independently is a C₁₋₈ alkyl group, a C₂₋₈ alkenyl group, a C₃₋₈ cycloalkyl group, phenyl, or a C₁₋₈ alkyl group having phenyl; and
each of R²¹ and R²² independently is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms.

The invention also relates to a compound having the following formula (III) or a pharmaceutically acceptable salt thereof: 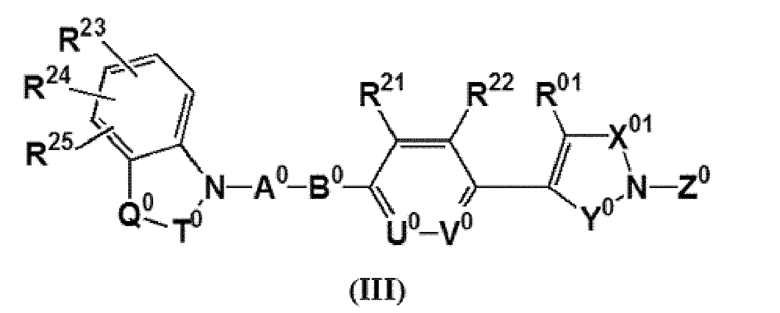 wherein each of R²³, R²⁴, and R²⁵ independently is hydrogen, a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group having a C₁₋₈ alkoxy group, carboxyl, carbamoyl, an acyl group having a C₁₋₈ alkyl group, an alkylaminocarbonyl group having a C₁₋₈ alkyl group, a dialkylaminocarbonyl group having C₂₋₁₂ alkyl groups, an alkoxycarbonylmethylcarbonyl group having a C₁₋₈ alkoxy group, an alkylsulfonylmethyl group having a C₁₋₈ alkyl group, amino, a C₁₋₈ alkylamino group, a C₂₋₁₂ dialkylamino group, a C₁₋₈ alkylsulfonylamino group, an acylamino group having a C₁₋₈ alkyl group, a C₁₋₈ alkylsulfinyl group, a C₁₋₈ alkylsulfonyl group, sulfamoyl, a C₁₋₈ alkylaminosulfonyl group, a C₂₋₁₂ dialkylaminosulfonyl group, phenylsulfonyl, or a five-membered or six-membered heteroaryl group;
each of Q⁰ and T⁰ independently is C(R²⁶)(R²⁷), or Q⁰ and T⁰ are combined to form CR²⁸=CR²⁹ or CR³⁰=N, wherein each of R²⁶ and R²⁷ independently is hydrogen or a C₁₋₈ alkyl group, each of R²⁸ and R²⁹ independently is hydrogen or a C₁₋₈ alkyl group, and R³⁰ is hydrogen or a C₁₋₈ alkyl group;
A⁰ is (CH₂)ₚ, C(O), or a bond, wherein p is an integer of 1 to 3;
B⁰ is (C(R³¹)H)_{q}, S, O, CH=CH, NR³², or a bond, wherein q is an integer of 1 to 3, and each of R³¹ and R³² is hydrogen or a C₁₋₈ alkyl group, provided that B⁰ is neither S, O, nor NR³² when A⁰ is CH₂ or a bond;
one of U⁰ and V⁰ is N and the other is CR³³, or each of U⁰ and V⁰ is N, wherein R³³ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
X⁰¹ is methylene or ethylene, which optionally has a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms;
R⁰¹ is hydrogen, a C₁₋₈ alkyl group, or a C₁₋₈ alkyl group having one to three halogen atoms;
Y⁰ is a C₁₋₃ alkylene group, which optionally has a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms;
Z⁰ is C(O)OR³⁴, C(O)R³⁵, SO₂R³⁶, C(O)NR³⁷R³⁸, CH₂C(O)N(R³⁹) (R⁴⁰), or a five-membered or six-membered heteroaryl group comprising carbon and nitrogen atoms, said carbon atom combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R³⁴, R³⁵, R³⁶, R37, R³⁸, R³⁹, and R⁴⁰ independently is a C₁₋₈ alkyl group, a C₂₋₈ alkenyl group, a C₃₋₈ cycloalyl group, phenyl, or a C₁₋₈ alkyl group having phenyl; and
each of R²¹ and R²² independently is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms.

The invention further relates to an agent for treating diabetes containing the compound of the formula (I), (II), or (III) described above, or a pharmaceutically acceptable salt thereof as an active ingredient.

The invention further relates to a GPR119 agonist containing the compound of the formula (I), (II), or (III) described above, or a pharmaceutically acceptable salt thereof as an active ingredient.

### Embodiments for conducting the invention

Preferred embodiments of the compound of the formula (I) are described below.
(1) A compound having the above-mentioned formula (I) or a pharmaceutically acceptable salt thereof, wherein Ar is indole, indoline, indazole, pyrrolopyridine, benzofuran, benzothiophene, benzo[b]thiophene-1,1-dioxide, or tetrahydroquinoline, which optionally has a substituent or substituents selected from the group consisting of a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group having a C₁₋₈ alkoxy group, carboxyl, carbamoyl, an acyl group having a C₁₋₈ alkyl group, an alkylaminocarbonyl group having a C₁₋₈ alkyl group, a dialkylaminocarbonyl group having C₂₋₁₂ alkyl groups, an alkoxycarbonylmethylcarbonyl group having a C₁₋₈ alkoxy group, an alkylsulfonylmethyl group having a C₁₋₈ alkyl group, amino, a C₁₋₈ alkylamino group, a C₂₋₁₂ dialkylamino group, a C₁₋₈ alkylsulfonylamino group, an acylamino group having a C₁₋₈ alkyl group, a C₁₋₈ alkylsulfinyl group, a C₁₋₈ alkylsulfonyl group, sulfamoyl, a C₁₋₈ alkylaminosulfonyl group, a C₂₋₁₂ dialkylaminosulfonyl group, phenylsulfonyl, and a five-membered or six-membered heteroaryl group.
(2) A compound having the above-mentioned formula (I) or a pharmaceutically acceptable salt thereof, wherein Ar is indole, indoline, indazole, pyrrolopyridine, benzofuran, benzothiophene, benzo[b]thiophene-1,1-dioxide, or tetrahydroquinoline, which has one substituent selected from the group consisting of cyano, an alkoxycarbonyl group having a C₁₋₈ alkoxy group, a C₁₋₈ alkylsulfonyl group, sulfamoyl, phenylsulfonyl, and a five-membered or six-membered heteroaryl group.
(3) A compound having the above-mentioned formula (I) or a pharmaceutically acceptable salt thereof, wherein Ar is indole, indoline, indazole, pyrrolopyridine, benzofuran, benzothiophene, benzo[b]thiophene-1,1-dioxide, or tetrahydroquinoline having a substituent or substituents, said substituent being a C₁₋₈ alkylsulfonyl group.
(4) A compound having the above-mentioned formula (I) or a pharmaceutically acceptable salt thereof, wherein Ar is indole, indoline, indazole, pyrrolopyridine, benzofuran, benzothiophene, benzo[b]thiophene-1,1-dioxide, or tetrahydroquinoline having two substituents, one of said substituents being a C₁₋₈ alkylsulfonyl group, and the other being a C₁₋₈ alkyl group or a halogen atom.
(5) A compound having the above-mentioned formula (I) or a pharmaceutically acceptable salt thereof, wherein Ar is indole, indoline, indazole, pyrrolopyridine, benzofuran, benzothiophene, benzo[b]thiophene-1,1-dioxide, or tetrahydroquinoline having a substituent or substituents, said substituent being 1-tetrazolyl.
(6) A compound having the above-mentioned formula (I) or a pharmaceutically acceptable salt thereof, wherein Ar is indole, indoline, indazole, pyrrolopyridine, benzofuran, benzothiophene, benzo[b]thiophene-1,1-dioxide, or tetrahydroquinoline having two substituents, one of said substituents being 1-tetrazolyl, and the other being a C₁₋₈ alkyl group or a halogen atom.
(7) A compound having the above-mentioned formula (I) or described in one of (1) to (6) or a pharmaceutically acceptable salt thereof, wherein A is CH₂, and B is a bond.
(8) A compound having the above-mentioned formula (I) or described in one of (1) to (6) or a pharmaceutically acceptable salt thereof, wherein A is O, and B is CH₂.
(9) A compound having the above-mentioned formula (I) or described in one of (1) to (8) or a pharmaceutically acceptable salt thereof, wherein one of U and V is N, and the other is CH.
(10) A compound having the above-mentioned formula (I) or described in one of (1) to (8) or a pharmaceutically acceptable salt thereof, wherein U is N, and V is CH.
(11) A compound having the above-mentioned formula (I) or described in one of (1) to (10) or a pharmaceutically acceptable salt thereof, wherein each of X and Y is ethylene.
(12) A compound having the above-mentioned formula (I) or described in one of (1) to (11) or a pharmaceutically acceptable salt thereof, wherein Z is C(O)OR⁸.
(13) A compound described in (12) or a pharmaceutically acceptable salt thereof, wherein R⁸ is a C₁₋₈ alkyl group.
(14) A compound described in (12) or a pharmaceutically acceptable salt thereof, wherein R⁸ is a C₃₋₅ alkyl group.
(15) A compound having the above-mentioned formula (I) or described in one of (1) to (11) or a pharmaceutically acceptable salt thereof, wherein Z is 3-C₁₋₈ alkyl-1,2,4-oxadiazol-5-yl or 5-C₁₋₈ alkyl-1,2,4-oxadiazol-3-yl.
(16) A compound having the above-mentioned formula (I) or described in one of (1) to (11) or a pharmaceutically acceptable salt thereof, wherein Z is 5-C₁₋₈ alkylpyrimidin-2-yl.
(17) A compound having the above-mentioned formula (I) or described in one of (1) to (16) or a pharmaceutically acceptable salt thereof, wherein each of R¹ and R² is hydrogen.
(18) A compound having the above-mentioned formula (I) or described in one of (1) to (16) or a pharmaceutically acceptable salt thereof, wherein one of R¹ and R² is a C₁₋₈ alkyl group, and the other is hydrogen.
(19) A compound having the above-mentioned formula (I) or described in one of (1) to (16) or a pharmaceutically acceptable salt thereof, wherein one of R¹ and R² is a halogen atom, and the other is hydrogen.

Preferred embodiments of the compound of the formula (II) are described below.
(20) A compound having the above-mentioned formula (II) or a pharmaceutically acceptable salt thereof, wherein each of R²³, R²⁴, and R²⁵ independently is hydrogen, a halogen atom, cyano, a C₁₋₈ alkyl group, an alkoxycarbonyl group having a C₁₋₈ alkoxy group, a C₁₋₈ alkylsulfonyl group, sulfamoyl, phenylsulfonyl, or a five-membered or six-membered heteroaryl group.
(21) A compound having the above-mentioned formula (II) or a pharmaceutically acceptable salt thereof, wherein one of R²³, R²⁴, and R²⁵ is a C₁₋₈ alkylsulfonyl group.
(22) A compound having the above-mentioned formula (II) or a pharmaceutically acceptable salt thereof, wherein one of R²³, R²⁴, and R²⁵ is a C₁₋₈ alkylsulfonyl group, and another is a C₁₋₈ alkyl group or a halogen atom.
(23) A compound having the above-mentioned formula (II) or a pharmaceutically acceptable salt thereof, wherein one of R²³, R²⁴, and R²⁵ is 1-tetrazolyl.
(24) A compound having the above-mentioned formula (II) or a pharmaceutically acceptable salt thereof, wherein one of R²³, R²⁴, and R²⁵ is 1-tetrazolyl, and another is a C₁₋₈ alkyl group or a halogen atom.
(25) A compound having the above-mentioned formula (II) or described in one of (20) to (24) or a pharmaceutically acceptable salt thereof, wherein Q⁰ and T° are combined to form CH=CH.
(26) A compound having the above-mentioned formula (II) or described in one of (20) to (24) or a pharmaceutically acceptable salt thereof, wherein each of Q⁰ and T⁰ is CH₂.
(27) A compound having the above-mentioned formula (II) or described in one of (20) to (26) or a pharmaceutically acceptable salt thereof, wherein A⁰ is CH₂, and B⁰ is a bond.
(28) A compound having the above-mentioned formula (II) or described in one of (20) to (27) or a pharmaceutically acceptable salt thereof, wherein one of U⁰ and V⁰ is N, and the other is CH.
(29) A compound having the above-mentioned formula (II) or described in one of (20) to (27) or a pharmaceutically acceptable salt thereof, wherein U⁰ is N, and V⁰ is CH.
(30) A compound having the above-mentioned formula (II) or described in one of (20) to (29) or a pharmaceutically acceptable salt thereof, wherein each of X⁰ and Y⁰ is ethylene.
(31) A compound having the above-mentioned formula (II) or described in one of (20) to (30) or a pharmaceutically acceptable salt thereof, wherein Z⁰ is C(O)OR³⁴.
(32) A compound described in (31) or a pharmaceutically acceptable salt thereof, wherein R³⁴ is a C₁₋₈ alkyl group.
(33) A compound described in (31) or a pharmaceutically acceptable salt thereof, wherein R³⁴ is a C₃₋₅ alkyl group.
(34) A compound having the above-mentioned formula (II) or described in one of (20) to (30) or a pharmaceutically acceptable salt thereof, wherein Z⁰ is 3-C₁₋₈ alkyl-1,2,4-oxadiazol-5-yl or 5-C₁₋₈ alkyl-1,2,4-oxadiazol-3-yl.
(35) A compound having the above-mentioned formula (II) or described in one of (20) to (30) or a pharmaceutically acceptable salt thereof, wherein Z⁰ is 5-C₁₋₈ alkylpyrimidin-2-yl.
(36) A compound having the above-mentioned formula (II) or described in one of (20) to (35) or a pharmaceutically acceptable salt thereof, wherein each of R²¹ and R²² is hydrogen.
(37) A compound having the above-mentioned formula (II) or described in one of (20) to (35) or a pharmaceutically acceptable salt thereof, wherein one of R²¹ and R²² is a C₁₋₈ alkyl group, and the other is hydrogen.
(38) A compound having the above-mentioned formula (II) or described in one of (20) to (35) or a pharmaceutically acceptable salt thereof, wherein one of R²¹ and R²² is a halogen atom, and the other is hydrogen.

Preferred embodiments of the compound of the formula (III) are described below.
(39) A compound having the above-mentioned formula (III) or a pharmaceutically acceptable salt thereof, wherein each of R²³, R²⁴, and R²⁵ independently is hydrogen, a halogen atom, cyano, a C₁₋₈ alkyl group, an alkoxycarbonyl group having a C₁₋₈ alkoxy group, a C₁₋₈ alkylsulfonyl group, sulfamoyl, phenylsulfonyl, or a five-membered or six-membered heteroaryl group.
(40) A compound having the above-mentioned formula (III) or a pharmaceutically acceptable salt thereof, wherein one of R²³, R²⁴, and R²⁵ is a C₁₋₈ alkylsulfonyl group.
(41) A compound having the above-mentioned formula (III) or a pharmaceutically acceptable salt thereof, wherein one of R²³, R²⁴, and R²⁵ is a C₁₋₈ alkylsulfonyl group, and another is a C₁₋₈ alkyl group or a halogen atom.
(42) A compound having the above-mentioned formula (III) or a pharmaceutically acceptable salt thereof, wherein one of R²³, R²⁴, and R²⁵ is 1-tetrazolyl or 1,2,4-triazol-1-yl.
(43) A compound having the above-mentioned formula (III) or a pharmaceutically acceptable salt thereof, wherein one of R²³, R²⁴, and R²⁵ is 1-tetrazolyl or 1,2,4-triazol-1-yl, and another is a C₁₋₈ alkyl group or a halogen atom.
(44) A compound having the above-mentioned formula (III) or described in one of (39) to (43) or a pharmaceutically acceptable salt thereof, wherein Q⁰ and T⁰ are combined to form CH=CH.
(45) A compound having the above-mentioned formula (III) or described in one of (39) to (43) or a pharmaceutically acceptable salt thereof, wherein each of Q⁰ and T⁰ is CH₂.
(46) A compound having the above-mentioned formula (III) or described in one of (39) to (45) or a pharmaceutically acceptable salt thereof, wherein A⁰ is CH₂, and B⁰ is a bond.
(47) A compound having the above-mentioned formula (III) or described in one of (39) to (46) or a pharmaceutically acceptable salt thereof, wherein one of U⁰ and V⁰ is N, and the other is CH.
(48) A compound having the above-mentioned formula (III) or described in one of (39) to (46) or a pharmaceutically acceptable salt thereof, wherein U⁰ is N, and V⁰ is CH.
(49) A compound having the above-mentioned formula (III) or described in one of (39) to (48) or a pharmaceutically acceptable salt thereof, wherein the heterocyclic ring comprising X⁰¹, N, Y⁰, and C=C is 3,6-dihydro-2H-pyridine.
(50) A compound having the above-mentioned formula (III) or described in one of (39) to (49) or a pharmaceutically acceptable salt thereof, wherein Z⁰ is C(O)OR³⁴.
(51) A compound described in (50) or a pharmaceutically acceptable salt thereof, wherein R³⁴ is a C₁₋₈ alkyl group.
(52) A compound described in (50) or a pharmaceutically acceptable salt thereof, wherein R³⁴ is a C₃₋₅ alkyl group.
(53) A compound having the above-mentioned formula (III) or described in one of (39) to (49) or a pharmaceutically acceptable salt thereof, wherein Z⁰ is 3-C₁₋₈ alkyl-1,2,4-oxadiazol-5-yl or 5-C₁₋₈ alkyl-1,2,4-oxadiazol-3-yl.
(54) A compound having the above-mentioned formula (III) or described in one of (39) to (49) or a pharmaceutically acceptable salt thereof, wherein Z⁰ is 5-C₁₋₈ alkylpyrimidin-2-yl.
(55) A compound having the above-mentioned formula (III) or described in one of (39) to (54) or a pharmaceutically acceptable salt thereof, wherein each of R²¹ and R²² is hydrogen.
(56) A compound having the above-mentioned formula (III) or described in one of (39) to (54) or a pharmaceutically acceptable salt thereof, wherein one of R²¹ and R²² is a C₁₋₈ alkyl group, and the other is hydrogen.
(57) A compound having the above-mentioned formula (III) or described in one of (39) to (54) or a pharmaceutically acceptable salt thereof, wherein one of R²¹ and R²² is a halogen atom, and the other is hydrogen.

In the compound of the above-mentioned formula (I), (II), or (III), examples of the halogen atoms include a fluorine atom, a chlorine atom, and a bromine atom. Examples of the C₁₋₈ alkyl groups include methyl, ethyl, propyl, isopropyl, butyl, t-butyl, pentyl, neopentyl, and hexyl.

Examples of the C₃₋₈ cycloalkyl groups include cyclopropyl, cyclopentyl, and cyclohexyl.

Examples of the C₁₋₈ alkoxy groups include methoxy, ethoxy, and propoxy.

Examples of the C₁₋₈ alkyl groups having one to three halogen atoms include chloromethyl, fluoromethyl, 2-fluoroethyl, and trifluoromethyl. Examples of the C₁₋₈ alkoxy groups having one to three halogen atoms include fluoromethoxy and trifluoromethoxy.

Examples of the alkoxycarbonyl groups having a C₁₋₈ alkoxy group include methoxycarbonyl and ethoxycarbonyl. Examples of the acyl groups having a C₁₋₈ alkyl group include acetyl. Examples of the alkylaminocarbonyl groups having a C₁₋₈ alkyl group include methylaminocarbonyl and ethylaminocarbonyl. Examples of the dialkylaminocarbonyl groups having C₂₋₁₂ alkyl groups include dimethylaminocarbonyl and diethylaminocarbonyl. Examples of the alkoxycarbonylmethylcarbonyl groups having a C₁₋₈ alkoxy group include methoxycarbonylmethylcarbonyl and ethoxycarbonylmethylcarbonyl.

Examples of the alkylsulfonylmethyl groups having a C₁₋₈ alkyl group include methanesulfonylmethyl and ethanesulfonylmethyl. Examples of the C₁₋₈ alkylamino groups include methylamino and ethylamino. Examples of the C₂₋₁₂ dialkylamino groups include dimethylamino and diethylamino. Examples of the C₁₋₈ alkylsulfonylamino groups include methanesulfonylamino and ethanesulfonylamino. Examples of the acylamino groups having a C₁₋₈ alkyl group include acetylamino.

Examples of the C₁₋₈ alkylsulfinyl groups include methylsulfinyl and ethylsulfinyl. Examples of the C₁₋₈ alkylsulfonyl groups include methanesulfonyl and ethanesulfonyl. Examples of the C₁₋₈ alkylaminosulfonyl groups include methylaminosulfonyl and ethylaminosulfonyl. Examples of the C₂₋₁₂ dialkylaminosulfonyl groups include dimethylaminosulfonyl and diethylaminosulfonyl.

Examples of the C₁₋₈ alkyl groups having phenyl include benzyl.

Examples of the C₂₋₈ alkenyl groups include vinyl and propenyl.

In the formula (I), examples of the bicyclic heterocyclic rings comprising a benzene or pyridine ring condensed with a five-membered or six-membered heterocyclic ring include indole, indoline, indazole, pyrrolopyridine, benzofuran, benzothiophene, benzo[b]thiophene-1,1-dioxide, and tetrahydroquinoline.

The nitrogen atom contained in the ring of indole, indoline, indazole, or tetrahydroquinoline is preferably combined with A. The nitrogen atom contained in the pyrrole ring of pyrrolopyridine is preferably combined with A. Examples of pyrrolopyridines include pyrrolo[2,3-b]pyridine, pyrrolo[3,2-b]pyridine, and pyrrolo[2,3-c]pyridine.

Examples of the heteroaryl groups substituting the bicyclic heterocyclic ring in the formula (I) or the five-membered or six-membered heteroaryl groups of _{R}²³, R²⁴, or R²⁵ in the formula (II) or (III) include 1,2,4-triazolyl and tetrazolyl.

Examples of the five-membered or six-membered heteroaryl groups (comprising carbon and nitrogen atoms, said carbon atom combining to the nitrogen atom of the neighboring cyclic amine) of Z in the in the formula (I) and Z⁰ in the formula (II) or (III) include pyrimidinyl and oxadiazolyl.

The five-membered or six-membered heteroaryl group may have a substituent or substituents such as a halogen atom (e.g., fluorine atom), a C₁₋₈ alkyl group (e.g., methyl, ethyl, propyl, or isopropyl), a three-membered to seven-membered cycloalkyl group (e.g., cyclopropyl, cyclopentyl, or cyclohexyl), a C₁₋₈ alkyl group having one to three halogen atoms (e.g., trifluoromethyl).

The number of the substituents of the bicyclic heterocyclic ring comprising a benzene or pyridine ring condensed with a five-membered or six-membered heterocyclic ring in the formula (I) preferably is 1 to 3, and more preferably is 1 or 2.

Examples of the pharmaceutically acceptable salts of the compound of the formula (I), (II), or (III) include a salt with an inorganic acid such as a chloride salt or a sulfate salt and a salt with an organic acid such as a fumarate salt or a methanesulfonate salt.

In the present invention, the compound of the formula (I), (II), or (III) includes a racemic mixture and optically active isomers.

In the present invention, the compound of the formula (I), (II), or (III) includes a hydrate and a solvate.

Processes for preparation of the compound of the formula (I), (II), or (III) are described below.
(1) A compound of the formula (I) in which A is O, B is (CH₂)_{n,} W is CH, and each of X and Y is CH₂CH₂

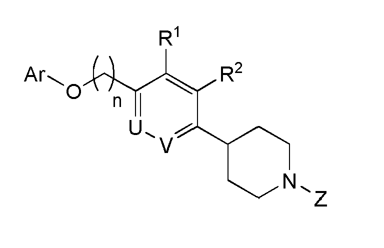

### <Method A>

### (First process)

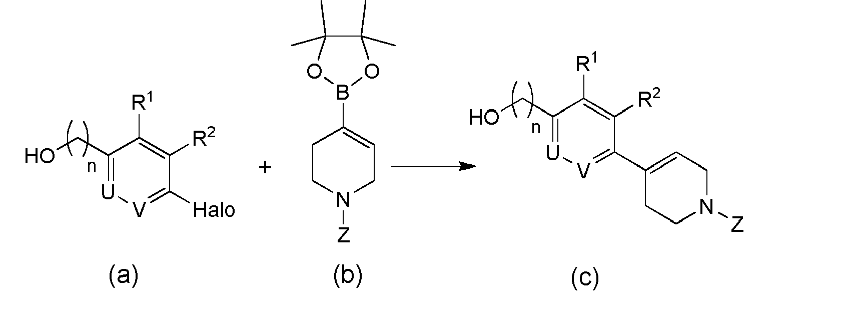

### (Second process)

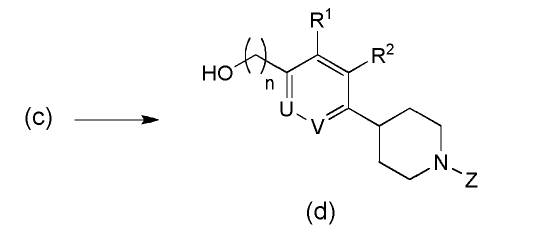

### (Third process)

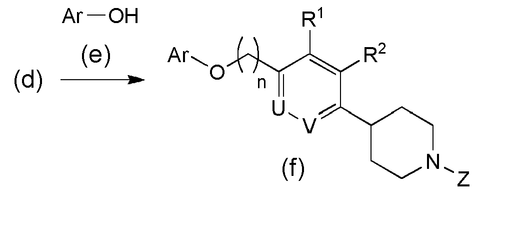

In the formulas, Halo is halogen such as chlorine, bromine, and iodine, and each of Ar, R¹, R², U, V, Z, and n is described above.

### 1)

The starting material (a) can be synthesized according to a known method (cf., M.V. Chelliah et al., J. Med. Chem., 2007, 50, 5147; and WO 2006/114213) or an analogous method thereof. The starting material (b) can also be synthesized according to a known method (cf., D.J. Wustrow et al., Synthesis, 1991, 993) or an analogous method thereof.

### 2) First process

The condensation reaction of the starting material (a) with the starting material (b) to be converted into the compound of the formula (c) can be conducted in an inert solvent such as toluene, tetrahydrofuran, dioxane and N,N-dimethylformamide, in the presence of a base such as potassium carbonate, cesium carbonate and sodium carbonate, using a catalyst such as tetrakis(triphenylphosphine)palladium and [1,1'-bis(diphenylphosphino)-ferrocene]palladium(II) dichloride dichloromethane complex. The reaction temperature is in the range from 20 to 110°C.

### 3) Second process

The compound of the formula (c) can be converted into the compound of the formula (d) in an inert solvent such as methanol and ethanol, in the presence of a catalyst such as palladium-carbon according to a catalytic hydrogenation method.

### 4) Third process

The compound of the formula (d) can be converted into the compound of the formula (f) by a reaction of the compound (d) with the heteroaryl alcohol of the formula (e) in an inert solvent such as tetrahydrofuran, dioxane and toluene, in the presence of an azodicarboxylic ester such as diisopropyl azodicarboxylate and diethyl azodicarboxylate, and a phosphine such as triphenylphosphine. The reaction temperature is in the range from 0 to 80°C.

The intermediate of the formula (d) can also be synthesized according to the following method B or C.

### <Method B>

### (First process)

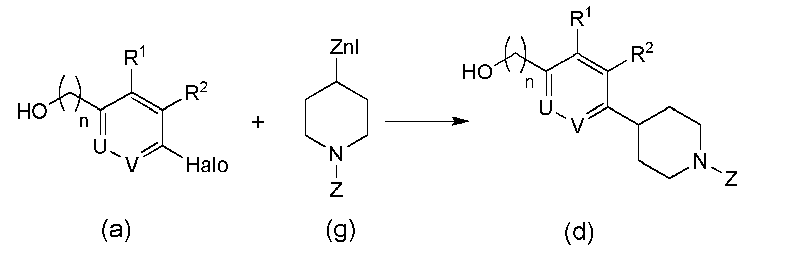

In the formulas, ZnI is zinc iodide, and each of Halo, R¹, R², U, V, Z, and n is described above.

### 1)

The starting material (g) can be synthesized according to a known method (cf., S., Billotte, Synlett, 1998, 379) or an analogous method thereof.

### 2) First process

The condensation reaction of the starting material (a) with the starting material (g) to be converted into the compound of the formula (d) can be conducted in an inert solvent such as toluene, tetrahydrofuran and N,N-dimethylformamide, optionally in the presence of an additive such as tri(2-furyl)phosphine, using a catalyst such as tris(dibenzylideneacetone)palladium and tetrakis(triphenylphosphine)palladium. The reaction temperature is in the range from 20 to 110°C.

### <Method C>

### (First process)

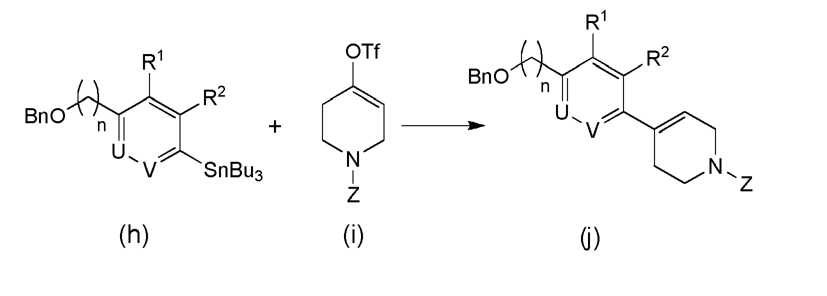

### (Second process)

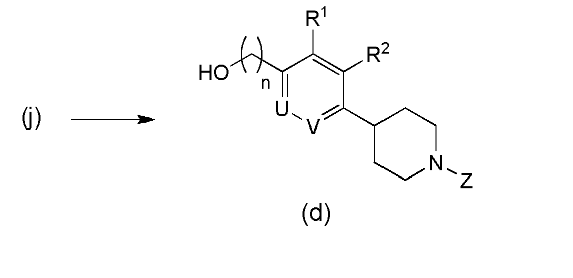

In the formulas, Bn is benzyl, Bu is butyl, Tf is trifluoromethanesulfonyl, and each of R¹, R², U, V, Z, and n is described above.

### 1)

The starting material (h) can be synthesized according to a known method (cf., H. Azizian et al., J. Organomet. Chem., 1981, 215, 49; and C. Eaborn et al., J. Chem. Soc., 1962, 1131) or an analogous method thereof. The starting material (i) can also be synthesized according to a known method (cf., D.J. Wustrow et al., Synthesis, 1991, 993) or an analogous method thereof.

### 2) First process

The condensation reaction of the starting material (h) with the starting material (i) to be converted into the compound of the formula (d) can be conducted in an inert solvent such as toluene, tetrahydrofuran and N,N-dimethylformamide, using a catalyst such as tetrakis(triphenylphosphine)palladium and tris(dibenzylideneacetone)-palladium. The reaction temperature is in the range from 20 to 110°C.

### 3) Second process

The compound of the formula (j) can be converted into the compound of the formula (d) by releasing benzyl simultaneously with the reduction reaction in the same manner as in the second process of the above-mentioned method A.

The compound of the formula (d) can also be converted into the compound of the formula (f) according to the following method D.

### <Method D>

### (First process)

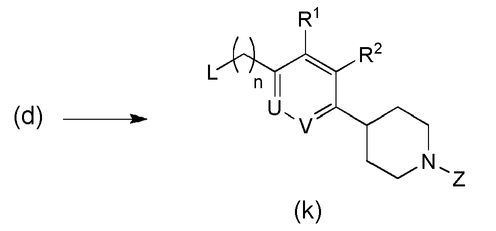

### (Second process)

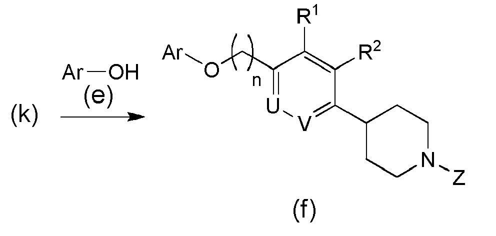

In the formulas, L is a halogen atom such as chlorine atom, bromine atom, iodine atom, or a leaving group such as methanesulfonyloxy and p-toluenesulfonyloxy, and each of Ar, R¹, R², U, V, Z, and n is described above.

### 1) First process

The compound of the formula (d) can be converted into the compound of the formula (k) by a reaction of the compound (d) with a reagent such as methanesulfonyl chloride, p-toluenesulfonyl chloride, and thionyl chloride, in an inert solvent such as toluene and dichloromethane, optionally in the presence of a base such as pyridine and triethylamine.

### 3) Second process

The compound of the formula (k) can be converted into the compound of the formula (f) by a reaction of the compound (k) with a heteroaryl alcohol represented by the formula (e) in an inert solvent such as N,N-dimethylformamide and acetone, in the presence of a base such as sodium hydride and potassium carbonate. The reaction temperature is in the range from 0 to 80°C.

The compound of the formula (n), which is a compound of the present invention and is an intermediate in preparation of the compound represented by the formula (f), can also be prepared according to the following method E.

### <Method E>

### (First process)

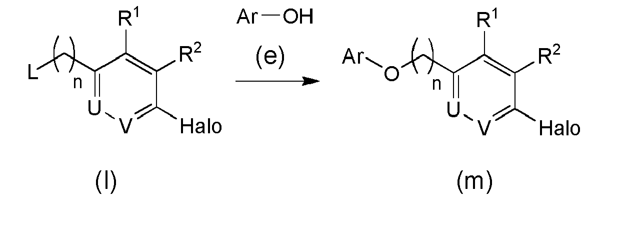

### (Second process)

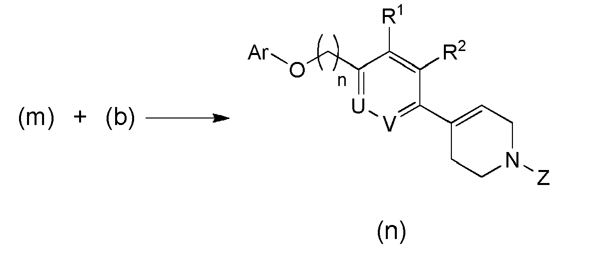

### (Third process)

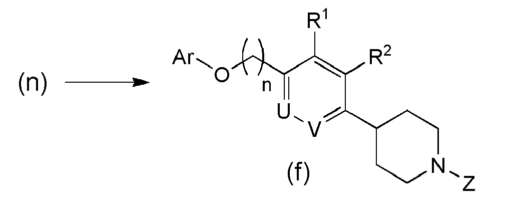

In the formulas, each of Ar, Halo, L, R¹, R², U, V, Z, and n is described above.

### 1)

The starting material (l) can be synthesized according to a known method (cf., EP 1555259) or an analogous method thereof.

### 2) First process

The starting material (l) can be converted into the compound of the formula (m) in the same manner as in the second process of the above-mentioned method D.

### 3) Second process

The compound of the formula (m) can be converted into the compound of the formula (n) in the same manner as in the first process of the above-mentioned method A.

### 4) Third process

The compound of the formula (n) can be converted into the compound of the formula (f) in the same manner as in the second process of the above-mentioned method A.
(2) A compound of the formula (II) in which A⁰ is CH₂, B⁰ is a bond, and each of X° and Y° is CH₂CH₂

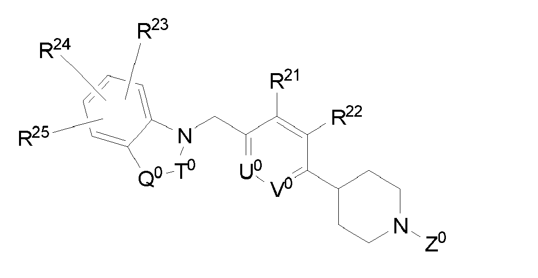

### <Method F>

### (First process)

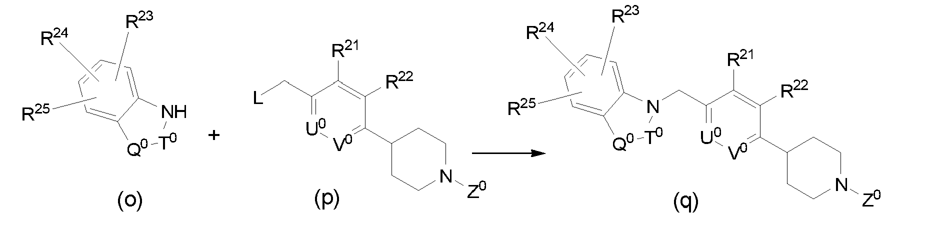

In the formulas, each of L, R²¹, R22, R²³, R²⁴, R²⁵, Q⁰, T⁰, U⁰, V⁰, and Z⁰ is described above.

### 1) First process

The compound of the formula (p) can be converted into the compound of the formula (q) by a reaction of the compound (p) with the compound of the formula (O) in an inert solvent such as toluene, N,N-dimethylformamide, and acetone, in the presence of a base such aspotassium hydroxide, sodium hydride, and potassium carbonate, and optionally in the presence of an additive such as a crown ether and potassium iodide. The reaction temperature is in the range from the room temperature to 130°C.

The compound represented by the formula (I), (II), or (III) can be prepared, for example by referring to the above-described methods, the below-described examples, and the Patent Documents 1 to 7.

Examples of the representative compounds of the present invention are shown below.

### Representative compound (1)

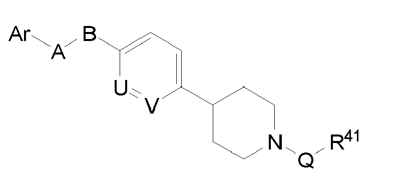

In the formula, Ar, A, B, U, V, Q, and R⁴¹ are set forth in Tables 1 and 2.

**TABLE 1**

| Ar | A | B | U | V | Q | R⁴¹ |
|---|---|---|---|---|---|---|
| 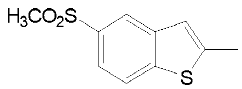 | Bond | Bond | N | CH | C(O)O | Isopropyl |
| 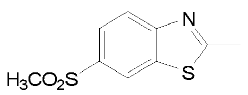 | Bond | Bond | CH | N | C(O)O | t-Butyl |
| 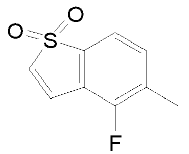 | O | CH₂ | N | N | C(O)O | t-Butyl |
| 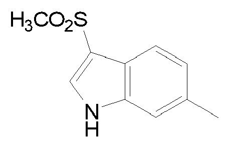 | O | CH₂ | N | CH | 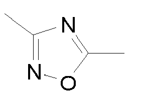 | Isopropyl |
| 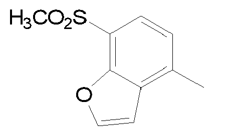 | O | CH₂ | N | CH | C(O)O | Isopropyl |

**TABLE 2**

| Ar | A | B | U | V | Q | R⁴¹ |
|---|---|---|---|---|---|---|
| 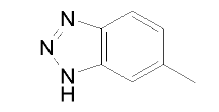 | O | CH₂ | N | CH | C(O)O | Isopropyl |
| 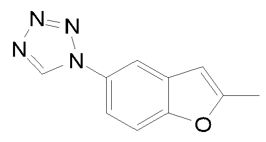 | Bond | Bond | N | CH | C(O)O | t-Butyl |
| 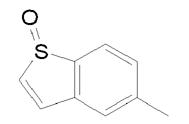 | O | CH₂ | N | CH | 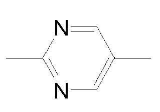 | Ethyl |
| 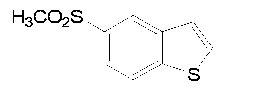 | CH₂ | Bond | N | CH | C(O)O | t-Butyl |
| 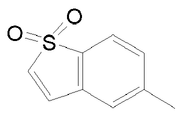 | O | CH₂ | N | CH | 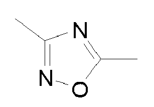 | Isopropyl |
| 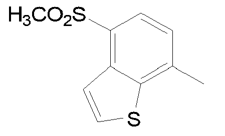 | O | CH₂ | N | CH | 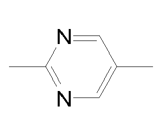 | Isopropyl |

### Representative compound (2)

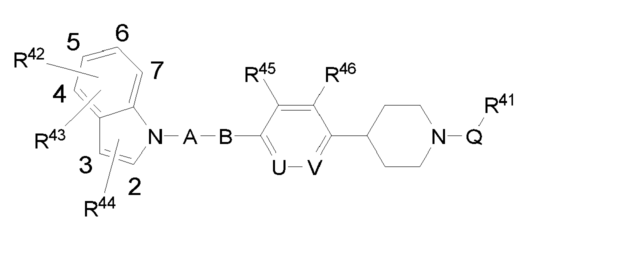

In the formula, R⁴², R⁴³, R⁴⁴, R⁴⁵, R⁴⁶, A, B, U, V, Q, and R⁴¹ are set forth in Tables 3 to 5.

**TABLE 3**

| R⁴² | R⁴³ | R⁴⁴ | R⁴⁵ | R⁴⁶ | A | B | U | V | Q | R⁴¹ |
|---|---|---|---|---|---|---|---|---|---|---|
| 5-SO₂CH₃ | 6-F | - | - | - | CH₂ | B* | N | N | C(O)O | propyl |
| 5- Tetrazolyl | 7-F | - | CH₃ | - | CH₂ | B* | N C | C H | C(O)O | t-Butyl |
| 5-SO₂CH₃ | 7-F | - | Cl | - | CH₂ | B* | N | C H | C(0) O | t-Butyl |
| 5-SO₂CH₃ | 7-CH₃ | 3-CH₃ | - | - | CH₂ | B* | CH | N | C (O) O | n-Butyl |
| 4-CF₃ | - | - | F | - | CH₂CH₂ | B* C | H | N | C(O) | Cyclopropyl |
| 5-SO₂NH₂ | - | - | Cl | - | CH₂ | B* | N C | H | C(O)O | Isopropyl |
| 5-SO₂CH₃ | - | - | | CH₃ | CH₂ | B* | N C | H | C(O)O | Ethyl |

| | | | | | | | | | | |
|---|---|---|---|---|---|---|---|---|---|---|
| (Remark) 5-Tetrazolyl: 5-(Tetrazol-1-yl) B*: Bond | | | | | | | | | | |

**TABLE 4**

| R⁴² | R⁴³ | R⁴⁴ | R⁴⁵ | R⁴⁶ | A | B | U | V | Q | R⁴¹ |
|---|---|---|---|---|---|---|---|---|---|---|
| 5-SO₂NH₂ | 4-F | 2-CH₃ | 2-CH₃ | - | B* | C(CH3)H | N | N | C(O)O | Isopropyl |
| 5-Tetrazolyl | - | - | - | - | CH₂ | B* | N | CH | C(O)O | t- Butyl |
| 5-SO₂C₂H₅ | 7-OCH₃ | 7- | - | Cl | CH₂ | B* | N | CH | S(O)₂ | n-Propyl |
| 4-N(CH₃)₂ | - | - | CF₃ | - | CH₂ | O | C H | N | C(O)O | n-Butyl |
| 5-SO₂NHCH₃ | - | - | - | - | CH₂ | O | N | C | N C C(O)O | t-Butyl |
| 5-SO₂CH₃ | - | - | - | - | O | CH₂ | N | C H | C(O)O | Isopropyl |
| 5-Tetrazolyl | 7-F | - | Cl | - | CH₂ | B* | N | C H | C(O)O | t-Butyl |
| 5-CO₂CH₃ | - | - | - | - | CH₂ | B* | N | N | C(O)O | Bn |

| | | | | | | | | | | |
|---|---|---|---|---|---|---|---|---|---|---|
| (Remark) 5-Tetrazolyl: 5-(Tetrazol*-*1-yl) B*: Bond Bn: Benzyl | | | | | | | | | | |

**TABLE 5**

| R⁴² | R⁴³ | R⁴⁴ | R⁴⁵ | R⁴⁶ | A | B | U | V | Q | R⁴¹ |
|---|---|---|---|---|---|---|---|---|---|---|
| 5-Tetrazolyl | 4-F | - | - | - | CH₂ | B* | N | C H | 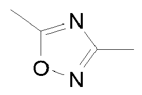 | Isopropyl |
| 5-SO₂CH₃ | - | - | Cl | - | CH₂ | B* | N | C H | 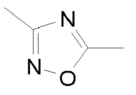 | Isopropyl |
| 5- SO₂CH₃ | 7-Cl | - | - | - | CH₂ | B* | N | C H | 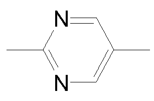 | Ethyl |
| 5-SO₂CH₃ | 6-CF₃ | - | - | - | CH₂ | B* | N | C H | 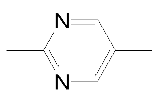 | n-Propyl |

| | | | | | | | | | | |
|---|---|---|---|---|---|---|---|---|---|---|
| (Remark) 5-Tetrazolyl: 5-(Tetrazol-1-yl) B*: Bond | | | | | | | | | | |

### Representative compound (3)

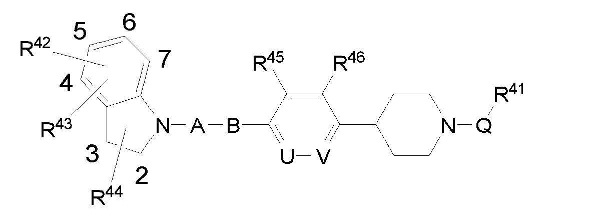

In the formula, R⁴², R⁴³, R⁴⁴, R⁴⁵, R⁴⁶, A, B, U, V, Q, and R⁴¹ are set forth in Tables 6 to 8.

**TABLE 6**

| R⁴² | R⁴³ | R⁴⁴ | R⁴⁵ | R⁴⁶ | A | B | U | V | Q | R⁴¹ |
|---|---|---|---|---|---|---|---|---|---|---|
| 5-Tetrazolyl | 7-CH₃ | - | - | CH₃ | CH₂ | B* | C H | N | C(O)O | t-Butyl |
| 5-SO₂CH₃ | - | 2-CH₃ | - | - | CH₂ | B* | N | N | C(O)O | t-Butyl |
| 5- | 6- | - | CH₃ | - | CH₂ | B* | N | C | C(O)O | t―Butyl |
| 5-SO₂CH₃ | 6-F | - | - | - | CH₂ | B* | N | H | C(O)O | n-Butyl |
| 6-NO₂ | - | - | OCH₃ | - | CH₂ | O | N | C H | C(O)O | Ethyl |
| 5-Tetrazolyl | 7-F | - | Cl | - | CH₂ | B* | N | C H | C(O)O | Isopropyl |

| | | | | | | | | | | |
|---|---|---|---|---|---|---|---|---|---|---|
| (Remark) 5-Tetrazolyl: 5-(Tetrazol-1-yl) B*: Bond | | | | | | | | | | |

**TABLE 7**

| R⁴² | R⁴³ | R⁴⁴ | R⁴⁵ | R⁴⁶ | A | B | U | V | Q | R⁴¹ |
|---|---|---|---|---|---|---|---|---|---|---|
| 5-SO₂CH₃ | 7-Cl | - | - | - | CH₂ | B* | N | CH | C(O)O | t-Butyl |
| 5-Tetrazolyl | - | 3-CH₃ | - | Cl | CH₂ | B* | N | CH | C(O)O | Isopropyl |
| 5-SO₂Ph | - | - | - | - | CH₂ | B* | N | CH | C(O)O | t-Butyl |
| 5-SO₂CH₃ | - | - | - | - | CH₂ | B* | N | CH | C(O) | Isobutyl |
| 5-SO₂CH₃ | 7-F | - | Cl | - | CH₂ | B* | N | CH | C(O)O | t-Butyl |

| | | | | | | | | | | |
|---|---|---|---|---|---|---|---|---|---|---|
| (Remark) 5-Tetrazolyl: 5-(Tetrazol-1-yl) B*: Bond | | | | | | | | | | |

**TABLE 8**

| R⁴² | R⁴³ | R⁴⁴ | R⁴⁵ | R⁴⁶ | A | B | U | V | Q | R⁴¹ |
|---|---|---|---|---|---|---|---|---|---|---|
| 5-SO₂CH₃ | - | - | - | - | CH₂ | B* | N | C H | 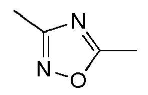 | Isopropyl |
| 5- SO₂CH₃ | 7- F | - | Cl | - | CH₂ | B* | N | C H | 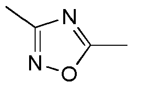 | Isopropyl |
| 5-Tetrazolyl | - | - | - | - | CH₂ | B* | N | C H | 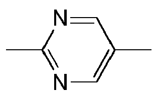 | Ethyl |
| 5- SO₂CH₃ | - | - | - | - | CH₂ | B* | N | C H | 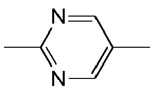 | Ethyl |

| | | | | | | | | | | |
|---|---|---|---|---|---|---|---|---|---|---|
| (Remark) 5-Tetrazolyl: 5-(Tetrazol-1-yl) B*: Bond | | | | | | | | | | |

### Representative compound (4)

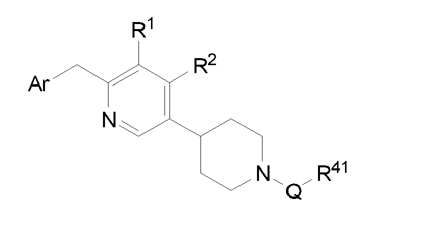

In the formula, Ar, R¹, R², Q, and R⁴¹ are set forth in Table 9.

**TABLE 9**

| Ar | R¹ | R² | Q | R⁴¹ |
|---|---|---|---|---|
| 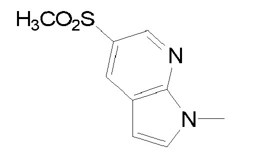 | H | H | C(O)O | Isopropyl |
| 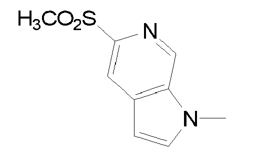 | H | H | C(O)O | t-Butyl |
| 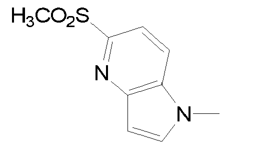 | H | H | C(O)O | t-Butyl |
| 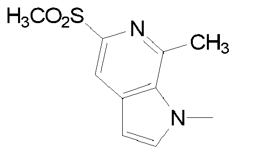 | H | H | C(O)O | t-Butyl |
| 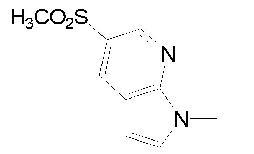 | Cl | H | 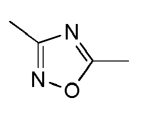 | Isopropyl |
| 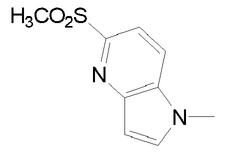 | CH₃ | H | 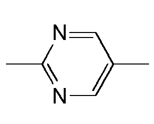 | Isopropyl |

The pharmacological tests are described below.

### (Pharmacological test A)

The GPR119 agonist effect is studied by measuring the effect of an analyte on increase of intracellular amount of cAMP in human GPR119 introduced cells. The testing method is described below.

### (1) Construction of the stable cell line expressing human GPR119

Human GPR119 gene (NM 178471) is purchased from ATCC (ATCC No. 10807349), and is amplyfied according to PCR to form BamHI site at 5' side and Apa I site at 3' side. The primers are tcctggatccatggaatcatctttctcatt (sequence No. 1) and tcctgggcccttagccatcaaactctgagc (sequence No. 2). The PCR conditions are described below.

The double-stranded DNA is thermally denatured using a DNA polymerase (KOD-Plus-Ver. 2; TOYOBO #KOD-211) at 98°C for 10 seconds in one cycle. The denatured single-stranded DNA is annealed with the primers at 55°C for 30 seconds. The DNA is subjected to an extension reaction at 68°C for 1 minute and 15 seconds. The above-mentioned steps are repeated in 35 cycles. The PCR product is inserted into pcDNA5/FRT/TO (Invitrogen #V6520-20) plasmid. Flp-in T-Rex-293 cells (Invitorogen #R78007) are transfected with the obtained plasmid. The method of transfection is conducted in accordance with the protocol of the product.

### (2) Measurement of intracellular cAMP

The stable cell line expressing human GPR119 prepared in the above-mentioned method is plated on a 96-well plate at the concentration of 2,500 cells/well using Dulbecco's Modified Eagle Medium (DMEM) containing 10% fetal bovine serum (FBS). Twenty-four hours after plating, tetracyclin (Invitrogen #Q10019) is added at the final concentration of 20 ng/mL to induce hGPR119 gene expression. Twenty-four hours after, the medium is removed, and the cells are stimulated with an assay buffer (0.5 mM IBMX PBS (-)) containing the test compound at 37°C for 30 minutes. The amount of the intracellular cAMP is measured using a commercially available kit (HitHunter^{™} cAMP XS+ Assay: GE Healthcare #90007503) and a reader (FLUOstar Optima: BMG LABTECH). The test compound is dissolved in 100% DMSO, and added at the final concentration of 1%.

### (3) Experimental results

As is evident from Table 10 of Example 87 described below, the compounds of Examples 2, 14, or the like show an excellent GPR119 agonist effect.

As is also evident from Tables 11 to 13 of Example 88 described below, the compounds of Examples 38, 41, 83, or the like show an excellent GPR119 agonist effect.

### (Pharmacological test B)

Oral glucose tolerance is tested in normal mice.

### (1) Experimental procedure

In this experiment, the inhibitory effect of an analyte on glycemic excursions is examined after glucose administration in normal mice. The test methods are described below.

Male 9-week-old ICR mice, habituated to the experimental environment for two weeks, are fasted for 18 hours and used to this experiment. Mice are orally administered the analyte or vehicle (polyethylene glycol 400: ethanol: Tween 80 = 8:1:1). After 30 minutes, they were orally given glucose at the dose of 3 g/kg.

Blood was collected at just before the analyte or vehicle administration (-30 minutes), immediately before glucose challenge (0 minute), 20 minutes, 40 minutes, 60 minutes, and 120 minutes after glucose ingestion to determine blood glucose levels.

Inhibition rate (%) of the analyte versus vehicle in areas under the glycemic excursion curve between 0 and 120 min after glucose challenge is determined.

### (2) Experimental results

As is evident from Table 14 of Example 89 described below, the compounds of Examples 1, 2 show an excellent inhibitory effect on glycemic excursions.

As is also evident from Table 15 of Example 90 described below, the compounds of Examples 24, 54 show an excellent inhibitory effect on glycemic excursions.

As is described above, the compound represented by the formula (I), (II), or (III), or a pharmaceutically acceptable salt thereof has a GPR119 agonist effect and an inhibitory effect on glycemic excursions. Therefore, they are expected to be used for treatment of diabetes. They are also expected to be used for a life-style related diseases such as obesity and metabolic syndrome.

The compound represented by the formula (I), (II), or (III), or a pharmaceutically acceptable salt thereof can be used in combination with a conventional agent for treatment of diabetes.

The compound represented by the formula (I), (II), or (III), or a pharmaceutically acceptable salt thereof can be administered to human beings by suitable administration methods such as oral administration or parenteral administration. It can also be useed in combination with another agent for treatment of diabetes.

The compound or salt can be granulated in suitable manners for the preparation of pharmaceuticals. For instance, the compound or salt can be processed to give tablets, granule, powder, capsule, suspension, injection, suppository, and the like.

For the preparation of these pharmaceuticals, when they are tablets, appropriate additives such as excipients, disintegrators, binders, lubricants and dyes can be used. Lactose, D-mannitol, crystalline cellulose and glucose can be used as the excipients. Starch and carboxymethylcellulose calcium (CMC-Ca) can be used as the disintegrators, magnesium stearate, and talc as the lubricants. Hydroxypropylcellulose (HPC), gelatin and polyvinylpyrrolidone (PVP) can be used as the binders. For the preparation of injection, a solvent, a stabilizer, a solubilizer, a suspending agent, an emulsifier, an analgesic, a buffer, and a preservative can be used.

The compound represented by the formula (I), (II), or (III), or a pharmaceutically acceptable salt thereof can be administered to an adult generally in an amount of 0.01 mg to 100 mg a day by injection and 1 mg to 2,000 mg a day by oral administration. The dosage can be adjusted according to age and conditions of the patient.

The invention is further described by the following non-limiting examples.

### [Example 1]

### tert-Butyl 4-[2-(5-methanesulfonylindol-1-ylmethyl)pyridin-5-yl]piperidine-1-carboxylate

### (1) Benzyl 4- [2- (hydroxymethyl) pyridin-5-yl]-3, 6-dihydro-2H-pyridine-1-carboxylate

To a solution of 5-bromopyridine-2-methanol (100 mg, 0.532 mmol) and benzyl 4-(4,4,5,5-tetramethyl-1,3,2-dioxaborolan-2-yl)-3,6-dihydro-2H-pyridine-1-carboxylate (200 mg, 0.585 mmol) in dry N,N-dimethylformamide (1.3 mL) - dry tetrahydrofuran (1.3 mL) was added [1,1'-bis(diphenylphosphino)ferrocene]dichloropalladium(II) dichloromethane adduct (22 mg, 0.027 mmol) and cesium carbonate (347 mg, 1.06 mmol) under N₂. After stirring at 90°C for 2.5 hours, cooled to room temperature, the reaction mixture was poured into water (5 mL), and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/2), to give the title compound as an orange oil (120 mg, yield 70%) .
¹H NMR (CDCl₃ 400 MHz): δ= 2.5-2.6 6 (2H, m), 3.58 (1H, br s), 3.7-3.8 (2H, m), 4.1-4.2 (2H, m), 4.76 (2H, s), 5.18 (2H, s), 6.0-6.2 (1H, m), 7.22 (1H, d, J = 8 Hz), 7.3-7.4 (5H, m), 7.65 (1H, dd, J = 2 Hz, 8 Hz), 8.57 (1H, d, J = 2 Hz).

### (2) tert-Butyl 4-[2-(hydroxymethyl)pyridin-5-yl]piperidine-1-carboxylate

To a solution of benzyl 4-[2-(hydroxymethyl)pyridin-5-yl]-3,6-dihydro-2H-pyridine-1-carboxylate (527 mg, 1.63 mmol) in methanol (16 mL) was added 10% palladium-carbon (105 mg) and then the mixture was hydrogenated at room temperature for 17 hours under 1 atm of H₂. The reaction mixture was filtered through a celite pad, and the filtrate was concentrated under reduced pressure.

To a solution of the above crude 4-[2-(hydroxymethyl)pyridin-5-yl]-1H-piperidine in tetrahydrofuran (8 mL) - water (8 mL) was added triethylamine (0.34 mL, 2.43 mmol) and di-tert-butyl dicarbonate (390 mg, 1.79 mmol). After stirring at room temperature for 10 minutes, the reaction mixture was poured into water (15 mL), and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/2 → chloroform/methanol = 30/1), to give the title compound as a pale yellow oil (322 mg, yield 68%) .
¹H NMR (CDCl₃ 400 MHz): δ= 1.49 (9H, s), 1.5-1.7 7 (2H, m), 1.8-1.9 (2H, m), 2.6-2.8 (1H, m), 2.7-2.9 (2H, m), 3.61 (1H, br s), 4.2-4.4 (2H, m), 4.74 (2H, s), 7.20 (1H, d, J = 8 Hz), 7.51 (1H, dd, J = 2 Hz, 8 Hz), 8.43 (1H, d, J = 2 Hz).

### (3) tert-Butyl 4-[2-(methanesulfonyloxymethyl)pyridin-5-yl]piperidine-1-carboxylate

To a solution of tert-butyl 4-[2-(hydroxymethyl)pyridin-5-yl]piperidine-1-carboxylate (100 mg, 0.342 mmol) in chloroform (2.4 mL) was added triethylamine (0.07 mL, 0.51 mmol) under ice-cooling. To this was added dropwise a solution of methanesulfonyl chloride (0.030 mL, 0.39 mmol) in chloroform (1 mL). After stirring at room temperature for 3 hours, the solvent was removed under reduced pressure. The residue was purified by silica gel column chromatography (hexane/ethyl acetate = 9/1 to 0/100), to give the title compound as an orange oil (59 mg, yield 47%) .
¹H NMR (CDCl₃ 400 MHz): δ= 1.49 (9H, s), 1.6-1.7 (2H, m), 1.8-1.9 (2H, m), 2.6-2.8 (1H, m), 2.7-2.9 (2H, m), 3.09 (3H, s), 4.2-4.3 (2H, m), 5.31 (2H, s), 7.42 (1H, d, J = 8 Hz), 7.58 (1H, dd, J = 2 Hz, 8 Hz), 8.48 (1H, d, J = 2 Hz).

### (4) tert-Butyl 4-[2-(5-methanesulfonylindol-1-ylmethyl)pyridin-5-yl]piperidine-1-carboxylate

A suspension of tert-butyl 4-[2-(methanesulfonyloxymethyl)pyridin-5-yl]piperidine-1-carboxylate (100 mg, 0.270 mmol), 5-methanesulfonylindol (44 mg, 0.225 mmol), potassium hydroxide (13 mg, 0.225 mmol) and 18-crown-6-ether (60 mg, 0.226 mmol) in dry toluene (3.0 mL) was stirred at 80°C overnight. After cooling to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (chloroform/ethyl acetate = 1/1), to give the title compound as a pale yellow amorphous (48 mg, yield 45%).
FAB-MS (m/z): 470 (M+1)
¹H NMR (CDCl₃ 400 MHz): δ= 1.47 (9H, s), 1.5-1.7 7 (2H, m), 1.7-1.9 (2H, m), 2.6-2.8 (1H, m), 2.7-2.9 (2H, m), 3.07 (3H, s), 4.1-4.4 (2H, m), 5.47 (2H, s), 6.7-6.8 (2H, m), 7.37 (1H, d, J = 3 Hz), 7.40 (1H, dd, J = 2 Hz, 8 Hz), 7.44 (1H, d, J = 8 Hz), 7.70 (1H, dd, J = 1 Hz, 8 Hz), 8.29 (1H, d, J = 1 Hz), 8.46 (1H, d, J = 2 Hz).

### [Example 2]

### tert-Butyl 4-[2-(5-methanesulfonylindolin-1-ylmethyl)pyridin-5-yl]piperidine-1-carboxylate

The title compound was prepared from 5-methanesulfonylindoline (44 mg, 0.223 mmol) by the similar manner as described in Example 1 (4) as a pale yellow amorphous (39 mg, yield 37%) .
FAB-MS (m/z): 472 (M+1)
¹H NMR (CDCl₃ 400 MHz): δ= 1.48 (9H, s), 1.5-1.7 7 (2H, m), 1.7-1.9 (2H, m), 2.6-2.8 (1H, m), 2.7-2.9 (2H, m), 2.99 (3H, s), 3.11 (2H, t, J = 8 Hz), 3.68 (2H, t, J =8 Hz), 4.1-4.4 4 (2H, m), 4.49 (2H, s), 6.43 (1H, d, J = 8 Hz), 7.21 (1H, d, J = 8Hz), 7.49 (1H, dd, J = 2 Hz, 8 Hz), 7.54 (1H, br s), 7.60 (1H, br d), 8.45 (1H, d, J = 2 Hz).

### [Example 3]

### tert-Butyl 4-[2-(4-methanesulfonylindol-1-ylmethyl)pyridin-5-yl]piperidine-1-carboxylate

The title compound was prepared from 4-methanesulfonylindole (44 mg, 0.225 mmol) by the similar manner as described in Example 1 (4) as a yellow amorphous (47 mg, yield 44%) .
FAB-MS (m/z): 470 (M+1)
¹H NMR (CDCl₃ 400 MHz): δ= 1.47 (9H, s), 1.5-1.7 7 (2H, m), 1.7-1.9 (2H, m), 2.6-2.7 (1H, m), 2.7-2.9 (2H, m), 3.14 (3H, s), 4.1-4.4 (2H, m), 5.49 (2H, s), 6.74 (1H, d, J = 8 Hz), 7.04 (1H, d, J = 3 Hz), 7.30 (1H, t, J = 8 Hz), 7.39 (1H, dd, J = 2 Hz, 8 Hz), 7.43 (1H, d, J = 3 Hz), 7.60 (1H, d, J = 8 Hz), 7.77 (1H, d, J = 8 Hz), 8.46 (1H, d, J = 2 Hz).

### [Example 4]

### tert-Butyl 4-[2-(5-methanesulfonyl-2-methylindol-1-ylmethyl)pyridin-5-yl]piperidine-1-carboxylate

The title compound was prepared from 5-methanesulfonyl-2-methylindole (60 mg, 0.287 mmol) by the similar manner as described in Example 1 (4) as a white amorphous (58 mg, yield 42%) .
FAB-MS (m/z): 484 (M+1)
¹H NMR (CDCl₃ 400 MHz): δ= 1.47 (9H, s), 1.5-1.7 7 (2H, m), 1.7-1.9 (2H, m), 2.45 (3H, s), 2.6-2.7 (1H, m), 2.7-2.9 (2H, m), 3.07 (3H, s), 4.1-4.4 (2H, m), 5.43 (2H, s), 6.4-6.6 (2H, m), 7.33 (1H, d, J = 8 Hz), 7.35 (1H, dd, J ₌ 2 Hz, 8 Hz), 7.64 (1H, dd, J = 2 Hz, 8 Hz), 8.18 (1H, d, J = 2 Hz), 8.46 6 (1H, d, J = 2 Hz) .

### [Example 5]

### tert-Butyl 4-[2-(5-methanesulfonylindol-1-ylmethyl)-3-methylpyridin-5-yl]piperidine-1-carboxylate

### (1) 5-Bromo-2-(tert-butyldimethylsilyloxymethyl)-3-methylpyridine

To a solution of 5-bromo-2-hydroxymethyl-3-methylpyridine (1.0 g, 5.0 mmol) in N,N-dimethylformamide (50 mL) was added triethylamine (1.56 mL, 15.0 mmol) and tert-butyldimethylsilyl chloride (1.13 g, 7.5 mmol), and the mixture was stirred at room temperature for 4 hours. The reaction mixture was poured into water (50 mL), and was extracted with ethyl acetate. The organic layer was washed with brine, dried over anhydrous sodium sulfate, and the solvent was removed under reduced pressure. The residue was purified by silica gel column chromatography (hexane/ethyl acetate = 4/1), to give the title compound as a colorless oil (1.51 g, yield 100%).
¹H NMR (CDCl₃ 400 MHz): δ= 0.07 (6H, s), 0.89 (9H, s), 2.40 (3H, s), 4.77 (2H, s), 7.61 (1H, br s), 8.41 (1H, d, J = 2 Hz).

### (2) tert-Butyl 4-[2-(tert-butyldimethylsilyloxymethyl)-3-methylpyridin-5-yl]-3,6-dihydro-2H-pyridine-1-carboxylate

The title compound was prepared from 5-bromo-2-(tert-butyldimethylsilyloxymethyl)-3-methylpyridine (907 mg, 3.00 mmol) and tert-butyl 4-(4,4,5,5-tetramethyl-1,3,2-dioxaborolan-2-yl)-3,6-dihydro-2H-pyridine-1-carboxylate (928 mg, 3.00 mmol) by the similar manner as described in Example 1 (1) as a yellow oil (1.12 g, yield 97%) .
¹H NMR (CDCl₃ 400 MHz): δ= 0.07 (6H, s), 0.89 (9H, s), 1.49 (9H, s), 2.41 (3H, s), 2.4-2.6 (2H, m), 3.6-3.7 (2H, m), 4.0-4.1 (2H, m), 4.81 (2H, s), 6.07 (1H, br s), 7.42 (1H, d, J = 2 Hz), 8 . 37 (1H, d, J = 2 Hz).

### (3) tert-Butyl 4-[2-(hydroxymethyl)-3-methylpyridin-5-yl]piperidine-1-carboxylate

To a solution of tert-butyl 4-[2-(tert-butyldimethylsilyloxymethyl)-3-methylpyridin-5-yl]-3,6-dihydro-2H-pyridine-1-carboxylate (1.18 g, 2.91 mmol) in dry tetrahydrofuran (29 mL) was added 1.0 M tetrabutylammonium fluoride-tetrahydrofuran solution (4.37 mL, 4.37 mmol), and the mixture was stirred at room temperature for 5 hours. The reaction mixture was added ethyl acetate, the organic layer was washed with brine, dried over anhydrous sodium sulfate, and the solvent was removed under reduced pressure. To a solution of the resulting mixture in methanol (29 mL) was added 10% palladium-carbon (617 mg) and then the mixture was hydrogenated at room temperature for 1 hour under 1 atm of H₂. The reaction mixture was filtered through a celite pad, and the filtrate was concentrated under reduced pressure. The residue was purified by silica gel column chromatography (hexane/ethyl acetate = 1/2), to give the title compound as a white crystal (605 mg, yield 68%) .
¹H NMR (CDCl₃ 400 MHz): δ= 1.49 (9H, s), 1.5-1.7 (2H, m), 1.7-1.9 (2H, m), 2.20 0 (3H, s), 2.6-2.9 (3H, m), 4.2-4.4 4 (2H, m), 4.6-4.8 (1H, m), 4.66 6 (2H, s), 7.26 6 (1H, s), 8.26 (1H, s).

### (4) tert-Butyl 4-[2-(5-methanesulfonylindol-1-ylmethyl)-3-methylpyridin-5-yl]piperidine-1-carboxylate

To a solution of tert-butyl 4-[2-(hydroxymethyl)-3-methylpyridin-5-yl]piperidine-1-carboxylate (61 mg, 0.20 mmol) in dichloromethane (2.0 mL) was added triethylamine (42 µL, 0.30 mmol) under ice-cooling. To this was added methanesulfonyl chloride (17 µL, 0.22 mmol) slowly. After stirring at room temperature for 3 hours, the solvent was removed under reduced pressure. The residue was added 5-methanesulfonylindole (31 mg, 0.16 mmol), potassium hydroxide (9 mg, 0.16 mmol), 18-crown-6-ether (42 mg, 0.16 mmol) and dry toluene (2.0 mL) and the mixture was stirred overnight at 80°C. After cooling to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (chloroform/ethyl acetate = 1/1), to give the title compound as a pale yellow amorphous (23 mg, yield 24%) .
FAB-MS (m/z): 484 (M+1)
¹H NMR (CDCl₃ 400 MHz): δ= 1.48 (9H, s), 1.5-1.7 7 (2H, m), 1.7-1.9 (2H, m), 2.23 (3H, s), 2.6-2.7 (1H, m), 2.7-2.9 (2H, m), 3.06 (3H, s), 4.1-4.4 (2H, m), 5.44 (2H, s), 6.66 (1H, d, J = 3 Hz), 7.2-7.3 (2H, m), 7.55 (1H, d, J = 9 Hz), 7.70 (1H, dd, J = 1 Hz, 9 Hz), 8.25 (1H, d, J = 2 Hz), 8.29 (1H, d, J = 1 Hz).

### [Example 6]

### tert-Butyl 4-[2-(5-methanesulfonylindolin-1-ylmethyl)-3-methylpyridin-5-yl]piperidine-1-carboxylate

The title compound was prepared from 5-methanesulfonylindoline (32 mg, 0.16 mmol) by the similar manner as described in Example 5 (4) as a pale yellow amorphous (28 mg, yield 36%) .
FAB-MS (m/z): 486 (M+1)
¹H NMR (CDCl₃ 400 MHz): δ= 1.48 (9H, s), 1.5-1.7 7 (2H, m), 1.7-1.9 (2H, m), 2.32 (3H, s), 2.5-2.7 (1H, m), 2.7-2.9 (2H, m), 2.99 (3H, s), 3.03 (2H, t, J = 8 Hz), 3.52 (2H, t, J = 8 Hz), 4.1-4.4 (2H, m), 4.43 (2H, s), 6.55 (1H, d, J = 8 Hz), 7.31 (1H, s), 7.52 (1H, s), 7.61 (1H, d, J = 8 Hz), 8.27 (1H, s).

### [Example 7]

### tert-Butyl 4-[2-(6-methanesulfonyl-1,2,3,4-tetrahydroquinolin-1-ylmethyl)pyridin-5-yl]piperidine-1-carboxylate

The title compound was prepared from 6-methanesulfonyl-1,2,3,4-tetrahydroquinoline (63 mg, 0.298 mmol) by the similar manner as described in Example 1 (4) as a pale yellow amorphous (25 mg, yield 17%) .
FAB-MS (m/z): 486 (M+1)
¹H NMR (CDCl₃ 400 MHz): δ= 1.48 (9H, s), 1.5-1.7 (2H, m), 1.7-1.9 (2H, m), 2.0-2.1 (2H, m), 2.6-2.8 (1H, m), 2.7-2.9 (2H, m), 2.86 (2H, t, J = 6 Hz), 2.98 (3H, s), 3.55 (2H, t, J = 6 Hz), 4.1-4.4 4 (2H, m), 4.63 (2H, s), 6.49 (1H, d, J = 8 Hz), 7.07 (1H, d, J = 8 Hz), 7.4-7.5 (3H, m), 8.46 (1H, d, J = 2 Hz).

### [Example 8]

### tert-Butyl 4-[2-(5-methanesulfonylindoline-1-carbonyl)pyridin-5-yl]piperidine-1-carboxylate

### (1) tert-Butyl 4-[2-(benzyloxycarbonyl)pyridin-5-yl]-3,6-dihydro-2H-pyridine-1-carboxylate

Under N₂ atmosphere, to a solution of benzyl 5-bromopyridine-2-carboxylate (790 mg, 2.70 mmol), tert-butyl 4-(4,4,5,5-tetramethyl-1,3,2-dioxaborolan-2-yl)-3,6-dihydro-2H-pyridine-1-carboxylate(1.09 g, 3.53 mmol), tetrakis(triphenylphosphine)palladium(0) (187 mg, 0.162 mmol) and cesium carbonate (1.15 g, 3.53 mmol) in dry N,N-dimethylformamide (10 mL) was stirred at 80°C for 6 hours. After cooling to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/1), to give the title compound (659 mg, yield 62%).
¹H NMR (CDCl₃ 400 MHz): δ= 1.49 (9H, s), 1.5-1.6 (2H, m), 2.5-2.6 (2H, m), 3.6-3.7 (2H, m), 5.46 (2H, s), 6.1-6.3 (1H, m), 7.2-7.6 (5H, m), 7.75 (1H, dd, J = 2 Hz, 8 Hz), 8.09 (1H, d, J = 8 Hz), 8.78 (1H, d, J = 2 Hz).

### (2) tert-Butyl 4-(2-carboxypyridin-5-yl)piperidine-1-carboxylate

To a solution of tert-butyl 4-[2-(benzyloxycarbonyl)pyridin-5-yl]-3,6-dihydro-2H-pyridine-1-carboxylate (490 mg, 1.24 mmol) in methanol (5.0 mL) was added 10% palladium-carbon (50 mg) and then the mixture was hydrogenated at room temperature overnight under 1 atm of H₂. The reaction mixture was filtered through a celite pad, and the filtrate was concentrated under reduced pressure. The residue was purified by silica gel column chromatography (chloroform/methanol = 97/3), to give the title compound (125 mg, yield 33%).
¹H NMR (CD₃OD 400 MHz): δ= 1.46 6 (9H, s), 1.5-1.7 7 (2H, m), 1.7-1.9 (2H, m), 2.7-3.0 (3H, m), 4.1-4.3 (2H, m), 7.92 (1H, dd, J = 2 Hz, 8 Hz), 8.11 (1H, d, J = 8 Hz), 8.56 (1H, d, J = 2 Hz).

### (3) tert-Butyl 4-[2-(5-methanesulfonylindoline-1-carbonyl)pyridin-5-yl]piperidine-1-carboxylate

To a solution of tert-butyl 4-(2-carboxypyridin-5-yl)piperidine-1-carboxylate (61 mg, 0.199 mmol), 5-methanesulfonylindoline (47 mg, 0.238 mmol), 1-hydroxybenzotriazole monohydrate (34 mg, 0.222 mmol), N-methylmorpholine (61 µL, 0.555 mmol) and 1-(3-dimethylaminopropyl)-3-ethylcarbodiimide hydrochloride (42 mg, 0.219 mmol) in dry N,N-dimethylformamide (5.0 mL) was stirred at room temperature overnight. The reaction mixture was poured into water, and was extracted with ethyl acetate. The organic layer was dried over anhydrous sodium sulfate, and the solvent was removed under reduced pressure. The residue was purified by silica gel column chromatography (hexane/ethyl acetate =1/2 → 1/3), to give the title compound as a white crystal (66 mg, yield 68%) .
FAB-MS (m/z): 486 (M+1)
m.p.: 202-205°C
¹H NMR (CDCl₃ 400 MHz): δ= 1.49 (9H, s), 1.6-1.8 (2H, m), 1.8-1.9 (2H, m), 2.7-3.0 (3H, m), 3.05 (3H, s), 3.23 (2H, t, J = 8 Hz), 4.1-4.4 (2H, m), 4.52 (2H, t, J = 8 Hz), 7.70 0 (1H, br d), 7.7-7.9 (2H, m), 7.88 (1H, d, J = 8 Hz), 8.3-8.6 (2H, m).
IR (KBr, cm⁻¹): 2976, 2931, 1693, 1650, 1591, 1477, 1427, 1390, 1303, 1236, 1171, 1138, 1070, 1007, 947, 883, 854, 827, 762, 719, 669, 629, 573, 521, 490.

### [Example 9]

### tert-Butyl 4-[2-(5-methanesulfonylbenzofuran-2-yl)pyridin-5-yl]piperidine-1-carboxylate

### (1) tert-butyl 4-[2-(4-Bromo-2-formylphenoxymethyl)pyridin-5-yl]piperidine-1-carboxylate

To a solution of tert-butyl 4-[2-(methanesulfonyloxymethyl)pyridin-5-yl]pyridine-1-carboxylate (Example 1 (3)) (78 mg, 0.211 mmol), 5-bromosalicylaldehyde (21 mg, 0.104 mmol), potassium carbonate (29 mg, 0.210 mmol) and potassium iodide (35 mg, 0.211 mmol) in acetonitrile (3.0 mL) was refluxed overnight. After cooling to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 7/3 → 1/9), to give the title compound as a pale yellow oil (34 mg, yield 69%) .
¹H NMR (CDCl₃ 400 MHz): δ= 1.49 (9H, s), 1.5-1.7 7 (2H, m), 1.7-1.9 (2H, m), 2.6-2.8 (1H, m), 2.7-2.9 (2H, m), 4.1-4.4 (2H, m), 5.28 (2H, s), 6.97 (1H, d, J = 8 Hz), 7.44 (1H, d, J = 8 Hz), 7.5-7.7 (2H, m), 7.95 (1H, d, J = 2 Hz), 8.48 (1H, d, J = 2 Hz), 10.5 (1H, s).

### (2) 5-(1-Benzylpiperidin-4-yl)-2-(5-bromobenzofuran-2-yl)pyridine

To a solution of above tert-butyl 6-[2-(4-bromo-2-formylphenoxymethyl)pyridin-5-yl]piperidine-1-carboxylate (55 mg, 0.116 mmol) in dry dichloromethane (3.0 mL) was added trifluoroacetic acid (3.0 mL) and then the mixture was stirred at room temperature for 3 hours. After the solvent was removed under reduced pressure, the residue was dissolved in dry N,N-dimethylformamide (5.0 mL), added benzyl bromide (16.5 µL, 0.139 mmol) and potassium carbonate (48 mg, 0.347 mmol) and the mixture was stirred at 110°C overnight. After cooling to room temperature, the reaction mixture was poured into water, and was extracted with ethyl acetate. The organic layer was washed with brine, dried over anhydrous sodium sulfate, and the solvent was removed under reduced pressure. The residue was added acetic acid (3.0 mL) and then the mixture was refluxed overnight. After cooling to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/2), to give the title compound as a white crystal (29 mg, yield 56%) .
¹H NMR (CDCl₃ 400 MHz): δ= 1.7-1.9 (4H, m), 2.0-2.2 (2H, m), 2.5-2.7 (1H, m), 3.0-3.1 (2H, m), 3.57 (2H, s), 7.2-7.5 (8H, m), 7.64 (1H, dd, J = 1 Hz, 8 Hz), 7.76 (1H, d, J = 1 Hz), 7.81 (1H, d, J = 8 Hz), 8.56 (1H, d, J = 2 Hz).

### (3) 5-(1-Benzylpiperidin-4-yl)-2-(5-methanesulfonylbenzofuran-2-yl)pyridine

A suspension of above 5-(1-benzylpiperidin-4-yl)-2-(5-bromobenzofuran-2-yl)pyridine (29 mg, 64.8 µmol), sodium methanesulfinate (6.5 mg, 64.6 µmol), copper(I) trifluoromethanesulfonate benzene complex (3.2 mg, 6.36 µmol) and N,N'-dimethylethylenediamine (1.4 µL, 13.0 µmol) in dimethylsulfoxide (3.0 mL) was heated at 130°C overnight under N₂. After cooling to room temperature, the mixture was added water and ethyl acetate, and resulting insoluble materials were removed by filtration through a celite pad. The organic layer was washed with brine, dried over anhydrous sodium sulfate, and the solvent was removed under reduced pressure. The residue was purified by silica gel column chromatography, to give the title compound (12 mg, yield 41%) .
¹H NMR (CD₃OD 400 MHz): δ= 1.7-2.0 (4H, m), 2.1-2.3 (2H, m), 2.6-2.8 (1H, m), 3.0-3.1 (2H, m), 3.16 (3H, s), 3.61 (2H, s), 7.2-7.4 (5H, m), 7.61 (1H, s), 7.81 (1H, d, J = 8 Hz), 7.86 (1H, dd, J = 2 Hz, 8 Hz), 7.95 (1H, dd, J = 2 Hz, 8 Hz), 7.99 (1H, d, J = 8 Hz), 8.33 (1H, d, J = 2 Hz), 8.55 (1H, d, J = 2 Hz).

### (4) tert-Butyl 4-[2-(5-methanesulfonylbenzofuran-2-yl)pyridin-5-yl]piperidine-1-carboxylate

To a solution of above 5-(1-benzylpiperidin-4-yl)-2-(5-methanesulfonylbenzofuran-2-yl)pyridine (12 mg, 26.9 µmol) in methanol (2.0 mL) - tetrahydrofuran (2.0 mL) was added 10% palladium-carbon (2.0 mg) and then the mixture was hydrogenated at room temperature overnight under 1 atm of H₂. The reaction mixture was filtered through a celite pad, and the filtrate was concentrated under reduced pressure. To a solution of the residue in water (2.0 mL) - tetrahydrofuran (2.0 mL) was added di-tert-butyl dicarbonate (7 mg, 32.1 µmol) and triethylamine (5 µL, 36.1 µmol) and then the mixture was stirred at room temperature overnight. The reaction mixture was poured into water, and was extracted with ethyl acetate. The organic layer was dried over anhydrous sodium sulfate, and the solvent was removed under reduced pressure. The residue was purified by silica gel column chromatography (hexane/ethyl acetate = 1/2), to give the title compound as a white amorphous (4.2 mg, yield 34%) .
FAB-MS (m/z): 457 (M+1)
¹H NMR (CDCl₃ 400 MHz): δ= 1.50 (9H, s), 1.6-1.8 (2H, m), 1.8-2.0 (2H, m), 2.7-2.9 (3H, m), 3.11 (3H, s), 4.1-4.4 (2H, m), 7.49 (1H, s), 7.66 (1H, dd, J = 2 Hz, 8 Hz), 7.71 (1H, d, J = 8 Hz), 7.88 (1H, d, J = 9 Hz), 7.91 (1H, dd, J = 2 Hz, 9 Hz), 8.28 (1H, d, J = 2 Hz), 8.58 (1H, d, J = 2 Hz).

### [Example 10]

### tert-Butyl 4-[2-(1,1-dioxo-1H-benzo[b]thiophen-5-yloxymethyl)pyridin-5-yl]piperidine-1-carboxylate

### (1) 1,1-Dioxo-1H-benzo[b]thiophen-5-ol

To a solution of Oxone^{®} (1.23 g, 2.00 mmol) in water (10 mL) was added dropwise a solution of 5-hydroxybenzothiophene (200 mg, 1.33 mmol) in methanol (10 mL) over 5 minutes under ice-cooling. After stirring at room temperature for 6 hours, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/1), to give the title compound as a pale pink crystal (234 mg, yield 98%) .
¹H NMR (CD₃OD 400 MHz): δ= 6.8-7.0 0 (3H, m), 7.31 1 (1H, d, J = 7 Hz), 7.51 (1H, d, J = 8 Hz).

### (2) 1,1-Dioxo-1H-benzo[b]thiophen-5-ol potassium salt

To a solution of 1,1-dioxo-1H-benzo[b]thiophen-5-ol (50 mg, 0.28 mmol) in ethanol (1 mL) was added 0.5M potassium hydroxide ethanolic solution (0.6 mL). After stirring at room temperature for 1 hour, the solvent was removed under reduced pressure, to give the title compound as a yellow crystal quantitatively.

### (3) tert-Butyl 4-[2-(1,1-dioxo-1H-benzo[b]thiophen-5-yloxymethyl)pyridin-5-yl]piperidine-1-carboxylate

To a solution of tert-butyl 4-[2-(hydroxymethyl)pyridin-5-yl]pyridine-1-carboxylate (Example 1 (2)) (76 mg, 0.260 mmol) in dichloromethane (2.0 mL) was added triethylamine (54 µL, 0.390 mmol) under ice-cooling. To this was added dropwise a solution of methanesulfonyl chloride (24 µL, 0.310 mmol) in dichloromethane (0.6 mL). After stirring at room temperature for 3 hours, the reaction mixture was poured into water, and was extracted with chloroform. The organic layer was washed with brine, dried over anhydrous sodium sulfate, and the solvent was removed under reduced pressure. To a solution of 1,1-dioxo-1H-benzo[b]thiophen-5-ol potassium salt (57 mg, 0.259 mmol) in dimethylsulfoxide (0.5 mL) was added a solution of the residue in dimethylsulfoxide (0.5 mL). After stirring at room temperature overnight, the reaction mixture was poured into water, and was extracted with ethyl acetate. The organic layer was washed with water and brine, dried over anhydrous sodium sulfate, and the solvent was removed under reduced pressure. The residue was purified by silica gel column chromatography (hexane/ethyl acetate = 1/1 → 1:8, and hexane/tetrahydrofuran = 3/2 → 1:2), to give the title compound as a white crystal (40 mg, yield 34%) .
FAB-MS (m/z): 457 (M+1)
m.p.: 154-157°C
¹H NMR (CDCl₃ 400 MHz): δ= 1.48 (9H, s), 1.5-1.7 7 (2H, m), 1.8-1.9 (2H, m), 2.6-2.9 (3H, m), 4.2-4.4 (2H, m), 5.24 (2H, s), 6.71 (1H, d, J = 7 Hz), 6.95 (1H, d, J = 2 Hz), 7.04 (1H, dd, J = 2 Hz, 8 Hz), 7 . 12 (1H, d, J = 7 Hz), 7.39 (1H, d, J = 8 Hz), 7.56 (1H, dd, J = 2 Hz, 8 Hz), 7.61 (1H, d, J = 8 Hz), 8.48 8 (1H, d, J = 2 Hz).
IR (KBr, cm-¹): 3095, 2970, 2929, 2850, 1689, 1610, 1556, 1427, 1365, 1338, 1302, 1281, 1240, 1171, 1151, 1126, 1074, 1011, 856, 843, 812, 764, 700, 617, 571, 517.

### [Example 11]

### tert-Butyl 4-[2-(7-fluoro-5-methanesulfonylindol-1-ylmethyl)pyridin-5-yl]piperidine-1-carboxylate

### (1) 7-Fluoro-5-methanesulfonylindole

The title compound was prepared from 5-bromo-7-fluoroindole (100 mg, 0.467 mmol) by the similar manner as described in Example 9 (3) as a white crystal (57 mg, yield 57%).
¹H NMR (CDCl₃ 400 MHz): δ= 3.09 (3H, s), 6.75 (1H, dd, J = 3 Hz, 5 Hz), 7.41 (1H, t, J = 3 Hz), 7.47 (1H, dd, J = 1 Hz, 10 Hz), 8.10 (1H, s), 8.83 (1H, d, br s).

### (2) tert-Butyl 4-[2-(7-fluoro-5-methanesulfonylindol-1-ylmethyl)pyridin-5-yl]piperidine-1-carboxylate

The title compound was prepared from 7-fluoro-5-methanesulfonylindole (57 mg, 0.267 mmol) by the similar manner as described in Example 1 (4) as a white amorphous (74 mg, yield 57%) .
FAB-MS (m/z): 488 (M+1)
¹H NMR (CDCl₃ 400 MHz): δ= 1.47 (9H, s), 1.5-1.7 7 (2H, m), 1.7-1.9 (2H, m), 2.5-2.7 (1H, m), 2.7-2.9 (2H, m), 3.07 (3H, s), 4.1-4.4 (2H, m), 5.60 (2H, s), 6.73 (1H, dd, J = 2 Hz, 3 Hz), 6.88 (1H, d, J = 8 Hz), 7.3-7.5 (3H, m), 8.06 (1H, d, J = 1 Hz), 8.43 (1H, d, J = 2 Hz).

### [Example 12]

### tert-Butyl 4-[2-(5-methanesulfonyl-3-methylindol-1-ylmethyl)pyridin-5-yl]piperidine-1-carboxylate

The title compound was prepared from 5-methanesulfonyl-3-methylindole (103 mg, 0.492 mmol) by the similar manner as described in Example 1 (4) as a pale yellow amorphous (78 mg, yield 33%) .
FAB-MS (m/z): 484 (M+1)
¹H NMR (CDCl₃ 400 MHz): δ= 1.47 (9H, s), 1.5-1.7 (2H, m), 1.7-1.9 (2H, m), 2.38 (3H, s), 2.5-2.7 (1H, m), 2.7-2.9 (2H, m), 3.08 (3H, s), 4.1-4.4 (2H, m), 5.40 (2H, s), 6.73 (1H, d, J = 8 Hz), 7.12 (1H, s), 7.3-7.4 (2H, m), 7.69 (1H, dd, J = 2 Hz, 8 Hz), 8.23 (1H, d, J = 1 Hz), 8.45 (1H, d, J = 2 Hz).

### [Example 13]

### tert-Butyl 4-[3-fluoro-2-(5-methanesulfonylindol-1-ylmethyl)pyridin-5-yl]piperidine-1-carboxylate

### (1) tert-Butyl 4-[3-fluoro-2-(hydroxymethyl)pyridin-5-yl]-3,6-dihydro-2H-pyridine-1-carboxylate

The title compound was prepared from 5-bromo-3-fluoropyridine-2-methanol (235 mg, 1.141 mmol) and tert-butyl 4-(4,4,5,5-tetramethyl-1,3,2-dioxaborolan-2-yl)-3,6-dihydro-2H-pyridine-1-carboxylate(353 mg, 1.141 mmol) by the similar manner as described in Example 1 (1) as a yellow oil (342 mg, yield 97%) .
¹H NMR (CDCl₃ 400 MHz): δ= 1.95 (9H, s), 2.4-2.6 (2H, m), 3.66 (2H, t, J = 5 Hz), 3.76 (1H, t, J = 5 Hz), 4.0-4.2 (2H, m), 4.82 (2H, d, J = 4 Hz), 6.14 (1H, br s), 7.35 (1H, dd, J = 2 Hz, 10 Hz), 8.4-8.5 (1H, m).

### (2) tert-Butyl 4-[3-fluoro-2-(hydroxymethyl)pyridin-5-yl]piperidine-1-carboxylate

The title compound was prepared from tert-butyl 4-[3-fluoro-2-(hydroxymethyl)pyridin-5-yl]-3,6-dihydro-2H-pyridine-1-carboxylate (340 mg, 1.10 mmol) by the similar manner as described in Example 8 (2) as a pale yellow crystal (296 mg, yield 86%) .
¹H NMR (CDCl₃ 400 MHz): δ= 1.49 (9H, s), 1.5-1.7 (2H, m), 1.8-1.9 (2H, m), 2.7-2.9 (3H, m), 4.2-4.4 (2H, m), 4.80 (2H, s), 7.22 (1H, d, J = 10 Hz), 8.27 (1H, s).

### (3) tert-Butyl 4-[3-fluoro-2-(methanesulfonyloxymethyl)pyridin-5-yl]piperidine-1-carboxylate

The title compound was prepared from tert-butyl 4-[3-fluoro-2-(hydroxymethyl)pyridin-5-yl]piperidine-1-carboxylate (50 mg, 0.161 mmol) by the similar manner as described in Example 1 (3) as a red oil (29 mg, yield 46%) .
¹H NMR (CDCl₃ 400 MHz): δ= 1.48 (9H, s), 1.5-1.7 7 (2H, m), 1.8-1.9 (2H, m), 2.7-2.9 (3H, m), 3.10 (3H, s), 4.2-4.4 (2H, m), 5.39 (2H, d, J = 2 Hz), 7.30 (1H, dd, J = 2 Hz, 10 Hz), 8.3-8.4 (1H, m).

### (4) tert-Butyl 4-[3-fluoro-2-(5-methanesulfonylindol-1-ylmethyl)pyridin-5-yl]piperidine-1-carboxylate

The title compound was prepared from tert-butyl 4-[3-fluoro-2-(methanesulfonyloxymethyl)pyridin-5-yl]piperidine-1-carboxylate (29 mg, 0.0747 mmol) by the similar manner as described in Example 1 (4) as a white amorphous (21 mg, yield 70%) .
FAB-MS (m/z): 488 (M+1)
¹H NMR (CDCl₃ 400 MHz): δ= 1.47 (9H, s), 1.5-1.7 7 (2H, m), 1.7-1.9 (2H, m), 2.7-2.9 (3H, m), 3.05 (3H, s), 4.2-4.3 (2H, m), 5.48 8 (2H, d, J = 2 Hz), 6.67 (1H, d, J = 3 Hz), 7.23 (1H, dd, J = 2 Hz, 9 Hz), 7.45 (1H, d, J = 3 Hz), 7.6-7.8 (2H, m), 8.2-8.3 (2H, m).

### [Example 14]

### tert-Butyl 4-[3-chloro-2-(5-methanesulfonylindol-1-ylmethyl)pyridin-5-yl]piperidine-1-carboxylate

### (1) (5-Bromo-3-chloropyridin-2-yl)methanol

A solution of 3-chloro-2,5-dibromopyridine (2.26 g, 8.3 mmol) in toluene (83 mL) was cooled to -78°C under N₂. To this was added dropwise 1.6M n-butyllithium/n-hexane solution (6.23 mL, 9.96 mmol). After stirring at -78°C for 2 hours, the reaction mixture was added dry N,N-dimethylformamide (1.29 mL, 16.6 mmol). The reaction mixture was warmed up to room temperature slowly, and added methanol (83 mL) and sodium borohydride (314 mg, 8.3 mmol). After stirring at room temperature 30 minutes, the reaction mixture was added saturated ammonium chloride solution, and was extracted with ethyl acetate. The organic layer was washed with brine, dried over anhydrous sodium sulfate, and the solvent was removed under reduced pressure. The residue was purified by silica gel column chromatography (hexane/ethyl acetate = 4/1), to give the title compound as a pale yellow crystal (967 mg, yield 52%) .
¹H NMR (CDCl₃ 400 MHz): δ= 3.93 (1H, br s), 4.75 (2H, s), 7.86 (1H, d, J = 2 Hz), 8.56 (1H, br s).

### (2) 5-Bromo-2-(tert-butyldimethylsilyloxymethyl)-3-chloropyridine

The title compound was prepared from (5-bromo-3-chloropyridin-2-yl)methanol by the similar manner as described in Example 5 (1) as a colorless oil (1.39 g, yield 100%) .
¹H NMR (CDCl₃ 400 MHz): δ= 0.11 (6H, s), 0.91 (9H, s), 4.86 (2H, s), 7.83 (1H, d, J = 2 Hz), 8.53 (1H, d, J = 2 Hz).

### (3) tert-Butyl 4-[2-(tert-butyldimethylsilyloxymethyl)-3-chloropyridin-5-yl]-3,6-dihydro-2H-pyridine-1-carboxylate

The title compound was prepared from 5-Bromo-2-(tert-butyldimethylsilyloxymethyl)-3-chloropyridine (1.39 g, 4.35 mmol) and tert-butyl 4-(4,4,5,5-tetramethyl-1,3,2-dioxaborolan-2-yl)-3,6-dihydro-2H-pyridin-1-carboxylate(1.35 g, 4.35 mmol) by the similar manner as described in Example 1 (1) as a yellow oil (1.91 g, yield 100%).
¹H NMR (CDCl₃ 400 MHz): δ= 0.12 (6H, s), 0.92 (9H, s), 1.49 (9H, s), 2.4-2.6 (2H, m), 3.64 (2H, t, J = 4 Hz), 4.1-4.1 (2H, m), 4.89 (2H, s), 6.12 (1H, br s), 7.62 (1H, d, J = 2 Hz), 8.50 (1H, d, J = 2 Hz).

### (4) tert-Butyl 4-[3-chloro-2-(hydroxymethyl)pyridin-5-yl]piperidine-1-carboxylate

The title compound was prepared from tert-butyl 4-[2-(tert-butyldimethylsilyloxymethyl)-3-chloropyridin-5-yl]-3,6-dihydro-2H-pyridine-1-carboxylate (179 mg, 0.55 mmol) by the similar manner as described in Example 5 (3) as a colorless oil (91 mg, yield 51%).
¹H NMR (CDCl₃ 400 MHz): δ= 1.49 (9H, s), 1.5-1.7 7 (2H, m), 1.8-1.9 (2H, m), 2.6-2.9 (3H, m), 4.1-4.4 (3H, m), 4.76 (2H, d, J = 4 Hz), 7.52 (1H, d, J = 2 Hz), 8.35 (1H, d, J = 2 Hz).

### (5) tert-Butyl 4-[3-chloro-2-(chloromethyl)pyridin-5-yl]piperidine-1-carboxylate

Above tert-butyl 4-[3-chloro-2-(hydroxymethyl)pyridin-5-yl]piperidine-1-carboxylate (12 mg, 0.037 mmol) was dissolved in dry dichloromethane (0.37 mL) . To this was added dry pyridine (9 µL, 0.111 mmol) and thionyl chloride (3 µL, 0.044 mmol) under ice-cooling, and then the reaction mixture was warmed up to room temperature slowly. After stirring at room temperature for 2 hours, the solvent was removed under reduced pressure. The residue was dissolved in chloroform, and was washed with saturated aqueous sodium hydrogen carbonate solution and brine, dried over anhydrous sodium sulfate, and the solvent was removed under reduced pressure. The residue was purified by silica gel column chromatography (hexane/ethyl acetate = 4/1), to give the title compound as a colorless oil (5 mg, yield 39%) .
¹H NMR (CDCl₃ 400 MHz): δ= 1.48 (9H, s), 1.5-1.7 7 (2H, m), 1.8-1.9 (2H, m), 2.6-2.9 (3H, m), 4.2-4.4 (2H, m), 4.79 (2H, s), 7.5-7.6 (1H, m), 8.3-8.4 (1H, m).

### (6) tert-Butyl 4-[3-chloro-2-(5-methanesulfonylindol-1-ylmethyl)pyridin-5-yl]piperidine-1-carboxylate

The title compound was prepared from tert-butyl 4-[3-chloro-2-(chloromethyl)pyridin-5-yl]piperidine-1-carboxylate (30 mg, 0.087 mmol) by the similar manner as described in Example 1 (4) as a pale yellow amorphous (37 mg, yield 84%).
FAB-MS (m/z): 488 (M+1)
¹H NMR (CDCl₃ 400 MHz): δ= 1.47 (9H, s), 1.5-1.7 7 (2H, m), 1.7-1.9 (2H, m), 2.6-2.9 (3H, m), 3.05 (3H, s), 4.1-4.4 (2H, m), 5.56 (2H, s), 6.68 (1H, d, J = 3 Hz), 7.44 (1H, d, J = 3 Hz), 7.52 (1H, d, J = 2 Hz), 7.66 (1H, d, J = 8 Hz), 7.71 (1H, dd, J = 2 Hz, 8 Hz), 8.25 (1H, d, J = 2 Hz), 8.31 (1H, d, J = 2Hz).

### [Example 15]

### tert-Butyl 4-[3-chloro-2-(7-fluoro-5-methanesulfonylindol-1-ylmethyl)pyridin-5-yl]piperidine-1-carboxylate

The title compound was prepared from tert-butyl 4-[3-chloro-2-(hydroxymethyl)pyridin-5-yl]piperidine-1-carboxylate (Example 14 (4)) by the similar manner as described in Example 5 (4) as a white amorphous.
FAB-MS (m/z): 522 (M+1)
¹H NMR (CDCl₃ 400 MHz): δ= 1.47 (9H, s), 1.5-1.7 7 (2H, m), 1.7-1.9 (2H, m), 2.6-2.9 (3H, m), 3.07 (3H, s), 4.1-4.4 (2H, m), 5.73 (2H, s), 6.72 (1H, t, J = 3 Hz), 7.2-7.3 (1H, m), 7.39 (1H, dd, J = 1 Hz, 8 Hz), 7.54 (1H, d, J = 2 Hz), 8.06 (1H, d, J = 1 Hz), 8.21 (1H, d, J = 2 Hz).

### [Example 16]

### 5-Isopropyl-3-[4-[2-(5-methanesulfonylindol-1-ylmethyl)pyridin-5-yl]piperidin-1-yl]-[1,2,4]oxadiazole

### (1) 4-[2-(5-Methanesulfonylindol-1-ylmethyl)pyridin-5-yl]piperidine

To a solution of tert-butyl 4-[2-(5-methanesulfonylindol-1-ylmethyl)pyridin-5-yl]piperidine-1-carboxylate (Example 1) (92 mg, 0.196 mmol)in dichloromethane (1 mL) was added trifluoroacetic acid (1 mL) and the mixture was stirred at room temperature overnight. After the solvent was removed under reduced pressure, added saturated sodium hydrogen carbonate solution, and was extracted with chloroform. The organic layer was dried over anhydrous sodium sulfate, and the solvent was removed under reduced pressure, to give the title compound as a pale yellow amorphous (70 mg, yield 97%) .
¹H NMR (CDCl₃ 400 MHz): δ= 1.5-1.7 (2H, m), 1.7-1.8 (2H, m), 2.5-2.7 (1H, m), 2.7-2.8 (2H, m), 3.07 (3H, s), 3.1-3.3 (2H, m), 5.47 (2H, s), 6.7-6.8 (2H, m), 7.37 (1H, d, J = 3 Hz), 7.42 (1H, dd, J = 2 Hz, 8 Hz), 7.44 (1H, d, J = 8 Hz), 7.70 (1H, dd, J = 2 Hz, 8 Hz), 8.29 (1H, d, J = 2 Hz), 8.46 6 (1H, d, J = 2 Hz) .

### (2) 4-[2-(5-Methanesulfonylindol-1-ylmethyl)pyridin-5-yIJpiperidine-1-carbonitrile

To a solution of 4-[2-(5-methanesulfonylindol-1-ylmethyl)pyridin-5-yl]piperidine (70 mg, 0.190 mmol) in dichloromethane (1 mL) was added a solution of sodium hydrogen carbonate (32 mg, 0.379 mmol) in water (0.5 mL). To this was added a solution of cyanogen bromide (24 mg, 0.227 mmol) in dichloromethane (1 mL) under ice-cooling and stirred at 0°C for 30 minutes. After stirring at room temperature for an additional 2 hours, the reaction mixture was poured into saturated aqueous sodium hydrogen carbonate solution, and was extracted with chloroform. The organic layer was dried over anhydrous sodium sulfate, and the solvent was removed under reduced pressure. The residue was purified by silica gel column chromatography (hexane/ethyl acetate =1/2 → 0/100), to give the title compound (65 mg, yield 87%) .
¹H NMR (CDCl₃ 400 MHz): δ= 1.7-1.9 (4H, m), 2.6-2.7 (1H, m), 3.07 (3H, s), 3.1-3.2 (2H, m), 3.5-3.6 (2H, m), 5.48 (2H, s), 6.7-6.8 (2H, m), 7.3-7.5 (3H, m), 7.71 (1H, dd, J = 2 Hz, 8 Hz), 8.29 (1H, d, J = 2 Hz), 8.46 (1H, d, J = 2 Hz).

### (3) N-Hydroxy-4-[2-(5-methanesulfonylindol-1-ylmethyl)pyridin-5-yl]piperidine-1-carboxamidine

To a solution of 4-[2-(5-methanesulfonylindol-1-ylmethyl)pyridin-5-yl]piperidine-1-carbonitrile (65 mg, 0.165 mmol) in ethanol (0.8 mL) was added 50% hydroxylamine solution (0.13 mL) and the mixture was stirred at 60°C for 3 hours. After cooling to room temperature, the solvent was removed under reduced pressure, to give the title compound (70 mg, yield 99%) .
FAB-MS (m/z): 428 (M+1)

### (4) 5-Isopropyl-3-[4-[2-(5-methanesulfonylindol-1-ylmethyl)pyridin-5-yl]piperidin-1-yl]-[1,2,4]oxadiazole

To a solution of N-hydroxy-4-[2-(5-methanesulfonylindol-1-ylmethyl)pyridin-5-yl]piperidine-1-carboxamidine (70 mg, 0.164 mmol), isobutyric acid (15 µL, 0.164 mmol) and 1-hydroxybenzotriazole monohydrate (28 mg, 0.180 mmol) in N,N-dimethylformamide (2 mL) was added N,N-diisopropylethylamine (94 µL, 0.540 mmol) and 1-(3-dimethylaminopropyl)-3-ethylcarbodiimide hydrochloride (38 mg, 0.196 mmol) under ice-cooling. After stirring at room temperature overnight, the reaction mixture was poured into saturated aqueous sodium hydrogen carbonate solution, and was extracted with ethyl acetate. The organic layer was dried over anhydrous sodium sulfate, and the solvent was removed under reduced pressure. A suspension of the residue in toluene (4 mL) was refluxed for 2 hours. After cooling to room temperature, the solvent was removed under reduced pressure. The residue was purified by silica gel column chromatography (hexane/ethyl acetate = 1/2 → 0/100), to give the title compound as a white amorphous (35 mg, yield 45%) .
FAB-MS (m/z): 480 (M+1)
¹H NMR (CDCl₃ 400 MHz): δ= 1.35 (6H, d, J = 7 Hz), 1.6-1.8 (2H, m), 1.8-1.9 (2H, m), 2.6-2.8 (1H, m), 2.9-3.2 (3H, m), 3.07 (3H, s), 4.1-4.2 (2H, m), 5.47 (2H, s), 6.7-6.8 (2H, m), 7.37 (1H, d, J = 2 Hz), 7.42 (1H, dd, J = 2 Hz, 8 Hz), 7.43 (1H, d, J = 8 Hz), 7.71 1 (1H, dd, J = 2 Hz, 8 Hz), 8.29 (1H, d, J = 2 Hz), 8.48 8 (1H, d, J = 2 Hz).

### [Example 17]

### tert-Butyl 4-[2-(5-methanesulfonyl-1H-pyrrolo[2,3-blpyridin-1-ylmethyl)pyridin-5-yl]piperidine-1-carboxylate

The title compound was prepared from 5-methanesulfonyl-1H-pyrrolo[2,3-b]pyridine by the similar manner as described in Example 1 (4) as a pale yellow amorphous.
FAB-MS (m/z): 471 (M+1)
¹H NMR (CDCl₃ 400 MHz): δ= 1.47 (9H, s), 1.5-1.7 7 (2H, m), 1.7-1.9 (2H, m), 2.6-2.9 (3H, m), 3.12 (3H, s), 4.1-4.4 (2H, m), 5.64 (2H, s), 6.67 (1H, d, J = 3 Hz), 7.05 (1H, J = 8 Hz), 7.43 (1H, dd, J = 2 Hz, 8 Hz), 7.54 (1H, d, J = 3 Hz), 8.43 (1H, d, J = 2 Hz), 8.49 (1H, d, J = 2 Hz), 8.86 (1H, d, J = 2 Hz).

### [Example 18]

### tert-Butyl 4-[2-(5-methanesulfonyl-7-nitroindol-1-ylmethyl)pyridin-5-yl]piperidine-1-carboxylate

Under N₂ atmosphere, to a solution of tert-butyl 4-[2-(hydroxymethyl)pyridin-5-yl]pyridine-1-carboxylate (100 mg, 0.342 mmol) in dichloromethane (5 mL) was added triethylamine (71 µL, 0.512 mmol) under ice-cooling. To this was added dropwise methanesulfonyl chloride (29 µL, 0.375 mmol). After stirring at room temperature for 2 hours, the reaction mixture was added water and was extracted with dichloromethane. The organic layer was washed with brine, dried over anhydrous sodium sulfate, and the solvent was removed under reduced pressure, to give the crude tert-butyl 4-[2-(methanesulfonyloxymethyl)pyridin-5-yl]piperidine-1-carboxylate.

A suspension of above tert-butyl 4-[2-(methanesulfonyloxymethyl)pyridin-5-yl]piperidine-1-carboxylate, 5-methanesulfonyl-7-nitroindole (66 mg, 0.275 mmol), potassium hydroxide (16 mg, 0.285 mmol) and 18-crown-6-ether (73 mg, 0.276 mmol) in dry toluene (5 mL) was stirred at 80°C overnight. After cooling to room temperature, the reaction mixture was added water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/3), to give the title compound as a pale red amorphous (33 mg, yield 23%).
FAB-MS (m/z): 515 (M+1)
¹H NMR (CDCl₃ 400 MHz): δ= 1.46 (9H, s), 1.4-1.6 (2H, m), 1.7-1.9 (2H, m), 2.5-2.7 (1H, m), 2.6-2.9 (2H, m), 3.12 (3H, s), 4.1-4.4 (2H, m), 5.61 (2H, s), 6.90 (1H, d, J = 3 Hz), 6.96 (1H, d, J = 8 Hz), 7.43 (1H, dd, J = 2 Hz, 8 Hz), 7.51 (1H, d, J = 3 Hz), 8.27 (1H, d, J = 2 Hz), 8.30 (1H, d, J = 2 Hz), 8.47 (1H, d, J = 2 Hz).

### [Example 19]

### tert-Butyl 4-[2-(7-amino-5-methanesulfonylindol-1-ylmethyl)pyridin-5-yl]piperidine-1-carboxylate

To a solution of tert-butyl 4-[2-(5-methanesulfonyl-7-nitroindol-1-ylmethyl)pyridin-5-yl]piperidine-1-carboxylate (Example 18) (20 mg, 0.0389 mmol) in methanol (3.0 mL) was added platinum oxide (0.6 mg) and then the mixture was hydrogenated at room temperature for 5 hours under 1 atm of H₂. The reaction mixture was filtered through a celite pad, and the filtrate was concentrated under reduced pressure. The residue was purified by silica gel column chromatography (hexane/ethyl acetate = 1/3), to give the title compound as a pale yellow oil (8 mg, yield 43%) .
FAB-MS (m/z): 485 (M+1)
¹H NMR (CDCl₃ 400 MHz): δ= 1.46 (9H, s), 1.4-1.7 (2H, m), 1.7-1.9 (2H, m), 2.6-2.8 (1H, m), 2.7-2.9 (2H, m), 3.03 (3H, s), 4.1-4.44 (2H, m), 5.13 (2H, br s), 5.65 (2H, s), 6.57 (1H, d, J = 3 Hz), 6.96 (1H, d, J = 1 Hz), 7.19 (1H, d, J = 3 Hz), 7.22 (1H, d, J = 8 Hz), 7.52 (1H, dd, J = 2 Hz, 8 Hz), 7.68 (1H, d, J = 1 Hz), 8.42 (1H, d, J = 2 Hz).

### [Example 20]

### tert-Butyl 4-[2-[5-(tetrazol-1-yl)indol-1-ylmethyl]pyridin-5-yl]piperidine-1-carboxylate

### (1) 5-(Tetrazol-1-yl)indole

A solution of 5-aminoindole (300 mg, 2.27 mmol) in acetic acid (4 mL) was added triethyl orthoformate (2 mL) and sodium azide (738 mg, 11.35 mmol), and the mixture was stirred at 80°C for 2 hours. After cooling to room temperature, the reaction mixture was poured into water, and was extracted with ethyl acetate. The organic layer was washed with water, saturated aqueous sodium hydrogen carbonate solution and brine, dried over anhydrous sodium sulfate, and the solvent was removed under reduced pressure. The residue was purified by silica gel column chromatography (hexane/ethyl acetate = 1/1), to give the title compound as a white crystal (237 mg, yield 56%).
¹H NMR (CDCl₃ 400 MHz): δ= 6.65 (1H, d, J = 3 Hz), 7.38 8 (1H, d, J = 3 Hz), 7.44 (1H, dd, J = 2 Hz, 9 Hz), 7.56 (1H, d, J = 9 Hz), 7.90 (1H, d, J = 2 Hz), 9.03 (1H, s), 9.68 (1H, br s).

### (2) tert-Butyl 4-[2-[5-(tetrazol-1-yl)indol-1-ylmethyl]pyridin-5-yl]piperidine-1-carboxylate

The title compound was prepared from 5-(tetrazol-1-yl)indole by the similar manner as described in Example 18 as a brown amorphous.
FAB-MS (m/z): 460 (M+1)
¹H NMR (CDCl₃ 400 MHz): δ= 1.47 (9H, s), 1.5-1.7 7 (2H, m), 1.7-1.9 (2H, m), 2.6-2.7 (1H, m), 2.7-2.9 (2H, m), 4.1-4.4 (2H, m), 5.49 (2H, s), 6.70 (1H, d, J = 3 Hz), 6.75 (1H, d, J = 8 Hz), 7.3-7.5 (4H, m), 7.91 (1H, d, J = 2 Hz), 8.47 (1H, d, J = 2 Hz), 8.94 (1H, s).

### [Example 21]

### tert-Butyl 4-[2-(5-methanesulfonylindazol-1-ylmethyl)pyridin-5-yl]piperidine-1-carboxylate

### [Example 22]

### tert-Butyl 4-[2-(5-methanesulfonylindazol-2-ylmethyl)pyridin-5-yl]piperidine-1-carboxylate

### (1) 5-Methanesulfonylindazole

A solution of 5-bromo-1H-indazole (300 mg, 1.52 mmol), sodium methanesulfinate (155 mg, 1.52 mmol), copper(I) trifluoromethanesulfonate benzene complex (77 mg, 0.152 mmol) and N,N'-dimethylethylenediamine (33 µL, 0.304 mmol) in dimethylsulfoxide (15 mL) was heated at 130°C overnight. After cooling to room temperature, the reaction mixture was poured into water, and was extracted with ethyl acetate. The organic layer was washed with water and brine, dried over anhydrous sodium sulfate, and the solvent was removed under reduced pressure. The residue was purified by silica gel column chromatography (hexane/ethyl acetate = 1/2), to give the title compound (113 mg, yield 38%) .
¹H NMR (CDCl₃ 400 MHz): δ= 3.06 (3H, s), 7.67 (1H, d, J = 9 Hz), 7.94 (1H, dd, J = 2 Hz, 9 Hz), 8.25 (1H, s), 8.48 (1H, d, J = 2 Hz), 10.34 (1H, br s).

### (2) tert-Butyl 4-[2-(5-methanesulfonylindazol-1-ylmethyl)pyridin-5-yl]piperidine-1-carboxylate

### (3) tert-Butyl 4-[2-(5-methanesulfonylindazol-2-ylmethyl)pyridin-5-yl]piperidine-1-carboxylate

tert-Butyl 4-[2-(5-methanesulfonylindazol-1-ylmethyl)pyridin-5-yl]piperidine-1-carboxylate and tert-butyl 4-[2-(5-methanesulfonylindazol-2-ylmethyl)pyridin-5-yl]piperidine-1-carboxylate were prepared from 5-methanesulfonylindazole by the similar manner as described in Example 18.
tert-Butyl 4-[2-(5-methanesulfonylindazol-1-ylmethyl)pyridin-5-yl]piperidine-1-carboxylate
FAB-MS (m/z): 471 (M+1)
¹H NMR (CDCl₃ 400 MHz): δ= 1.47 (9H, s), 1.5-1.7 7 (2H, m), 1.7-1.9 (2H, m), 2.6-2.7 (1H, m), 2.7-2.9 (2H, m), 3.08 (3H, s), 4.1-4.4 (2H, m), 5.74 (2H, s), 6.96 (1H, d, J = 8 Hz), 7.43 (1H, dd, J = 2 Hz, 8 Hz), 7.63 (1H, d, J = 9 Hz), 7.87 (1H, dd, J = 2 Hz, 9 Hz), 8.23 (1H, s), 8.4-8.5 (2H, m).

### tert-Butyl 4-[2-(5-methanesulfonylindazol-2-ylmethyl)pyridin-5-yl]piperidine-1-carboxylate

FAB-MS (m/z): 471 (M+1)
¹H NMR (CDCl₃ 400 MHz): δ= 1.47 (9H, s), 1.5-1.7 7 (2H, m), 1.7-1.9 (2H, m), 2.6-2.7 (1H, m), 2.7-2.9 (2H, m), 3.07 (3H, s), 4.1-4.4 (2H, m), 5.73 (2H, s), 7.20 (1H, d, J = 8 Hz), 7.51 (1H, dd, J = 2 Hz, 8 Hz), 7.71 (1H, dd, J = 1 Hz, 9 Hz), 7.85 (1H, d, J = 9 Hz), 8.35 (1H, s), 8.43 (1H, d, J = 1 Hz), 8.46 6 (1H, d, J = 2 Hz).

### [Example 23]

### tert-Butyl 4-[2-(4-fluoro-5-methanesulfonylindol-1-ylmethyl)pyridin-5-yl]piperidine-1-carboxylate

The title compound was prepared from 4-fluoro-5-methanesulfonylindole by the similar manner as described in Example 18 as a pale brown amorphous.
FAB-MS (m/z): 488 (M+1)
¹H NMR (CDCl₃ 400 MHz): δ= 1.47 (9H, s), 1.5-1.7 7 (2H, m), 1.7-1.9 (2H, m), 2.6-2.9 (3H, m), 3.24 (3H, s), 4.1-4.4 (2H, m), 5.44 (2H, s), 6.77 (1H, d, J = 8 Hz), 6.80 (1H, d, J = 3 Hz), 7.20 (1H, d, J = 8 Hz), 7.31 (1H, d, J = 3 Hz), 7.41 (1H, dd, J = 2 Hz, 8 Hz), 7.68 (1H, dd, J = 7 Hz, 8 Hz), 8.45 (1H, d, J = 2 Hz).

### [Example 24]

### tert-Butyl 4-[2-(6-fluoro-5-methanesulfonylindol-1-ylmethyl)pyridin-5-yl]piperidine-1-carboxylate

The title compound was prepared from 6-fluoro-5-methanesulfonylindol by the similar manner as described in Example 18 as a pale yellow amorphous.
FAB-MS (m/z): 488 (M+1)
¹H NMR (CDCl₃ 400 MHz): δ= 1.47 (9H, s), 1.5-1.9 (4H, m), 2.6-2.9 (3H, m), 3.22 (3H, s), 4.1-4.4 (2H, m), 5.39 (2H, s), 6.68 (1H, d, J = 3 Hz), 6.77 (1H, d, J = 8 Hz), 7.14 (1H, d, J = 11 Hz), 7.32 (1H, d, J = 3 Hz), 7.42 (1H, dd, J = 2 Hz, 8 Hz), 8.22 (1H, d, J = 7 Hz), 8.46 (1H, d, J = 2 Hz).

### [Example 25]

### tert-Butyl 4-[2-(7-fluoro-5-methanesulfonylindolin-1-ylmethyl)pyridin-5-yl]piperidine-1-carboxylate

### (1) 7-Fluoro-5-methanesulfonylindoline

The title compound was prepared from 5-bromo-7-fluoroindoline (75 mg, 0.347 mmol) by the similar manner as described in Example 22 (1) as a pale yellow crystal (18 mg, yield 24%) .
¹H NMR (CDCl₃ 400 MHz): δ= 3.01 (3H, s), 3.15 (2H, t, J = 9 Hz), 3.78 (2H, t, J = 9 Hz), 4.30 (1H, br s), 7.41 (1H, d, J = 9 Hz), 7.43 (1H, s).

### (2) tert-Butyl 4-[2-(7-fluoro-5-methanesulfonylindolin-1-ylmethyl)pyridin-5-yl]piperidine-1-carboxylate

The title compound was prepared from 7-fluoro-5-methanesulfonylindoline (33 mg, 0.142 mmol) by the similar manner as described in Example 18 as a pale yellow amorphous (70 mg, yield 97%) .
FAB-MS (m/z): 490 (M+1)
¹H NMR (CDCl₃ 400 MHz): δ= 1.48 (9H, s), 1.5-1.7 7 (2H, m), 1.8-1.9 (2H, m), 2.6-2.9 (3H, m), 3.01 (3H, s), 3.11 (2H, t, J = 9 Hz), 3.67 (2H, t, J = 9 Hz), 4.2-4.3 (2H, m), 4.71 (2H, s), 7.24 (1H, d, J = 8 Hz), 7.36 (1H, d, J = 2 Hz), 7.40 (1H, dd, J = 2 Hz, 12 Hz), 7.50 (1H, dd, J = 2 Hz, 8 Hz), 8.43 (1H, d, J = 2 Hz).

### [Example 26]

### tert-Butyl 4-[2-(5-methanesulfonyl-7-methylindol-1-ylmethyl)pyridin-5-yl]piperidine-1-carboxylate

### (1) 5-Methanesulfonyl-7-methylindole

The title compound was prepared from 5-bromo-7-methylindole (98 mg, 0.467 mmol) by the similar manner as described in Example 22 (1) as a white crystal (73 mg, yield 75%).
¹H NMR (CDCl₃ 400 MHz): δ= 2.57 (3H, s), 3.08 (3H, s), 6.6-6.8 (1H, m), 7.3-7.4 (1H, m), 7.5-7.6 (1H, m), 8.1-8.2 (1H, m), 8.60 (1H, br s).

### (2) tert-Butyl 4-[2-(5-methanesulfonyl-7-methylindol-1-ylmethyl)pyridin-5-yl]piperidine-1-carboxylate

The title compound was prepared from 5-methanesulfonyl-7-methylindole by the similar manner as described in Example 18 as a white amorphous.
FAB-MS (m/z): 484 (M+1)
¹H NMR (CDCl₃ 400 MHz): δ= 1.47 (9H, s), 1.5-1.7 7 (2H, m), 1.7-1.9 (2H, m), 2.57 (3H, s), 2.6-2.8 (1H, m), 2.7-2.9 (2H, m), 3.07 (3H, s), 4.1-4.4 (2H, m), 5.69 (2H, s), 6.43 (1H, d, J = 8 Hz), 6.73 (1H, d, J = 3 Hz), 7.25 (1H, d, J = 3 Hz), 7.37 (1H, dd, J = 2 Hz, 8 Hz), 7.4-7.5 (1H, m), 8.14 (1H, d, J = 2 Hz), 8.46 (1H, d, J = 2 Hz).

### [Example 27]

### tert-Butyl 4-[2-(7-chloro-5-methanesulfonylindol-1-ylmethyl)pyridin-5-yl]piperidine-1-carboxylate

The title compound was prepared from 7-chloro-5-methanesulfonylindol by the similar manner as described in Example 18 as a pale yellow amorphous.
FAB-MS (m/z): 504 (M+1)
¹H NMR (CDCl₃ 400 MHz): 5= 1.47 (9H, s), 1.5-1.7 (2H, m), 1.7-1.9 (2H, m), 2.6-2.8 (1H, m), 2.7-2.9 (2H, m), 3.09 (3H, s), 4.1-4.4 (2H, m), 5.88 (2H, s), 6.67 (1H, d, J = 8 Hz), 6.76 (1H, d, J = 3 Hz), 7.35 (1H, d, J = 3 Hz), 7.40 (1H, dd, J = 2 Hz, 8 Hz), 7.68 (1H, d, J = 1 Hz), 8.17 (1H, d, J = 1 Hz), 8.44 (1H, d, J = 2 Hz).

### [Example 28]

### tert-Butyl 4-[2-(5-methanesulfonyl-7-methyl-1H-pyrrolo[2,3-c]pyridin-1-ylmethyl)pyridin-5-yl]piperidine-1-carboxylate

### (1) 5-Methanesulfonyl-7-methyl-1H-pyrrolo[2,3-c]pyridine

Under N₂ atmosphere, to a solution of 6-methanesulfonyl-2-methyl-3-nitropyridine (200 mg, 0.926 mmol) in tetrahydrofuran (5 mL) was added dropwise vinylmagnesium bromide (1.0M tetrahydrofuran solution, 2.8 mL, 2.8 mmol) at -78°C. After stirring at -20°C for 3 hours, the reaction mixture was added saturated aqueous ammonium chloride solution, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/3, and chloroform/methanol = 100/1), to give the title compound as a pale red crystal (34 mg, yield 18%) .
¹H NMR (CDCl₃ 400 MHz): δ= 2.68 8 (3H, s), 3.23 (3H, s), 6.6-6.7 (1H, m), 7.49 (1H, t, J = 3 Hz), 8.20 (1H, s), 9.63 (1H, br s).

### (2) tert-Butyl 4-[2-(5-methanesulfonyl-7-methyl-1H-pyrrolo[2,3-c]pyridin-1-ylmethyl)pyridin-5-yl]piperidine-1-carboxylate

The title compound was prepared from 5-methanesulfonyl-7-methyl-1H-pyrrolo[2,3-c]pyridine by the similar manner as described in Example 18 as a pale yellow amorphous.
FAB-MS (m/z): 485 (M+1)
¹H NMR (CDCl₃ 400 MHz): δ= 1.48 (9H, s), 1.5-1.7 7 (2H, m), 1.7-1.9 (2H, m), 2.6-2.9 (3H, m), 2.78 (3H, s), 3.21 (3H, s), 4.1-4.4 (2H, m), 5.72 (2H, s), 6.49 (1H, d, J = 8 Hz), 6.77 (1H, d, J = 3 Hz), 7.40 (1H, dd, J = 2 Hz, 8 Hz), 7.42 (1H, d, J = 3 Hz), 8.27 (1H, s), 8.46 (1H, d, J = 2 Hz).

### [Example 29]

### tert-Butyl 4-[2-(5-methanesulfonyl-1H-pyrrolo[2,3-c]pyridin-1-ylmethyl)pyridin-5-yl]piperidine-1-carboxylate

### (1) 2-(2-Bromo-5-nitropyridin-4-yl)-N,N-dimethylethenamine

A solution of 2-bromo-4-methyl-5-nitropyridine (50 mg, 0.23 mmol) and dimethylformamide dimethyl acetal (0.25 mL, 1.87 mmol) in N,N-dimethylformamide (1 mL) was stirred at 60°C for 1.5 hours under N₂. After cooling to room temperature, the reaction mixture was poured into water, and was extracted with ethyl acetate. The organic layer was washed with water and brine, dried over anhydrous sodium sulfate, and the solvent was removed under reduced pressure, to give the title compound as a deep red crystal (52 mg, yield 830).
H NMR (CDCl₃ 400 MHz): δ= 2.8-3.2 (6H, m), 5.93 (1H, d, J = 13 Hz), 7.32 (1H, d, J = 13 Hz), 7.42 (1H, s), 8.74 (1H, s).

### (2) 5-Bromo-1H-pyrrolo[2,3-c]pyridine

Under N₂, titanium(IV) chloride (75 µL, 0.69 mmol) was added dropwise to tetrahydrofuran (2 mL) at 0°C, then lithium aluminium hydride (19 mg, 0.50 mmol) was portionwise added to this mixture and stirred for 15 minutes. To this was added dropwise a solution of 2-(2-bromo-5-nitropyridin-4-yl)-N,N-dimethylethenamine in tetrahydrofuran (1.5 mL) over 5 minutes, and the mixture was stirred at room temperature for 50 minutes. The reaction mixture was added 25% ammonia solution (0.5 mL) slowly, and was extracted with ethyl acetate. The organic layer was washed with water and brine, dried over anhydrous sodium sulfate, and the solvent was removed under reduced pressure. The residue was purified by silica gel column chromatography (hexane/ethyl acetate = 2/3), to give the title compound as a pale yellow crystal (15 mg, yield 39%).
¹H NMR (CDCl₃ 400 MHz): δ= 6.5-6.6 (1H, m), 7.42 (1H, t, J = 3 Hz), 7.73 (1H, s), 8.59 (1H, s), 8.63 (1H, br s).

### (3) 5-Methanesulfonyl-1H-pyrrolo[2,3-c]pyridine

The title compound was prepared from 5-bromo-1H-pyrrolo[2,3-c]pyridine (21 mg, 0.11 mmol) by the similar manner as described in Example 22 (1) as a pale yellow crystal (6 mg, yield 27%) .
¹HNMR (CD₃OD 400 MHz): δ= 3.19 (3H, s), 6.80 (1H, d, J = 3 Hz), 7.70 (1H, d, J = 3 Hz), 8.37 (1H, s), 8.83 (1H, s) .

### (4) tert-Butyl 4-[2-(5-methanesulfonyl-1H-pyrrolo[2,3-clpyridin-1-ylmethyl)pyridin-5-yl]piperidine-1-carboxylate

The title compound was prepared from 5-methanesulfonyl-1H-pyrrolo[2,3-c]pyridine by the similar manner as described in Example 18 as a pale yellow amorphous.
FAB-MS (m/z): 471 (M+1)
¹H NMR (CDCl₃ 400 MHz): δ= 1.47 (9H, s), 1.5-1.9 (4H, m), 2.46 (3H, s), 2.6-2.9 (3H, m), 4.1-4.4 (2H, m), 5.55 (2H, s), 6.77 (1H, d, J = 3 Hz), 6.89 (1H, d, J = 8 Hz), 7.44 (1H, dd, J = 2 Hz, 8 Hz), 7.55 (1H, d, J = 3 Hz), 8.40 (1H, s), 8.45 (1H, d, J = 2 Hz), 8.80 (1H, s).

### [Example 30]

### 5-Ethyl-2-[4-[2-(5-methanesulfonylindol-1-ylmethyl)pyridin-5-yl]piperidin-1-yl]pyrimidine

To a solution of tert-butyl 4-[2-(5-methanesulfonylindol-1-ylmethyl)pyridin-5-yl]piperidine-1-carboxylate (Example 1) (17 mg, 0.037 mmol)in dichloromethane (1 mL) was added trifluoroacetic acid (0.2 mL). After stirring at room temperature for 1.5 hours, the solvent was removed under reduced pressure. The resulting crude 4-[2-(5-methanesulfonylindol-1-ylmethyl)pyridin-5-yl]piperidine was dissolved in dry acetonitrile (1 mL),and was added potassium carbonate (26 mg, 0.185 mmol) and 2-bromo-5-ethylpyrimidine (9 µL, 0.074 mmol). After stirring at 80°C for 16 hours, cooled to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/3), to give the title compound as a white amorphous (14 mg, yield 78%) .
FAB-MS (m/z): 476 (M+1)
¹H NMR CDCl₃ 400 MHz): δ= 1.18 (3H, t, J = 8 Hz), 1.6-1.8 (2H, m), 1.9-2.0 0 (2H, m), 2.46 6 (2H, q, J = 8 Hz), 2.8-2.9 (1H, m), 2.9-3.0 (2H, m), 3.06 (3H, s), 4.8-4.9 (2H, m), 5.46 (2H, s), 6.7-6.8 (2H, m), 7.36 (1H, d, J = 3 Hz), 7.40 (1H, dd, J = 2 Hz, 8 Hz), 7.44 (1H, d, J = 8 Hz), 7.70 (1H, dd, J = 1 Hz, 8 Hz), 8.18 (2H, s), 8.29 (1H, d, J = 1 Hz), 8.47 (1H, d, J = 2 Hz).

### [Example 31]

### tert-Butyl 4-[2-(6-chloro-5-methanesulfonylindol-1-ylmethyl)pyridin-5-yl]piperidine-1-carboxylate

### (1) 5-Bromo-6-chloroindole

The title compound was prepared from 5-bromo-4-chloro-2-nitrotoluene (2.00 g, 7.98 mmol) by the similar manner as described in Example 29 (1) and Example 29 (2) as a pale brown crystal (555 mg, yield 30%)).
¹H NMR (CDCl₃ 400 MHz): δ= 6.4-6.5 (1H, m), 7.2-7.3 (1H, m), 7.52 (1H, s), 7.89 (1H, s), 8.17 (1H, br s).

### (2) 6-Chloro-5-methanesulfonylindole

The title compound was prepared from 5-bromo-6-chloroindole (300 mg, 1.3 mmol) by the similar manner as described in Example 22 (1) as a pale brown crystal (44 mg, yield 15%).
¹H NMR (CDCl₃ 400 MHz): δ= 3.30 (3H, s), 6.6-6.7 (1H, m), 7.3-7.4 (1H, m), 7.54 (1H, s), 8.47 (1H, s), 8.70 (1H, br s) .

### (3) tert-Butyl 4-[2-(6-chloro-5-methanesulfonylindol-1-ylmethyl)pyridin-5-yl]piperidine-1-carboxylate

The title compound was prepared from 6-chloro-5-methanesulfonylindole by the similar manner as described in Example 18 as a pale yellow amorphous.
FAB-MS (m/z): 504 (M+1)
¹H NMR (CDCl₃ 400 MHz): δ= 1.47 (9H, s), 1.5-1.7 7 (2H, m), 1.7-1.9 (2H, m), 2.6-2.7 (1H, m), 2.7-2.9 (2H, m), 3.29 (3H, s), 4.1-4.4 (2H, m), 5.41 (2H, s), 6.72 (1H, d, J = 3 Hz), 6.77 (1H, d, J = 8 Hz), 7.34 (1H, d, J = 3 Hz), 7.42 (1H, dd, J = 2 Hz, 8 Hz), 7.46 (1H, s), 8.46 (1H, d, J = 2 Hz), 8.49 (1H, s).

### [Example 32]

### 5-Ethyl-2-[4-[2-(6-fluoro-5-methanesulfonylindol-1-ylmethyl)pyridin-5-yl]piperidin-1-yl]pyrimidine

The title compound was prepared from tert-butyl 4-[2-(6-fluoro-5-methanesulfonylindol-1-ylmethyl)pyridin-5-yl]piperidine-1-carboxylate (Example 24) (11 mg, 0.023 mmol) by the similar manner as described in Example 30 as a white amorphous (2 mg, yield 22%).
FAB-MS (m/z): 494 (M+1)
¹H NMR (CDCl₃ 400 MHz): δ= 1.19 (3H, t, J = 8 Hz), 1.6-1.8 8 (2H, m), 1.8-2.0 (2H, m), 2.47 (2H, q, J = 8 Hz), 2.7-3.0 (3H, m), 3.22 (3H, s), 4.8-5.0 (2H, m), 5.39 (2H, s), 6.70 (1H, d, J = 3 Hz), 6.75 (1H, d, J = 8 Hz), 7.14 (1H, d, J = 11 Hz), 7.31 (1H, d, J = 3 Hz), 7.43 (1H, dd, J = 2 Hz, 8 Hz), 8.18 (2H, s), 8.24 (1H, d, J = 7 Hz), 8.48 (1H, d, J = 2 Hz).

### [Example 33]

### 5-Ethyl-2-[4-[2-[5-(tetrazol-1-yl)indol-1-ylmethyl]pyridin-5-yl]piperidin-1-yl]pyrimidine

The title compound was prepared from tert-butyl 4-[2-[5-(tetrazol-1-yl)indol-1-ylmethyl]pyridin-5-yIJpiperidine-1-carboxylate (Example 20) (33 mg, 0.072 mmol) by the similar manner as described in Example 30 as a pale brown amorphous (19 mg, yield 57%) .
FAB-MS (m/z): 466 (M+1)
¹H NMR (CDCl₃ 400 MHz): δ= 1.19 (3H, t, J = 8 Hz), 1.6-1.8 (2H, m), 1.8-2.0 (2H, m), 2.47 (2H, q, J = 8 Hz), 2.7-3.0 (3H, m), 4.8-5.0 (2H, m), 5.48 (2H, s), 6.70 (1H, d, J = 3 Hz), 6.74 (1H, d, J = 8 Hz), 7.37 (1H, d, J = 3 Hz), 7.4-7.5 (3H, m), 7.91 (1H, d, J = 2 Hz), 8.18 (2H, s), 8.49 (1H, d, J = 2 Hz), 8.94 (1H, s).

### [Example 34]

### 5-Ethyl-2-[4-[2-(7-fluoro-5-methanesulfonylindol-1-ylmethyl)pyridin-5-yl]piperidin-1-yl]pyrimidine

The title compound was prepared from tert-butyl 4-[2-(7-fluoro-5-methanesulfonylindol-1-ylmethyl)pyridin-5-yl]piperidine-1-carboxylate (Example 11) (106 mg, 0.22 mmol) by the similar manner as described in Example 30 as a white amorphous (59 mg, yield 62%).
FAB-MS (m/z): 494 (M+1)
¹H NMR (CDCl₃ 400 MHz): δ= 1.19 (3H, t, J = 8 Hz), 1.6-1.8 8 (2H, m), 1.8-2.0 (2H, m), 2.47 (2H, q, J = 8 Hz), 2.7-3.0 (3H, m), 3.07 (3H, s), 4.8-5.0 (2H, m), 5.60 (2H, s), 6.73 (1H, t, J = 3 Hz), 6.87 (1H, d, J = 8 Hz), 7.37 (1H, d, J = 3 Hz), 7.40 (1H, dd, J = 2 Hz, 12 Hz), 7.44 (1H, dd, J = 2 Hz, 8 Hz), 8.06 (1H, d, J = 2 Hz), 8.18 (2H, s), 8.46 (1H, d, J = 2 Hz).

### [Example 35]

### 5-Ethyl-2-[4-[2-(7-chloro-5-methanesulfonylindol-1-ylmethyl)pyridin-5-yl]piperidin-1-yl]pyrimidine

The title compound was prepared from tert-butyl 4-[2-(7-chloro-5-methanesulfonylindol-1-ylmethyl)pyridin-5-yIJpiperidine-1-carboxylate (100 mg, 0.20 mmol) by the similar manner as described in Example 30 as a pale yellow crystal (93 mg, yield 91%) .
FAB-MS (m/z): 510 (M+1)
H NMR (CDCl₃ 400 MHz): δ= 1.19 (3H, t, J = 8 Hz), 1.5-1.8 8 (2H, m), 1.8-1.9 (2H, m), 2.46 6 (2H, q, J = 8 Hz), 2.7-2.9 (1H, m), 2.9-3.0 (2H, m), 3.08 (3H, s), 4.8-5.0 (2H, m), 5.88 (2H, s), 6.66 (1H, d, J = 8 Hz), 6.76 (1H, d, J = 3 Hz), 7.34 (1H, d, J = 3 Hz), 7.41 (1H, dd, J = 2 Hz, 8 Hz), 7.68 (1H, d, J = 2 Hz), 8.1-8.2 (3H, m), 8.46 (1H, d, J = 2 Hz).

### [Example 36]

tert-Butyl 4-[2-(7-fluoro-5-nitroindolin-1-ylmethyl)pyridin-5-yl]piperidine-1-carboxylate

### (1) 7-Fluoro-5-nitroindoline

1-Acetyl-7-fluoro-5-nitroindoline (400 mg, 1.78 mmol) was added 1 mol/L hydrochloric acid (10 mL) and refluxed for 1.5 hours. After cooling to room temperature, the reaction mixture was added dropwise to saturated aqueous sodium hydrogen carbonate solution (3 mL), and was extracted with chloroform. The organic layer was dried over anhydrous sodium sulfate, and the solvent was removed under reduced pressure, to give the title compound as an orange oil (324 mg, yield 100%).
¹H NMR (CDCl₃ 400 MHz): 5= 3.18 (2H, t, J = 9 Hz), 3.84 4 (2H, dt, J = 1 Hz, 9 Hz), 4.4-4.5 (1H, br s), 7.8-7.9 (2H, m).

### (2) tert-Butyl 4-[2-(7-fluoro-5-nitroindolin-1-ylmethyl)pyridin-5-yl]piperidine-1-carboxylate

The title compound was prepared from 7-fluoro-5-nitroindoline (30 mg, 0.16 mmol) by the similar manner as described in Example 18 as a pale yellow oil (21 mg, yield 29%) .
¹H NMR (CDCl₃ 400 MHz): δ= 1.48 (9H, s), 1.5-1.7 7 (2H, m), 1.7-1.9 (2H, m), 2.6-2.8 (1H, m), 2.7-2.9 (2H, m), 3.14 (2H, t, J = 9 Hz), 3.75 (2H, t, J = 9 Hz), 4.1-4.4 (2H, m), 4.75 (2H, s), 7.24 (1H, d, J = 8 Hz), 7.51 (1H, dd, J = 2 Hz, 8 Hz), 7.75 (1H, s), 7.80 (1H, dd, J = 2 Hz, 13 Hz), 8.43 (1H, d, J = 2 Hz).

### [Example 37]

### tert-Butyl 4-[2-(5-amino-7-fluoroindolin-1-ylmethyl)pyridin-5-yl]piperidine-1-carboxylate

A suspension of zinc powder (108 mg, 1.65 mmol) in 0.5 mol/L hydrogen chloride was stirred at room temperature for 5 minutes, and then filtered. After washing by water (0.5 mL) and ethanol (0.5 mL), the zinc solid was added to a solution of calcium chloride (3.2 mg) in water (0.13 mL)-ethanol (0.52 mL), and then warmed to 90°C. To this was added a solution of tert-butyl 4-[2-(7-fluoro-5-nitroindolin-1-ylmethyl)pyridin-5-yl]piperidine-1-carboxylate (Example 36) (21.3 mg, 0.046 mmol) in ethanol (0.8 mL). After stirring at 90°C for 40 minutes, the mixture was cooled to room temperature and the insoluble material was filtered off. The filtrate was concentrated to dryness, and the residue was diluted with ethyl acetate, the organic layer was dried over anhydrous sodium sulfate. The solvent was removed under reduced pressure, to give the title compound as a dark brown oil (18 mg, yield 93%).
¹H NMR (CDCl₃ 400 MHz): 5= 1.48 (9H, s), 1.5-1.7 7 (2H, m), 1.7-1.9 (2H, m), 2.6-2.8 (1H, m), 2.7-2.9 (2H, m), 3.14 (2H, t, J = 8 Hz), 3.29 (2H, t, J = 8 Hz), 3.3-3.5 (2H, br s), 4.1-4.4 (2H, m), 4.50 (2H, s), 6.24 (1H, d, J = 14 Hz), 7.32 (1H, s), 7.39 (1H, d, J = 8 Hz), 7.48 (1H, dd, J = 2 Hz, 8 Hz), 8.42 (1H, d, J = 2 Hz).

### [Example 38]

### tert-Butyl 4-[2-[7-fluoro-5-(tetrazol-1-yl)indolin-1-ylmethyl]pyridin-5-yl]piperidine-1-carboxylate

To a solution of tert-butyl 4-[2-(5-amino-7-fluoroindolin-1-ylmethyl)pyridin-5-yl]piperidine-1-carboxylate (Example 37) (18.3 mg, 0.043 mmol) in acetic acid (0.5 mL) was added triethyl orthoformate (0.04 mL) and sodium azide (12.3 mg, 0.19 mmol), and the mixture was warmed up to 90°C slowly. After stirring at the same temperature for an additional 2 hours, then cooled, the reaction mixture was poured into water (5 mL), was added saturated aqueous sodium hydrogen carbonate solution until pH 6, and was extracted with chloroform. The organic layer was washed with water, dried over anhydrous sodium sulfate, and the solvent was removed under reduced pressure. The residue was purified by silica gel column chromatography (ethyl acetate/hexane = 1/5), to give the title compound as a dark brown oil (14 mg, yield 67%).
FAB-MS (m/z): 480 (M+1)
¹H NMR (CDCl₃ 400 MHz): 5= 1.48 (9H, s), 1.5-1.7 7 (2H, m), 1.7-1.9 (2H, m), 2.6-2.8 (1H, m), 2.7-2.9 (2H, m), 3.14 (2H, t, J = 9 Hz), 3.61 (2H, t, J = 9 Hz), 4.1-4.4 (2H, m), 4.69 (2H, s), 7.1-7.2 (2H, m), 7.31 (1H, d, J = 8 Hz), 7.51 (1H, dd, J = 2 Hz, 8 Hz), 8.45 (1H, d, J = 2 Hz), 8.84 (1H, s).

### [Example 39]

### tert-Butyl 4-[2-(7-fluoro-5-nitroindol-1-ylmethyl)pyridin-5-yl]piperidine-1-carboxylate

### (1) 7-Fluoro-5-nitroindole

To a solution of 7-fluoro-5-nitroindoline (Example 36 (1)) (100 mg, 0.55 mmol) in xylene (5 mL) was added 10% palladium-carbon (292 mg, 0.27 mmol) and then the mixture was refluxed for 2 hours. After cooling to room temperature, the insoluble materials were filtered off, and the solvent was removed under reduced pressure. The residue was purified by silica gel column chromatography (ethyl acetate/hexane = 1/1), to give the title compound as a yellow crystal (83 mg, yield 84%) .
¹H NMR (CDCl₃ 400 MHz): δ= 6.81 (1H, dd, J = 3 Hz, 5 Hz), 7.41(1H, t, J = 3 Hz), 7.86 (1H, dd, J = 2 Hz, 12 Hz), 8.46 (1H, d, J = 2 Hz), 8.5-8.9 (1H, br s).

### (2) tert-Butyl 4-[2-(7-fluoro-5-nitroindol-1-ylmethyl)pyridin-5-yl]piperidine-1-carboxylate

The title compound was prepared from 7-fluoro-5-nitroindole (46 mg, 0.26 mmol) by the similar manner as described in Example 18 as a yellow crystal (40 mg, yield 34%) .
H NMR (CDCl₃ 400 MHz): 5= 1.47 (9H, s), 1.5-1.7 (2H, m), 1.7-1.9 (2H, m), 2.6-2.8 (1H, m), 2.7-2.9 (2H, m), 4.1-4.4 (2H, m), 5.59 (2H, s), 6.78 (1H, t, J = 3 Hz), 6.91 (1H, d, J = 8 Hz), 7.37 (1H, d, J = 3 Hz), 7.44 (1H, dd, J = 2 Hz, 8 Hz), 7.79 (1H, dd, J = 2 Hz, 12 Hz), 8.41 (1H, d, J = 2 Hz), 8.43 (1H, d, J = 2 Hz).

### [Example 40]

### tert-Butyl 4-[2-(5-amino-7-fluoroindol-1-ylmethyl)pyridin-5-yl]piperidine-1-carboxylate

The title compound was prepared from tert-butyl 4-[2-(7-fluoro-5-nitroindol-1-ylmethyl)pyridin-5-yIJpiperidine-1-carboxylate (Example 39) (39.6 mg, 0.087 mmol) by the similar manner as described in Example 37 as a brown oil (36 mg, yield 99%) .
¹H NMR (CDCl₃ 400 MHz): 5= 1.47 (9H, s), 1.5-1.7 (2H, m), 1.7-1.9 (2H, m), 2.6-2.7 (1H, m), 2.7-2.9 (2H, m), 4.1-4.4 (2H, m), 5.48 (2H, s), 6.3-6.4 (2H, m), 6.67 (1H, d, J = 2 Hz), 6.73 (1H, d, J = 8 Hz), 7.08 8 (1H, d, J = 2 Hz), 7.36 (1H, dd, J = 2 Hz, 8 Hz), 8.43 (1H, d, J = 2 Hz).

### [Example 41]

### tert-Butyl 4-[2-[7-fluoro-5-(tetrazol-1-yl)indol-1-ylmethyl]pyridin-5-yl]piperidine-1-carboxylate

The title compound was prepared from tert-butyl 4-[2-(5-amino-7-fluoroindol-1-ylmethyl)pyridin-5-yIJpiperidine-1-carboxylate (Example 40) (36.4 mg, 0.086 mmol) by the similar manner as described in Example 38 as a brown oil (35 mg, yield 85%) .
FAB-MS (m/z): 478 (M+1)
¹H NMR (CDCl₃ 400 MHz): δ= 1.47 (9H, s), 1.5-1.7 7 (2H, m), 1.7-1.9 (2H, m), 2.6-2.8 (1H, m), 2.7-2.9 (2H, m), 4.1-4.4 (2H, m), 5.61 (2H, s), 6.6-6.8 (1H, m), 6.89 (1H, d, J = 8 Hz), 7.25 (1H, dd, J = 2 Hz, 12 Hz), 7.38 (1H, d, J ₌ 2 Hz), 7.45 (1H, dd, J = 2 Hz, 8 Hz), 7.70 (1H, d, J = 2 Hz), 8.45 (1H, d, J = 2 Hz), 8.98 (1H, s).

### [Example 42]

### tert-Butyl 4-[2-(4,7-difluoro-5-methanesulfonylindol-1-ylmethyl)pyridin-5-yl]piperidine-1-carboxylate

### (1) 5-Bromo-4,7-difluoroindole

The title compound was prepared from 4-bromo-2,5-difluoronitrobenzene (1.0 g, 4.20 mmol) by the similar manner as described in Example 28 (1) as a brown crystal (300 mg, yield 31%) .
¹H NMR (CDCl₃ 400 MHz): δ= 6.65 (1H, dd, J = 3 Hz, 5 Hz), 7.03(1H, dd, J = 5 Hz, 10 Hz), 7.21 1 (1H, t, J = 3 Hz), 8.46 (1H, br s).

### (2) 4,7-Difluoro-5-methanesulfonylindole

The title compound was prepared from 5-bromo-4,7-difluoroindole (300 mg, 1.29 mmol) by the similar manner as described in Example 22 (1) as a pale yellow crystal (70 mg, yield 23%).
¹H NMR (CDCl₃ 400 MHz): 5= 3.26 (3H, s), 6.8-6.9 (1H, m), 7.35 (1H, t, J = 3 Hz), 7.45 (1H, dd, J = 5 Hz, 10 Hz), 8.72 (1H, br s).

### (3) tert-Butyl 4-[2-(4,7-difluoro-5-methanesulfonylindol-1-ylmethyl)pyridin-5-yl]piperidine-1-carboxylate

The title compound was prepared from 4,7-difluoro-5-methanesulfonylindole by the similar manner as described in Example 18 as a pale yellow crystal.
FAB-MS (m/z): 506 (M+1)
¹H NMR (CDCl₃ 400 MHz): δ= 1.47 (9H, s), 1.5-1.7 7 (2H, m), 1.7-1.8 (2H, m), 2.6-2.9 (3H, m), 3.24 (3H, s), 4.2-4.3 (2H, m), 5.57 (2H, s), 6.80 (1H, dd, J = 2 Hz, 3 Hz), 6.93 (1H, d, J = 8 Hz), 7.32 (1H, d, J = 3 Hz), 7.38 (1H, dd, J = 4 Hz, 11 Hz), 7.45 (1H, dd, J = 2 Hz, 8 Hz), 8.43 (1H, d, J = 2 Hz).

### [Example 43]

### tert-Butyl 4-[2-(5-methanesulfonylindol-1-ylmethyl)pyridin-5-yl]-3-methyl-3,6-dihydro-2H-pyridine-1-carboxylate

### (1) 1-(5-Bromopyridin-2-ylmethyl)-5-methanesulfonylindole

The title compound was prepared from 5-bromo-2-hydroxymethyl)pyridine (226 mg, 1.20 mmol) and 5-methanesulfonylindole (195 mg, 1.00 mmol) by the similar manner as described in Example 18 as a pale yellow amorphous (345 mg, yield 94%).
¹H NMR (CDCl₃ 400 MHz): δ= 3.07 7 (3H, s), 5.45 (2H, s), 6.68 (1H, d, J = 8 Hz), 6.73 (1H, d, J = 3 Hz), 7.35 (1H, d, J = 3 Hz), 7 . 39 (1H, d, J = 8 Hz), 7.6-7.8 (2H, m), 8.28 (1H, s), 8.64 (1H, d, J = 2 Hz).

### (2) tert-Butyl 3-methyl-4-(trifluoromethylsulfonyloxy)-3,6-dihydro-2H-pyridine-1-carboxylate

Under N₂ atmosphere, a solution of 1.0M lithium bis(trimethylsilyl)amide-tetrahydrofuran solution (2.7 mL, 2.73 mmol) was dissolved in dry tetrahydrofuran (8 mL). The mixture was cooled to -78°C, was added dropwise a solution of tert-butyl 3-methyl-4-oxopiperidine-1-carboxylate (530 mg, 2.49 mmol) in dry tetrahydrofuran (2 mL), stirred at the same temperature for an additional 30 minutes, and then was added dropwise a solution of N-phenylbis(trifluoromethyl)sulfonamide (888 mg, 2.49 mmol) in dry tetrahydrofuran (2 mL). The reaction mixture was warmed up to 0°C, stirred for 1 hour, and the solvent was removed under reduced pressure. The residue was purified by silica gel column chromatography (hexane/chloroform =8/1 → 0/100), to give the title compound as a colorless oil (522 mg, yield 61%) .
¹H NMR (CDCl₃ 400 MHz): 5= 1.16 (3H, d, J = 7 Hz), 1.48 (9H, s), 2.62 (1H, br s), 3.2-3.8 8 (2H, m), 3.9-4.3 (2H, m), 5.73 (1H, br s).

### (3) tert-Butyl 3-methyl-4-(4,4,5,5-tetramethyl-1,3,2-dioxaborolan-2-yl)-3,6-dihydro-2H-pyridine-1-carboxylate

A suspension of tert-butyl 3-methyl-4-(trifluoromethylsulfonyloxy)-3,6-dihydro-2H-pyridine-1-carboxylate (522 mg, 1.51 mmol), potassium acetate (445 mg, 4.54 mmol), l,l-bis(diphenylphosphino)ferrocene (25 mg, 0.0454 mmol), [1,1-bis(diphenylphosphino)ferrocene]pal]adium(II) dichloride dichloromethane adduct (37 mg, 0.0454 mmol) and bis(pinacolato)diboron (422 mg, 1.66 mmol) in dry 1,4-dioxane (10 mL) was stirred at 80°C for 16 hours under N₂. After cooling to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate =7/1 → 1/1), to give the title compound as a colorless oil (395 mg, yield 81%).
¹H NMR (CDCl₃ 400 MHz) : 5= 1.03 (3H, d, J = 7 Hz), 1.26 6 (12 H, d, J = 3 Hz), 1.46 (9H, s), 2.45 (1H, br s), 3.15 (1H, d, J = 13 Hz), 3.55 (1H, dd, J = 3 Hz, 13 Hz), 3.7-3.8 (1H, m), 4.0-4.3 (1H, m), 6.3-6.5 (1H, m).

### (4) tert-Butyl 4-[2-(5-methanesulfonylindol-1-ylmethyl)pyridin-5-yl]-3-methyl-3,6-dihydro-2H-pyridine-1-carboxylate

To a solution of tert-butyl 3-methyl-4-(4,4,5,5-tetramethyl-1,3,2-dioxaborolan-2-yl)-3,6-dihydro-2H-pyridine-1-carboxylate(50 mg, 0.155 mmol) and 1-(5-bromopyridin-2-ylmethyl)-5-methanesulfonylindole (57 mg, 0.155 mmol) in dry N,N-dimethylformamide (1.5 mL) was added [1,1-bis(diphenylphosphino)ferrocene]dichloropal]adium(II), dichloromethane adduct (6.3 mg, 7.74 µmol) and cesium carbonate (101 mg, 0.309 mmol) under N₂. After stirring at 80°C for 21 hours, cooled to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/acetone = 5/1), to give the title compound as a white amorphous (25 mg, yield 35%) .
FAB-MS (m/z): 482 (M+1)
¹H NMR (CDCl₃ 400 MHz): 5= 0.99 (3H, d, J = 7 Hz), 1.48 (9H, s), 2.7-2.9 (1H, m), 3.07 (3H, s), 3.29 (1H, dd, J = 3 Hz, 13 Hz), 3.82 (1H, dd, J = 3 Hz, 13 Hz), 3.8-4.0 (1H, m), 4.2-4.4 (1H, m), 5.49 (2H, s), 5.90 (1H, br s), 6.7-6.8 (2H, m), 7.38 (1H, d, J = 3 Hz), 7.44 (1H, d, J = 8 Hz), 7.50 (1H, dd, J = 2 Hz, 8 Hz), 7.71 (1H, dd, J = 2 Hz, 8 Hz), 8.30 (1H, d, J = 2 Hz), 8.57 (1H, d, J = 2 Hz).

### [Example 44]

### tert-Butyl 4-[2-(5-methanesulfonylindol-1-ylmethyl)pyridin-5-yl]-3-methylpiperidine-1-carboxylate

To a solution of tert-butyl 4-[2-(5-methanesulfonylindol-1-ylmethyl)pyridin-5-yl]-3-methyl-3,6-dihydro-2H-pyridine-1-carboxylate (22 mg, 0.0457 mmol) in methanol (0.5 mL) was added 10% palladium-carbon (2 mg) and then the mixture was hydrogenated at room temperature for 2 hours under 1 atm of H₂. The reaction mixture was filtered through a celite pad, and the filtrate was concentrated under reduced pressure. The residue was purified by silica gel column chromatography (hexane/ethyl acetate =2/1 → 1/2), to give the title compound as a white amorphous (16 mg, yield 72%).
FAB-MS (m/z): 484 (M+1)
¹H NMR (CDCl₃ 400 MHz) : 5= 0.67 (3H, d, J = 7 Hz), 1.46 6 (9H, s), 1.57 (1H, dd, J = 3 Hz, 13 Hz), 1.9-2.1 (2H, m), 2.7-2.9 (1H, m), 2.9-3.0 (1H, m), 3.0-3.1 (1H, m), 3.07 (3H, s), 4.0-4.5 (2H, m), 5.48 (2H, s), 6.7-6.8 (2H, m), 7.34 (1H, dd, J = 2 Hz, 8 Hz), 7.37 (1H, d, J = 3 Hz), 7.43 (1H, d, J = 8 Hz), 7.71 (1H, dd, J = 2 Hz, 8 Hz), 8.30 (1H, d, J = 2 Hz), 8.42 (1H, d, J = 2 Hz).

### [Example 45]

### tert-Butyl 3,3-dimethyl-4-[2-(5-methanesulfonylindol-1-ylmethyl)pyridin-5-yl]-3,6-dihydro-2H-pyridine-1-carboxylate

### (1) tert-Butyl 3,3-dimethyl-4-(4,4,5,5-tetramethyl-1,3,2-dioxaborolan-2-yl)-3,6-dihydro-2H-pyridine-1-carboxylate

The title compound was prepared from tert-butyl 3,3-dimethyl-4-(trifluoromethylsulfonyloxy)-3,6-dihydro-2H-pyridine-1-carboxylate (183 mg, 0.51 mol) by the similar manner as described in Example 43 (3) as a white crystal (94 mg, yield 55%) .
¹H NMR (CDCl₃ 400 MHz): δ= 1.08 (6H, s), 1.25 (12H, s), 1.49 (9H, s), 3.16 (2H, s), 3.93 (2H, s), 6.2-6.4 (1H, m).

### (2) tert-Butyl 3,3-dimethyl-4-[2-(5-methanesulfonylindol-1-ylmethyl)pyridin-5-yl]-3,6-dihydro-2H-pyridine-1-carboxylate

The title compound was prepared from tert-butyl 3,3-dimethyl-4-(4,4,5,5-tetramethyl-1,3,2-dioxaborolan-2-yl)-3,6-dihydro-2H-pyridine-1-carboxylate(54 mg, 0.15 mmol) by the similar manner as described in Example 43 (4) as a pale yellow oil (16 mg, yield 22%).
FAB-MS (m/z): 496 (M+1)
¹H NMR (CDCl₃ 400 MHz): δ= 1.01 (6H, s), 1.49 (9H, s), 3.08 (3H, s), 3.34 (2H, s), 3.9-4.1 (2H, m), 5.49 (2H, s), 5.4-5.6 (1H, m), 6.69 (1H, d, J = 8 Hz), 6.75 (1H, d, J = 3 Hz), 7.3-7.4 (2H, m), 7.45 (1H, d, J = 9 Hz), 7.72 (1H, dd, J = 2 Hz, 9 Hz), 8.30 (1H, d, J = 2 Hz), 8.40 (1H, s).

### [Example 46]

### tert-Butyl 3,3-dimethyl-4-[2-(5-methanesulfonylindol-1-ylmethyl)pyridin-5-yl]piperidine-1-carboxylate

The title compound was prepared from tert-butyl 3,3-dimethyl-4-[2-(5-methanesulfonylindol-1-ylmethyl)pyridin-5-yl]-3,6-dihydro-2H-pyridine-1-carboxylate (Example 45) (14 mg, 0.028 mmol) by the similar manner as described in Example 44 as a white amorphous (6 mg, yield 44%).
FAB-MS (m/z): 498 (M+1)
¹H NMR (CDCl₃ 400 MHz): δ= 0.75 (3H, s), 0.77 (3H, s), 1.47 (9H, s), 1.4-1.6 (1H, m), 2.0-2.2 (1H, m), 2.4-2.8 (3H, m), 3.07 (3H, s), 3.7-4.5 (2H, m), 5.48 (2H, s), 6.70 (1H, d, J = 9 Hz), 6.74 (1H, d, J = 3 Hz), 7.33 (1H, dd, J = 2 Hz, 9 Hz), 7.38 (1H, d, J = 3 Hz), 7.44 (1H, d, J = 9 Hz), 7.71 (1H, dd, J = 2 Hz, 9 Hz), 8.30 (1H, s), 8.38 (1H, d, J = 2 Hz).

### [Example 47]

### tert-Butyl 4-[2-[1-(5-methanesulfonylindol-1-yl)ethyl]pyridin-5-yl]piperidine-1-carboxylate

### (1) tert-Butyl 4-[2-(1-hydroxyethyl)pyridin-5-yl]-3,6-dihydro-2H-pyridine-1-carboxylate

The title compound was prepared from 1-(5-bromopyridin-2-yl)ethanol (497 mg, 2.46 mmol) and tert-butyl 4-(4,4,5,5-tetramethyl-1,3,2-dioxaborolan-2-yl)-3,6-dihydro-2H-pyridine-l-carboxylate(761 mg, 2.46 mmol) by the similar manner as described in Example 43 (4) as a brown oil (573 mg, yield 77%).
¹H NMR (CDCl₃ 400 MHz): δ= 1.50 0 (9H, s), 1.51 (3H, s), 2.4-2.6 (2H, m), 3.6-3.7 (2H, m), 4.0-4.2 (2H, m), 4.12 (1H, br s), 4.89 (1H, q, J = 6 Hz), 6.09 (1H, br s), 7.25 (1H, d, J = 8 Hz), 7.66 (1H, dd, J = 2 Hz, 8 Hz), 8.55 (1H, d, J = 2 Hz).

### (2) tert-Butyl 4-[2-(1-hydroxyethyl)pyridin-5-yl]pyridine-1-carboxylate

The title compound was prepared from tert-butyl 4-[2-(1-hydroxyethyl)pyridin-5-yl]-3,6-dihydro-2H-pyridine-1-carboxylate (310 mg, 1.02 mmol) by the similar manner as described in Example 44 as a yellow oil (71 mg, yield 99%).
¹H NMR (CDCl₃ 400 MHz): δ= 1.49 (9H, s), 1.51 (3H, s), 1.5-1.7 (2H, m), 1.8-1.9 (2H, m), 2.6-2.8 (1H, m), 2.7-2.9 (2H, m), 4.1-4.4 4 (3H, m), 4.87 (1H, q, J = 6 Hz), 7.22 (1H, d, J = 8 Hz), 7.52 (1H, dd, J = 2 Hz, 8 Hz), 8.40 (1H, d, J = 2 Hz).

### (3) tert-Butyl 4-[2-[1-(5-methanesulfonylindol-1-yl)ethyl]pyridin-5-yl]piperidine-1-carboxylate

The title compound was prepared from tert-butyl 4-[2-(1-hydroxyethyl)pyridin-5-yl]piperidine-1-carboxylate (94 mg, 0.307 mmol) and 5-methanesulfonylindole (60 mg, 0.307 mmol) by the similar manner as described in Example 18 as a white amorphous (52 mg, yield 35%).
FAB-MS (m/z): 484 (M+1)
¹H NMR (CDCl₃ 400 MHz): δ= 1.47 (9H, s), 1.5-1.7 7 (2H, m), 1.7-1.9 (2H, m), 2.00 (3H, d, J = 7 Hz), 2.6-2.8 (1H, m), 2.7-2.9 (2H, m), 3.05 (3H, s), 4.1-4.4 (2H, m), 5.76 (1H, q, J = 7 Hz), 6.73 (1H, d, J = 3 Hz), 6.76 6 (1H, d, J = 8 Hz), 7.3-7.4 (1H, m), 7.40 (1H, d, J = 9 Hz), 7.56 (1H, d, J = 3 Hz), 7.66 (1H, d, J = 9 Hz), 8.2-8.3 (1H, m), 8.46 (1H, d, J = 1 Hz).

### [Example 48]

### tert-Butyl 4-[2-[1-(5-methanesulfonylindol-1-yl)ethyl]pyridin-5-yl]-3,6-dihydro-2H-pyridine-1-carboxylate

The title compound was prepared from tert-butyl 4-[2-(1-hydroxyethyl)pyridin-5-yl]-3,6-dihydro-2H-pyridine-1-carboxylate (Example 47 (1)) (74 mg, 0.243 mmol) by the similar manner as described in Example 18 as a white amorphous (13 mg, yield 11%).
FAB-MS (m/z): 482 (M+1)
¹H NMR (CDCl₃ 400 MHz): δ= 1.48 8 (9H, s), 2.01 (3H, d, J = 7 Hz), 2.4-2.6 (2H, m), 3.05 (3H, s), 3.5-3.7 (2H, m), 4.0-4.2 (2H, m), 5.78 (1H, q, J = 7 Hz), 6.0-6.1 (1H, br s), 6.74 (1H, d, J = 3 Hz), 6.76 (1H, d, J = 8 Hz), 7.38 (1H, d, J = 8 Hz), 7.51 (1H, dd, J = 2 Hz, 8 Hz), 7.56 (1H, d, J = 3 Hz), 7.66 (1H, d, = 8 Hz), 8.2-8.3 (1H, m), 8.62 (1H, d, J = 1 Hz).

### [Example 49]

### tert-Butyl 4-[2-(5-methanesulfonyl-7-trifluoromethylindol-1-ylmethyl)pyridin-5-yl]piperidine-1-carboxylate

### (1) 5-Bromo-7-trifluoromethylindole

The title compound was prepared from 5-bromo-2-nitrobenzotrifluoride (5.00 g, 18.5 mmol) by the similar manner as described in Example 28 (1) as a pale yellow crystal (116 mg, yield 2%).
¹H NMR (CDCl₃ 400 MHz): δ= 6.5-6.7 (1H, m), 7.31 (1H, t, J = 3 Hz), 7.5-7.6 (1H, m), 7.9-8.0 (1H, m), 8.4-8.7 (1H, br s).

### (2) 5-Methanesulfonyl-7-trifluoromethylindole

The title compound was prepared from 5-bromo-7-trifluoromethylindole (116 mg, 0.439 mmol) by the similar manner as described in Example 22 (1) as a white crystal (51 mg, yield 44%).
¹H NMR (CDCl₃ 400 MHz): δ= 3.12 (3H, s), 6.7-6.9 (1H, m), 7.50 (1H, t, J = 3 Hz), 8.0-8.1 (1H, m), 8.4-8.5 (1H, m), 9.08 (1H, br s).

### (3) tert-Butyl 4-[2-(5-methanesulfonyl-7-trifluoromethylindol-1-ylmethyl)pyridin-5-yl]piperidine-1-carboxylate

The title compound was prepared from 5-methanesulfonyl-7-trifluoromethylindole by the similar manner as described in Example 18 as a white amorphous.
FAB-MS (m/z): 538 (M+1)
¹H NMR (CDCl₃ 400 MHz): δ= 1.47 (9H, s), 1.5-1.7 (2H, m), 1.7-1.9 (2H, m), 2.6-2.8 (1H, m), 2.7-2.9 (2H, m), 3.12 (3H, s), 4.1-4.4 (2H, m), 5.66 (2H, s), 6.59 (1H, d, J = 8 Hz), 6.88 (1H, d, J = 3 Hz), 7.36 (1H, d, J = 3 Hz), 7.39 (1H, dd, J = 2 Hz, 8 Hz), 8.0-8.2 (1H, m), 8.44 (1H, d, J = 2 Hz), 8.48 (1H, d, J = 1 Hz).

### [Example 50]

### tert-Butyl 3-[2-(5-methanesulfonylindol-1-ylmethyl)pyridin-5-yllpyrrolidine-l-carboxylate

The mixture of tert-butyl 4-[6-(5-methanesulfonylindol-1-ylmethyl)pyridine-3-yl]-2,3-dihydropyrrole-1-carboxylate and tert-butyl 3-[6-(5-methanesulfonylindol-1-ylmethyl)pyridine-3-yl]-2,5-dihydropyrrole-1-carboxylate were prepared from 1-(5-bromopyridin-2-ylmethyl)-5-methanesulfonylindole (Example 43 (1)) (51 mg, 0.194 mmol) by the similar manner as described in Example 43 (4). The title compound was prepared from the resulting mixture by the similar manner as described in Example 44 as a white amorphous (25 mg, yield 63%).
FAB-MS (m/z): 456 (M+1)
¹H NMR (CDCl₃ 400 MHz): δ= 1.46 (9H, s), 1.4-1.5 (1H, m), 2.1-2.3 (1H, m), 3.07 (3H, s), 3.2-3.5 (3H, m), 3.5-3.6 (1H, m), 3.6-3.7 (1H, m), 5.48 (2H, s), 6.74 (1H, d, J = 3 Hz), 6.76 6 (1H, d, J = 8 Hz), 7.37 ( 1H, d, J = 3 Hz), 7.4-7.5 (2H, m), 7.71 1H, dd, J = 1 Hz, 8 Hz), 8.29 (1H, d, J = 1 Hz), 8.49 (1H, d, J = 2 Hz).

### [Example 51]

### tert-Butyl 4-[5-(5-methanesulfonylindol-1-ylmethyl)pyridin-2-yl]azepan-1-carboxylate

The mixture of tert-butyl 5-(4,4,5,5-tetramethyl-1,3,2-dioxaborolan-2-yl)-2,3,4,7-tetrahydroazepin-1-carboxylate and tert-butyl 4-(4,4,5,5-tetramethyl-1,3,2-dioxaborolan-2-yl)-2,3,6,7-tetrahydroazepin-1-carboxylate were prepared from tert-butyl 4-oxo-l-azepancarboxylate (500 mg, 2.34 mmol) by the similar manner as described in Example 43 (2), (3) and (4). The title compound was prepared from the resulting mixture and 1-(5-bromopyridine-2-ylmethyl)-5-methanesulfonylindole(65 mg, 0.178 mmol) by the similar manner as described in Example 50 as a white amorphous (33 mg, yield 38%).
FAB-MS (m/z): 484 (M+1)
¹H NMR (CDCl₃ 400 MHz): δ= 1.47 7 (9H, s), 1.5-2.0 (6H, m), 2.6-2.8 (1H, m), 3.07 (3H, s), 3.1-3.6 (3H, m), 3.6-3.8 (1H, m), 5.46 (2H, s), 6.7-6.8 (2H, m), 7.3-7.4 (2H, m), 7.44 (1H, d, J = 8 Hz), 7.70 (1H, dd, J = 1 Hz, 8 Hz), 8.29 (1H, d, J = 1 Hz), 8.43 (1H, d, J = 2 Hz).

### [Example 52]

### tert-Butyl 4-[6-(5-methanesulfonylindol-1-ylmethyl)pyridazin-3-yl]-3,6-dihydro-2H-pyridine-1-carboxylate

### (1) 1-(6-Chloropyridazin-3-ylmethyl)-5-methanesulfonylindole

The title compound was prepared from 3-bromomethyl-6-chloropyridazine (265 mg, 1.27 mmol) and 5-methanesulfonylindol (250 mg, 1.27 mmol) by the similar manner as described in Example 1 (4) as a pale yellow crystal (111 mg, yield 27%).
¹H NMR (CDCl₃ 400 MHz): δ= 3.06 (3H, s), 5.72 (2H, s), 6.76 (1H, d, J = 3 Hz), 6.91 (1H, d, J = 8 Hz), 7.3-7.5 (3H, m), 7.72 (1H, dd, J = 1 Hz, 8 Hz), 8.29 (1H, s).

### (2) tert-Butyl 4-[6-(5-methanesulfonylindol-1-ylmethyl)pyridazin-3-yl]-3,6-dihydro-2H-pyridine-1-carboxylate

The title compound was prepared from 1-(6-chloropyridazin-3-ylmethyl)-5-methanesulfonylindole (111 mg, 0.346 mmol) and tert-butyl 4-(4,4,5,5-tetramethyl-1,3,2-dioxaborolan-2-yl)-3,6-dihydro-2H-pyridine-1-carboxylate(107 mg, 0.346 mmol) by the similar manner as described in Example 43 (4) as a pale yellow crystal (35 mg, yield 22%).
FAB-MS (m/z): 469 (M+1)
¹H NMR (CDCl₃ 400 MHz): δ= 1.48 (9H, s), 2.7-2.8 (2H, m), 3.06 (3H, s), 3.6-3.7 (2H, m), 4.1-4.2 (2H, m), 5.71 (2H, s), 6.60 (1H, br s), 6.75 (1H, d, J = 3 Hz), 6.92 (1H, d, J = 8 Hz), 7.38 (1H, d, J = 3 Hz), 7.46 (2H, d, J = 8 Hz), 7.71 (1H, dd, J = 2 Hz, 8 Hz), 8.29 (1H, d, J = 2 Hz).

### [Example 53]

### tert-Butyl 4-[6-(5-methanesulfonylindol-1-ylmethyl)pyridazin-3-yl]piperidine-1-carboxylate

The title compound was prepared from tert-butyl 4-[6-(5-methanesulfonylindol-1-ylmethyl)pyridazin-3-yl]-3,6-dihydro-2H-pyridine-1-carboxylate (Example 52) (20 mg, 0.042 mmol) by the similar manner as described in Example 44 as a white amorphous (13 mg, yield 65%).
FAB-MS (m/z): 471 (M+1)
¹H NMR (CDCl₃ 400 MHz): δ= 1.46 (9H, s), 1.7-1.8 (2H, m), 1.9-2.0 (2H, m), 2.8-2.9 (2H, m), 3.07 (3H, s), 3.0-3.1 (1H, m), 4.2-4.3 (2H, m), 5.70 (2H, s), 6.75 (1H, d, J = 3 Hz), 6.88 (1H, d, J = 8 Hz), 7.21 (1H, d, J = 8 Hz), 7.37 (1H, d, J = 3 Hz), 7.47 (1H, d, J = 8 Hz), 7.72 (1H, dd, J = 2 Hz, 8 Hz), 8.30 (1H, d, J = 2 Hz).

### [Example 54]

### Isopropyl 4-[2-[7-fluoro-5-(tetrazol-1-yl)indol-l-ylmethyl]pyridin-5-yl]piperidine-1-carboxylate

A solution of tert-butyl 4-[2-[7-fluoro-5-(tetrazol-1-yl)indol-1-ylmethyl]pyridin-5-yl]piperidine-1-carboxylate (Example 41) (25 mg, 52.4 µmol) in trifluoroacetic acid (1 mL) was stirred at room temperature for 1 hour, and then the solvent was removed under reduced pressure.

A suspension of the resulting crude 4-[2-[7-fluoro-5-(tetrazol-1-yl)indol-1-ylmethyllpyridin-5-yl]piperidine in dichloromethane (1 mL) was added triethylamine (73 µL, 0.524 mmol) and isopropyl chloroformate (7 µL, 62.8 µmol). After stirring at room temperature for 1 hour, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/3), to give the title compound as a pale brown amorphous (15 mg, yield 62%).
FAB-MS (m/z): 464 (M+1)
¹H NMR (CDCl₃ 400 MHz): δ= 1.25 (6H, d, J = 6 Hz), 1.5-1.6 (2H, m), 1.7-1.9 (2H, m), 2.6-2.7 (1H, m), 2.8-2.8 (2H, m), 4.2-4.3 (2H, m), 4.9-5.0 (1H, m), 5.61 (2H, s), 6.69 (1H, t, J = 3 Hz), 6.89 (1H, d, J = 8 Hz), 7.24 (1H, d, J = 11 Hz), 7.37 (1H, d, J = 3 Hz), 7.43 (1H, dd, J = 2 Hz, 8 Hz), 7.68 (1H, d, J = 2 Hz), 8.44 (1H, d, J = 2 Hz), 8.93 (1H, s).

### [Example 55]

### tert-Butyl 4-[2-(6,7-difluoro-5-methanesulfonylindol-1-ylmethyl)pyridin-5-yl]piperidine-1-carboxylate

### (1) 1-(2-Amino-5-bromo-3,4-difluorophenyl)-2-chloroethanone

Under N₂ atmosphere, 1.0M boron trichloride/toluene solution (2.6 mL) was added dropwise to a solution of 4-bromo-2,3-difluoroaniline (500 mg, 2.40 mmol) in toluene (2.5 mL) under ice-cooling. To this was added chloroace-tonitrile (0.18 mL, 2.88 mmol) and aluminium chloride (353 mg, 2.64 mmol), and then the reaction mixture was stirred at 90°C overnight. After cooling to room temperature, the reaction mixture was neutralized by the addition of 2M hydrogen chloride. After stirring at 90°C for 10 minutes, cooled to room temperature, the reaction mixture was extracted with chloroform. The organic layer was washed with brine, dried over anhydrous sodium sulfate, and the solvent was removed under reduced pressure. The residue was purified by silica gel column chromatography (hexane/ethyl acetate = 4/1), silica gel column chromatography (Chromatorex NH (Fuji Silysia Chemical Ltd.), hexane/ethyl acetate = 10/1), to give the title compound as a yellow crystal (303 mg, yield 32%)).
¹H NMR (CDCl₃ 400 MHz): 5= 4.57 (2H, s), 6.4-6.6 6 (2H, br s), 7.64 (1H, dd, J = 2 Hz, 6 Hz).

### (2) 5-Bromo-6,7-difluoroindole

To a solution of 1-(2-amino-5-bromo-3,4-difluorophenyl)-2-chloroethanone (241 mg, 0.847 mmol) in 1,4-dioxane (4.2 mL) - water (0.43 mL) was added sodium borohydride (35 mg, 0.875 mmol). After stirring at 100°C for 45 minutes, the reaction mixture was cooled to room temperature, poured into water, and was extracted with chloroform. The organic layer was washed with brine, dried over anhydrous sodium sulfate, and the solvent was removed under reduced pressure.
To a solution of the resulting crude 1-(2-amino-5-bromo-3,4-difluorophenyl)-2-chloroethanol in ethanol (6 mL) was added potassium carbonate (235 mg, 1.69 mmol). Under N₂, after stirring at 85°C for 50 minutes, the reaction mixture was cooled to room temperature, and the solvent was removed under reduced pressure. The reaction mixture was poured into water (5 mL), and was extracted with ethyl acetate. The organic layer was washed with brine, dried over anhydrous sodium sulfate, and the solvent was removed under reduced pressure. The residue was purified by silica gel column chromatography (hexane/ethyl acetate = 10/1), to give the title compound as a white crystal (95 mg, yield 48%).
¹H NMR (CDCl₃ 400 MHz): δ= 6.4-6.6 (1H, m), 7.2-7.3 (1H, m), 7.55 (1H, dd, J = 1 Hz, 6 Hz), 8.2-8.5 (1H, br s).

### (3) 6,7-Difluoro-5-methanesulfonylindole

The title compound was prepared from 5-bromo-6,7-difluoroindole (110 mg, 0.477 mmol) by the similar manner as described in Example 22 (1) as a pale brown amorphous (11 mg, yield 10%).
¹H NMR (CDCl₃ 400 MHz) : δ= 3.26 (3H, s), 6.71 (1H, d, J = 3 Hz), 7.36 (1H, t, J = 3Hz), 8.04 (1H, d, J = 5 Hz), 8.6-8.7 (1H, br).

### (4) tert-Butyl 4-[2-(6,7-difluoro-5-methanesulfonylindol-1-ylmethyl)pyridin-5-yl]piperidine-1-carboxylate

The title compound was prepared from 6,7-difluoro-5-methanesulfonylindole by the similar manner as described in Example 18 as a yellow amorphous.
FAB-MS (m/z): 506 (M+1)
¹H NMR (CDCl₃ 400 MHz): δ= 1.47 (9H, s), 1.5-1.6 (2H, m), 1.7-1.9 (2H, m), 2.6-2.7 (1H, m), 2.7-2.9 (2H, m), 3.23 (3H, s), 4.2-4.3 (2H, m), 5.56 (2H, s), 6.69 (1H, t, J = 3 Hz), 6.89 (1H, d, J = 8 Hz), 7.33 (1H, d, J = 3 Hz), 7.45 (1H, dd, J = 2 Hz, 8 Hz), 8.00 (1H, d, J = 5 Hz), 8.43 (1H, d, J = 2 Hz).

### [Example 56]

### tert-Butyl 4-[2-[5-(1,2,4-triazol-1-yl)indol-1-ylmethyl]pyridin-5-yl]piperidine-1-carboxylate

### (1) 1-(3-Methyl-4-nitrophenyl)-1,2,4-triazole

A suspension of 5-fluoro-2-nitrotoluene (500 mg, 3.22 mmol), 1,2,4-triazole (223 mg, 3.22 mmol) and potassium hydroxide (358 mg, 3.38 mmol) in N,N-dimethylformamide (2 mL) was stirred at 90°C overnight. After cooling to room temperature, the reaction mixture was poured into water, and was extracted with chloroform. The organic layer was washed with brine, dried over anhydrous sodium sulfate, and the solvent was removed under reduced pressure. The residue was purified by silica gel column chromatography (hexane/ethyl acetate = 1/1), to give the title compound as a white crystal (566 mg, yield 86%).
¹H NMR (CDCl₃ 400 MHz): δ= 2.72 (3H, s), 7.68 8 (1H, dd, J = 2 Hz, 9 Hz), 7.74 (1H, d, J = 2 Hz), 8.14 (1H, s), 8.17 (1H, d, J = 9 Hz), 8.65 (1H, s).

### (2) 2-[2-Nitro-5-(1,2,4-triazol-1-yl)phenyl)-N,N-dimethylethenamine

The title compound was prepared from 1-(3-methyl-4-nitrophenyl)-1,2,4-triazole (452 mg, 2.21 mmol) by the similar manner as described in Example 29 (1) as a deep red crystal (568 mg, yield 99%).
¹H NMR (CDCl₃ 400 MHz): δ= 2.98 (6H, s), 5.98 (1H, d, J = 13 Hz), 7.11 (1H, d, J = 13 Hz), 7.18 (1H, dd, J = 2 Hz, 9 Hz), 7.80 (1H, d, J = 2 Hz), 8.02 (1H, d, J = 9 Hz), 8.14 (1H, s), 8.61 (1H, s).

### (3) 5-(1,2,4-Triazol-1-yl)indole

To a solution of 2-[2-nitro-5-(1,2,4-triazol-1-yl)phenyl]-N,N-dimethylethenamine (568 mg, 2.19 mmol) in ethanol (22 mL) was added 10% palladium-carbon (57 mg) and then the mixture was hydrogenated at room temperature overnight under 1 atm of H₂. The reaction mixture was filtered through a celite pad, and the filtrate was concentrated under reduced pressure. The residue was purified by silica gel column chromatography (chloroform/methanol = 98/2), to give the title compound as a pale yellow crystal (61 mg, yield 15%).
¹H NMR (CDCl₃ 400 MHz): δ= 6.64 4 (1H, t, J = 2 Hz), 7.32 (1H, t, J = 3 Hz), 7.48 (2H, m), 7.87 (1H, d, J = 1 Hz), 8.10 (1H, s), 8.3-8.5 (1H, br s), 8.51 (1H, s).

### (4) tert-Butyl 4-[2-[5-(1,2,4-triazol-1-yl)indol-1-ylmethyllpyridin-5-yllpiperidine-l-carboxylate

The title compound was prepared from 5-(1,2,4-triazol-1-yl)indole by the similar manner as described in Example 18 as a yellow amorphous.
FAB-MS (m/z): 459 (M+1)
¹H NMR (CDCl₃ 400 MHz): δ= 1.46 (9H, s), 1.5-1.6 (2H, m), 1.7-1.8 (2H, m), 2.6-2.7 (1H, m), 2.7-2.9 (2H, m), 4.2-4.3 (2H, m), 5.46 (2H, s), 6.66 (1H, d, J = 3 Hz), 6.70 (1H, d, J = 8 Hz), 7.31 (1H, d, J = 3 Hz), 7.38 (2H, m), 7.44 (1H, dd, J = 2 Hz, 8 Hz), 7.88 (1H, d, J = 2 Hz), 8.09 (1H, s), 8.46 (1H, d, J = 2 Hz), 8.49 (1H, s).

### [Example 57]

### tert-Butyl 4-[2-(4,6-difluoro-5-methanesulfonylindo1-1-ylmethyl)pyridin-5-yllpiperidine-1-carboxylate

### (1) 4,6-Difluoro-5-methanesulfonylindole

The title compound was prepared from 5-bromo-4,6-difluoroindole (578 mg, 2.49 mmol) by the similar manner as described in Example 22 (1) as a pale yellow crystal (69 mg, yield 12%).
¹H NMR (CDCl₃ 400 MHz): δ= 3.31 (3H, s), 6.74 4 (1H, m), 7.04 (1H, d, J = 11 Hz), 7.25 (1H, m), 8.4-8.7 (1H, br s).

### (2) tert-Butyl 4-[2-(4,6-difluoro-5-methanesulfonylindol-1-ylmethyl)pyridin-5-yllpiperidine-1-carboxylate

The title compound was prepared from 4,6-difluoro-5-methanesulfonylindole by the similar manner as described in Example 18 as a pale brown amorphous (12 mg, yield 8%).
FAB-MS (m/z): 505 (M+1)
¹H NMR (CDCl₃ 400 MHz): δ= 1.47 (9H, s), 1.5-1.7 7 (2H, m), 1.7-1.9 (2H, m), 2.6-2.7 (1H, m), 2.7-2.9 (2H, m), 3.29 (3H, s), 4.2-4.3 (2H, m), 5.35 (2H, s), 6.75 (1H, d, J = 3 Hz), 6.81 (1H, d, J = 8 Hz), 6.96 (1H, d, J = 11 Hz), 7.24 (1H, d, J = 3 Hz), 7.44 (1H, dd, J = 2 Hz, 8 Hz), 8.45 (1H, s).

### [Example 58]

### tert-Butyl 4-[3-methyl-2-[5-(1,2,4-triazol-1-yl)indo1-1-ylmethyl]pyridin-5-yl]piperidine-1-carboxylate

The title compound was prepared from tert-butyl 4-[2-(hydroxymethyl)-3-methylpyridin-5-y1]piperidine-1-carboxylate (Example 5(3)) and 5-(1,2,4-triazol-1-yl)indole by the similar manner as described in Example 18 as a pale yellow amorphous.
FAB-MS (m/z): 473 (M+1) ¹H NMR (CDCl₃ 400 MHz): δ= 1.48 (9H, s), 1.5-1.7 (2H, m), 1.7-1.9 (2H, m), 2.22 (3H, s), 2.6-2.7 (1H, m), 2.7-2.9 (2H, m), 4.1-4.4 (2H, m), 5.44 (2H, s), 6.58 (1H, d, J = 3 Hz), 7.22 (1H, d, J = 3 Hz), 7.2-7.3 (1H, m), 7.43 (1H, dd, J = 2 Hz, 9 Hz), 7.51 (1H, d, J = 9 Hz), 7.84 (1H, d, J = 2 Hz), 8.09 (1H, s), 8.3-8.4 (1H, m), 8.49 (1H, s).

### [Example 59]

### Isopropyl 4-[3-methyl-2-[5-(1,2,4-triazol-1-yl)indol-l-ylmethyllpyridin-5-yllpiperidine-1-carboxylate

The title compound was prepared from tert-butyl 4-[3-methyl-2-[5-(1,2,4-triazole-1-yl)indol-l-ylmethyllpyridin-5-yllpiperidine-l-carboxylate (Example 58) by the similar manner as described in Example 54 as a white amorphous.
FAB-MS (m/z): 459 (M+1)
¹H NMR (CDCl₃ 400 MHz): δ= 1.25 (6H, d, J = 6 Hz), 1.5-1.7 7 (2H, m), 1.7-1.9 (2H, m), 2.22 (3H, s), 2.6-2.7 (1H, m), 2.7-2.9 (2H, m), 4.2-4.4 (2H, m), 4.8-5.0 (1H, m), 5.44 (2H, s), 6.58 (1H, d, J = 3 Hz), 7.22 (1H, d, J = 3 Hz), 7.28 (1H, d, J = 2 Hz), 7.43 (1H, dd, J = 2 Hz, 9 Hz), 7.51 (1H, d, J = 9 Hz), 7.84 (1H, d, J = 2 Hz), 8.09 (1H, s), 8.31 (1H, d, J = 2 Hz), 8.49 (1H, s).

### [Example 60]

### tert-Butyl 4-[3-chloro-2-(4,6-difluoro-5-methanesulfonylindol-1-ylmethyl)pyridin-5-yl]-3-methyl-3,6-dihydro-2H-pyridine-1-carboxylate

### (1) tert-Butyl 4-[2-(tert-butyldimethylsilyloxymethyl)-3-chloropyridin-5-yl]-3-methyl-3,6-dihydro-2H-pyridine-1-carboxylate

The title compound was prepared from 5-bromo-2-(tert-butyldimethylsilyloxymethyl)-3-chloropyridine (Example 14 (2)) (260 mg, 0.773 mmol) by the similar manner as described in Example 43 (4) as a white crystal (237 mg, yield 70%).
¹H NMR (CDCl₃ 400 MHz): δ= 0.13 (6H, s), 0.92 (9H, s), 1.02 (3H, d, J = 7 Hz), 1.49 (9H, s), 2.80 (1H, br s), 3.29 (1H, dd, J = 4 Hz, 13 Hz), 3.7-4.0 (1H, m), 3.82 (1H, dd, J = 3 Hz, 13 Hz), 4.1-4.5 (1H, m), 4.90 (2H, s), 5.96 (1H, br s), 7.59 (1H, d, J = 2 Hz), 8.46 (1H, d, J = 2 Hz).

### (2) tert-Butyl 4-(3-chloro-2-hydroxymethylpyridin-5-yl)-3-methyl-3,6-dihydro-2H-pyridine-1-carboxylate

To a solution of tert-butyl 4-[2-(tert-butyldimethylsilyloxymethyl)-3-chloropyridin-5-yl]-3-methyl-3,6-dihydro-2H-pyridine-1-carboxylate (236 mg, 0.538 mmol) in dry tetrahydrofuran (5 mL) was added 1.0 M tetrabutylammonium fluoride-tetrahydrofuran solution (0.81 mL, 0.81 mmol), and the mixture was stirred at room temperature for 15 minutes. The reaction mixture was added ethyl acetate, the organic layer was washed with brine, dried over anhydrous sodium sulfate, and the solvent was removed under reduced pressure. The residue was purified by silica gel column chromatography (hexane/ethyl acetate =2/1 → 3/2), to give the title compound as a white crystal (158 mg, yield 87%).
¹H NMR (CDCl₃ 400 MHz) : δ= 1.03 (3H, d, J = 7 Hz), 1.50 0 9H, s), 2.81 (1H, br s), 3.32 (1H, dd, J = 4 Hz, 13 Hz), 3.7-3.9 (1H, m), 3.85 (1H, dd, J = 3 Hz, 13 Hz), 4.1-4.5 (1H, m), 4.18 (1H, t, J = 5 Hz), 4.79 (2H, d, J = 5 Hz), 5.96 (1H, br s), 7.63 (1H, d, J = 2 Hz), 8.45 (1H, d, J = 2 Hz).

### (3) tert-Butyl 4-[3-chloro-2-(4,6-difluoro-5-methanesulfonylindol-1-ylmethyl)pyridin-5-yl]-3-methyl-3,6-dihydro-2H-pyridine-1-carboxylate

The title compound was prepared from tert-butyl 4-(3-chloro-2-hydroxymethylpyridin-5-yl)-3-methyl-3,6-dihydro-2H-pyridine-l-carboxylate and 4,6-difluoro-5-methanesulfonylindole by the similar manner as described in Example 18 as a pale brown oil.
FAB-MS (m/z): 552 (M+1)
¹H NMR (CDCl₃ 400 MHz) : δ= 1.01 (3H, d, J = 7 Hz), 1.49 (9H, s), 2.77 (1H, br s), 3.2-3.3 (1H, m), 3.29 (3H, s), 3.7-4.0 (1H, m), 3.84 (1H, dd, J = 3 Hz, 13 Hz), 4.1-4.5 (1H, m), 5.48 (2H, s), 5.97 (1H, br s), 6.71 (1H, d, J = 3 Hz), 7.18 (1H, d, J = 11 Hz), 7.32 (1H, d, J = 3 Hz), 7.65 (1H, d, J = 2 Hz), 8.43 (1H, d, J = 2 Hz).

### [Example 61]

### tert-Butyl 4-[3-chloro-2-[5-(1,2,4-triazol-1-yl)indol-l-ylmethyl]pyridin-5-yl]-3-methyl-3,6-dihydro-2H-pyridine-1-carboxylate

The title compound was prepared from tert-butyl 4-(3-chloro-2-hydroxymethylpyridin-5-yl)-3-methyl-3,6-dihydro-2H-pyridine-l-carboxylate (Example 60(2)) and 5-(1,2,4-triazol-1-yl)indole by the similar manner as described in Example 18 as a white amorphous.
FAB-MS (m/z): 505 (M+1)
¹H NMR (CDCl₃ 400 MHz) : δ= 1.00 0 (3H, d, J = 7 Hz), 1.48 8 (9H, s), 2.75 (1H, br s), 3.26 (1H, dd, J = 4 Hz, 13 Hz), 3.7-4.0 0 (1H, m), 3.83 (1H, dd, J = 3 Hz, 13 Hz), 4.1-4.5 (1H, m), 5.57 (2H, s), 5.95 (1H, br s), 6.61 (1H, d, J = 3 Hz), 7.40 (1H, d, J = 3 Hz), 7.45 (1H, dd, J = 2 Hz, 9 Hz), 7.5-7.7 (2H, m), 7.84 (1H, d, J = 2 Hz), 8.09 (1H, s), 8.44 (1H, d, J = 2 Hz), 8.49 (1H, s).

### [Example 62]

### tert-Butyl 4-[2-(4,6-difluoro-5-methanesulfonylindol-1-ylmethyl)pyridin-5-yl]-3-methyl-3,6-dihydro-2H-pyridine-1-carboxylate

### (1) tert-Butyl 4-[2-(hydroxymethyl)pyridin-5-yl]-3-methyl-3,6-dihydro-2H-pyridine-1-carboxylate

The title compound was prepared from 5-bromo-2-hydroxymethylpyridine (198 mg, 1.05 mmol) by the similar manner as described in Example 43 (4) (104 mg, yield 30%).
¹H NMR (CDCl₃ 400 MHz) : δ= 1.01 (3H, d, J =7 Hz), 1.50 (9H, s), 2.83 (1H, br s), 3.33 (1H, dd, J =4 Hz, 13 Hz), 3.7-4.0 (1H, m), 3.84 (1H, dd, J = 3 Hz, 13 Hz), 4.1-4.5 (1H, m), 4.77 (2H, s), 5.91 (1H, br s), 7.23 (1H, d, J = 8 Hz), 7.63 (1H, dd, J = 2 Hz, 8 Hz), 8.54 (1H, br s).

### (2) tert-Butyl 4-[2-(4,6-difluoro-5-methanesulfonylindol-1-ylmethyl)pyridin-5-yl]-3-methyl-3,6-dihydro-2H-pyridine-1-carboxylate

The title compound was prepared from tert-butyl 4-(2-hydroxymethylpyridin-5-yl)-3-methyl-3,6-dihydro-2H-pyridine-1-carboxylate and 4,6-difluoro-5-methanesulfonylindole by the similar manner as described in Example 18 as a brown oil.
FAB-MS (m/z): 518 (M+1)
¹H NMR (CDCl₃ 400 MHz) : δ= 1.00 (3H, d, J = 7 Hz), 1.49 (9H, s), 2.79 (1H, br s), 3.2-3.4 (1H, m), 3.30 (3H, s), 3.7-4.0 (1H, m), 3.83 (1H, dd, J = 3 Hz, 13 Hz), 4.1-4.5 (1H, m), 5.38 (2H, s), 5.91 (1H, br s), 6.75 (1H, d, J = 3 Hz), 6.85 (1H, d, J = 8 Hz), 6.98 (1H, d, J = 11 Hz), 7.27 (1H, d, J = 3 Hz), 7.56 (1H, dd, J = 2 Hz, 8 Hz), 8.57 (1H, d, J = 2 Hz).

### [Example 63]

### tert-Butyl 4-[2-[5-(1,2,4-triazol-1-yl)indol-1-lmethyl]pyridin-5-yl]-3-methyl-3,6-dihydro-2H-pyridine-1-carboxylate

The title compound was prepared from tert-butyl 4-(2-hydroxymethylpyridin-5-yl)-3-methyl-3,6-dihydro-2H-pyridine-1-carboxylate (Example 62 (1)) and 5-(1,2,4-triazol-1-yl)indole by the similar manner as described in Example 18 as a white amorphous.
FAB-MS (m/z): 471 (M+1)
¹H NMR (CDCl₃ 400 MHz): δ= 0.99 (3H, d, J = 7 Hz), 1.49 (9H, s), 2.78 (1H, br s), 3.29 (1H, dd, J = 4 Hz, 13 Hz), 3.7-4.0 (1H, m), 3.82 (1H, dd, J = 3 Hz, 13 Hz), 4.1-4.5 (1H, m), 5.49 (2H, s), 5.89 (1H, br s), 6.67 (1H, d, J = 3 Hz), 6.71 (1H, d, J = 8 Hz), 7.32 (1H, d, J = 3 Hz), 7.38 (1H, d, J = 9 Hz), 7.45 (1H, dd, J = 2 Hz, 9 Hz), 7.49 (1H, dd, J = 2 Hz, 8 Hz), 7.89 (1H, d, J = 2 Hz), 8.10 (1H, s), 8.50 (1H, s), 8.58 (1H, d, J = 2 Hz).

### [Example 64]

### tert-Butyl 4-[2-(7-fluoro-5-nitroindol-1-ylmethyl)pyridin-5-yl]-3-methyl-3,6-dihydro-2H-pyridine-1-carboxylate

The title compound was prepared from tert-butyl 4-(2-hydroxymethylpyridin-5-yl)-3-methyl-3,6-dihydro-2H-pyridine-1-carboxylate (Example 62 (1)) (80 mg, 0.26 mmol) and 7-fluoro-5-nitroindole (43 mg, 0.24 mmol) by the similar manner as described in Example 18 as a pale yellow crystal (91 mg, yield 75%).
¹H NMR (CDCl₃ 400 MHz): δ= 0.99 (3H, d, J =7 Hz), 1.48 (9H, s), 2.7-2.9 (1H, m), 3.28 (1H, dd, J = 3 Hz, 12 Hz), 3.7-4.0 (2H, m), 4.0-4.5 (1H, m), 5.61 (2H, s), 5.8-6.0 (1H, m), 6.79 (1H, dd, J = 2 Hz, 3 Hz), 6.92 (1H, d, J = 8 Hz), 7.38 (1H, d, J = 3 Hz), 7.55 (1H, dd, J = 2 Hz, 8 Hz), 7.79 (1H, dd, J = 2 Hz, 12 Hz), 8.41 (1H, d, J = 2 Hz), 8.55 (1H, d, J = 2 Hz).

### [Example 65]

### Isopropyl 4-[2-(7-fluoro-5-nitroindol-1-ylmethyl)pyridin-5-yl]-3-methyl-3,6-dihydro-2H-pyridine-1-carboxylate

The title compound was prepared from tert-butyl 4-[2-(7-fluoro-5-nitroindol-1-ylmethyl)pyridin-5-yl]-3-methyl-3,6-dihydro-2H-pyridine-1-carboxylate (Example 64) (91 mg, 0.19 mmol) by the similar manner as described in Example 54 as a yellow oil (82 mg, yield 95%).
¹H NMR (CDCl₃ 400 MHz): δ= 0.99 (3H, d, J = 7 Hz), 1.27 (6H, d, J = 6 Hz), 2.7-2.9 (1H, m), 3.2-3.4 (1H, m), 3.7-4.0 0 (1H, m), 3.85 (1H, dd, J = 3 Hz, 12 Hz), 4.1-4.5 (1H, m), 4.9-5.1 (1H, m), 5.62 (2H, s), 5.8-6.0 (1H, m), 6.7-6.8 (1H, m), 6.93 (1H, d, J = 8 Hz), 7.39 (1H, d, J = 3 Hz), 7.55 (1H, dd, J = 3 Hz, 8 Hz), 7.79 (1H, dd, J = 2 Hz, 12 Hz), 8.41 (1H, d, J = 2 Hz), 8.56 (1H, s).

### [Example 66]

### Isopropyl 4-[2-(5-amino-7-fluoroindol-1-ylmethyl)pyridin-5-yl]-3-methyl-3,6-dihydro-2H-pyridine-1-carboxylate

The title compound was prepared from isopropyl 4-[2-(7-fluoro-5-nitroindol-1-ylmethyl)pyridin-5-yl]-3-methyl-3,6-dihydro-2H-pyridine-1-carboxylate (Example 65) (82 mg, 0.18 mmol) by the similar manner as described in Example 37 as a pale brown oil (39 mg, yield 51%).
¹H NMR (CDCl₃ 400 MHz) : δ= 0.98 (3H, d, J = 7 Hz), 1.27 (6H, d, J = 6 Hz), 2.7-2.9 (1H, m), 3.2-3.7 (2H, m), 3.32 (1H, dd, J = 11 Hz), 3.7-4.0 (1H, m), 3.85 (1H, dd, J = 3 Hz, 13 Hz), 4.1-4.5 (1H, m), 4.9-5.1 (1H, m), 5.49 (2H, s), 5.8-6.0 (1H, m), 6.3-6.4 (2H, m), 6.67 (1H, d, J = 2 Hz), 6.73 (1H, d, J = 8 Hz), 7.08 (1H, d, J = 3 Hz), 7.46 (1H, dd, J = 2 Hz, 8 Hz), 8.54 (1H, d, J = 2 Hz).

### [Example 67]

### Isopropyl 4-[2-[7-fluoro-5-(tetrazol-1-yl)indol-1-ylmethyl]pyridin-5-yl]-3-methyl-3,6-dihydro-2H-pyridine-1-carboxylate

The title compound was prepared from isopropyl 4-[2-(5-amino-7-fluoroindol-1-ylmethyl)pyridin-5-yl]-3-methyl-3,6-dihydro-2H-pyridine-1-carboxylate (Example 66) (39 mg, 0.092 mmol) by the similar manner as described in Example 38 as a pale brown amorphous (22 mg, yield 50%)).
FAB-MS (m/z): 475 (M)
¹H NMR (CDCl₃ 400 MHz): δ= 0.99 (3H, d, J = 7 Hz), 1.26 (6H, t, J = 6 Hz), 2.7-2.9 (1H, m), 3.2-3.4 (1H, m), 3.6-4.0 (2H, m), 4.1-4.5 (1H, m), 4.9-5.1 (1H, m), 5.63 (2H, s), 5.8-6.0 (1H, m), 6.71 (1H, d, J = 2 Hz), 6.90 (1H, d, J = 8 Hz), 7.2-7.3 (1H, m), 7.38 (1H, d, J = 2 Hz), 7.54 (1H, dd, J = 2 Hz, 8 Hz), 7.69 (1H, d, J = 2 Hz), 8.56 (1H, s), 8.94 (1H, s).

### [Example 68]

### tert-Butyl (2S, 6S)-2,6-dimethyl-4-[2-(5-methanesulfonylindol-1-ylmethyl)pyridin-5-yl]-3,6-dihydro-2H-pyridine-1-carboxylate

### (1) tert-Butyl (2S, 6S)-2,6-dimethyl-4-(trifluoromethylsulfonyloxy)-3,6-dihydro-2H-pyridine-1-carboxylate

A solution of 1.OM lithium bis(trimethylsilyl)amide-tetrahydrofuran solution (0.5 mL, 0.499 mmol) in dry tetrahydrofuran (1.5 mL) was cooled to -78°C under N₂. To this was added dropwise a solution of tert-butyl (2S, 6S)-2,6-dimethyl-4-oxopiperidine-1-carboxylate (103 mg, 0.453 mmol) in dry tetrahydrofuran (0.4 mL). After stirring at -78°C for 30 minutes, the reaction mixture was added dropwise a solution of N-phenylbis(trifluorometanesulfonimide) (154 mg, 0.453 mmol) in dry tetrahydrofuran (0.4 mL), and then slowly warmed up to 0°C. After stirring at 0°C for 1 hour, the solvent was removed under reduced pressure. The residue was purified by silica gel column chromatography (hexane/acetone =10/1), to give the title compound as a pale yellow oil (120 mg, yield 74%).
¹H NMR (CDCl₃ 400 MHz): δ= 1.23 (3H, d, J = 6 Hz), 1.36 (3H, d, J = 6 Hz), 1.48 (9H, s), 2.17 (1H, dd, J = 2 Hz, 16 Hz), 2.8-2.9 (1H, m), 4.3-4.4 (2H, m), 5.7-5.8 (1H, m).

### (2) tert-Butyl (2S, 6S)-2,6-dimethyl-4-(4,4,5,5-tetramethyl-1,3,2-dioxaborolan-2-yl)-3,6-dihydro-2H-pyridine-1-carboxylate

A suspension of tert-butyl (2S, 6S)-2,6-dimethyl-4-(trifluoromethylsulfonyloxy)-3,6-dihydro-2H-pyridine-1-carboxylate (120 mg, 0.334 mmol), potassium acetate (98 mg, 1.00 mmol), 1,1-bis(diphenylphosphino)ferrocene (5.5 mg, 10.0 µmol), [1,1-bis(diphenylphosphino)ferrocene]palladium(II) dichloride dichloromethane adduct (8.2 mg, 10.0 µmol) and bis(pinacolato)diboron (93 mg, 0.367 mmol) in dioxane (2.2 mL) was stirred at 80°C for 16 hours under N₂. After cooling to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/acetone = 10/1), to give the title compound as a colorless oil (116 mg, yield 100%).
¹H NMR (CDCl₃ 400 MHz): δ= 1.05 (3H, s), 1.26 (3H, d, J = 6 Hz), 1.27 (12H, s), 1.48 (9H, s), 2.18 (1H, dd, J = 2 Hz, 8 Hz), 2.3-2.5 (1H, m), 4.1-4.3 (2H, m), 6.5-6.6 (1H, m).

### (3) tert-Butyl (2S, 6S)-2,6-dimethyl-4-[2-(5-methanesulfonylindol-1-ylmethyl)pyridin-5-yl]-3,6-dihydro-2H-pyridine-1-carboxylate

The title compound was prepared from tert-butyl (2S, 6S)-2,6-dimethyl-4-(4,4,5,5-tetramethyl-1,3,2-dioxaborolan-2-yl)-3,6-dihydro-2H-pyridine-1-carboxylate(30 mg, 89.0 µmol) and 1-(5-bromopyridine-2-ylmethyl)-5-methanesulfonylindole (Example 43 (1)) (30 mg, 80.9 µmol) by the similar manner as described in Example 43 (4) as a white amorphous (18 mg, yield 44%).
FAB-MS (m/z): 496 (M+1)
¹H NMR (CDCl₃ 400 MHz): δ= 1.13 (3H, d, J = 6 Hz), 1.33 (3H, d, J = 6 Hz), 1.49 (9H, s), 2.31 (1H, dd, J = 2 Hz, 16 Hz), 2.7-2.9 (1H, m), 3.07 (3H, s), 4.3-4.5 (2H, m), 5.49 (2H, s), 6.1-6.2 (1H, m), 6.7-6.8 (1H, d, J = 3 Hz), 6.76 (1H, d, J = 8 Hz), 7.38 (1H, d, J = 3 Hz), 7.44 (1H, d, J = 8 Hz), 7.53 (1H, dd, J = 2 Hz, 8 Hz), 7.71 (1H, dd, J = 2 Hz, 8 Hz), 8.30 (1H, d, J = 2 Hz), 8.62 (1H, d, J = 2 Hz).

### [Example 69]

### tert-Butyl (2S, 6S)-2,6-dimethyl-4-[2-(5-methanesulfonylindol-1-ylmethyl)pyridin-5-yl]piperidine-1-carboxylate

The title compound was prepared from tert-butyl (2S, 6S)-2,6-dimethyl-4-[2-(5-methanesulfonylindol-1-ylmethyl)pyridin-5-yl]-3,6-dihydro-2H-pyridine-1-carboxylate (Example 68) (9 mg, 18.16 µmol) by the similar manner as described in Example 8 (2) as a white amorphous (10 mg, yield 100%).
FAB-MS (m/z): 498 (M+1)
¹H NMR (CDCl₃ 400 MHz) : δ= 1.28 8 (3H, d, J = 7 Hz), 1.40 (3H, d, J = 7 Hz), 1.47 (9H, s), 1.5-1.6 (1H, m), 1.6-1.8 (1H, m), 1.8-2.1 (2H, m), 3.0-3.1 (1H, m), 3.07 (3H, s), 3.6-3.8 (1H, m), 4.3-4.5 (1H, m), 5.47 (2H, s), 6.7-6.8 (2H, m), 7.37 (1H, d, J = 3 Hz), 7.42 (1H, dd, J = 2 Hz, 8 Hz), 7.43 (1H, d, J = 8 Hz), 7.70 (1H, dd, J = 2 Hz, 8 Hz), 8.29 (1H, d, J = 2 Hz), 8.47 (1H, d, J = 2 Hz).

### [Example 70]

### tert-Butyl 4-[2-(5-methanesulfonylindol-1-ylmethyl)-3-trifluoromethylpyridin-5-yl]-3-methyl-3,6-dihydro-2H-pyridine-1-carboxylate

### (1) 1-(5-Bromo-3-trifluoromethylpyridin-2-ylmethyl)-5-methanesulfonylindole

The title compound was prepared from 5-methanesulfonylindole (49 mg, 0.250 mmol) and (5-bromo-3-trifluoromethylpyridin-2-yl)methanol (130 mg, 0.508 mmol) by the similar manner as described in Example 18 as a colorless oil (33 mg, yield 30%).
¹H NMR (CDCl₃ 400 MHz): δ= 3.06 6 (3H, s), 5.59 (2H, s), 6.72 (1H, d, J = 3 Hz), 7.35 (1H, d, J = 3 Hz), 7.47 (1H, d, J = 8 Hz), 7.72 (1H, dd, J = 1 Hz, 8 Hz), 8.15 (1H, d, J = 1 Hz), 8.27 (1H, d, J = 1 Hz), 8.68 (1H, d, J = 1 Hz).

### (2) tert-Butyl 4-[2-(5-methanesulfonylindol-1-ylmethyl)-3-trifluoromethylpyridin-5-yl]-3-methyl-3,6-dihydro-2H-pyridine-1-carboxylate

The title compound was prepared from 1-(5-bromo-3-trifluoromethylpyridin-2-ylmethyl)-5-methanesulfonylindole (33 mg, 76.2 mmol) by the similar manner as described in Example 43 (4) as a white amorphous (11 mg, yield 26%).
FAB-MS (m/z): 550 (M+1)
¹H NMR (CDCl₃ 400 MHz) : δ= 1.00 0 (3H, d, J = 7 Hz), 1.48 8 (9H, s), 2.7-2.9 (1H, m), 3.06 (3H, s), 3.28 (1H, dd, J = 3 Hz, 13 Hz), 3.8-4.0 (1H, m), 3.84 (1H, dd, J = 3 Hz, 13 Hz), 4.2-4.5 (1H, m), 5.62 (2H, s), 5.9-6.1 (1H, m), 6.71 (1H, d, J = 3 Hz), 7.38 (1H, d, J = 3 Hz), 7.51 (1H, d, J = 8 Hz), 7.71 (1H, dd, J = 2 Hz, 8 Hz), 7.91 (1H, d, J = 2 Hz), 8.27 (1H, d, J = 2 Hz), 8.61 (1H, d, J = 2 Hz).

### [Example 71]

### tert-Butyl 4-[3-fluoro-2-[5-(methoxycarbonyl)indol-1-ylmethyl)pyridin-5-yl]piperidine-1-carboxylate

The title compound was prepared from tert-butyl 4-[3-fluoro-2-(hydroxymethyl)pyridin-5-yl]piperidine-1-carboxylate (Example 13(2)) (180 mg, 0.548 mmol) and methyl 1H-indole-5-carboxylate (80 mg, 0.457 mmol) by the similar manner as described in Example 18 as a pale brown amorphous (143 mg, yield 56%).
FAB-MS (m/z): 468 (M+1)
¹H NMR (CDCl₃ 400 MHz): δ= 1.47 (9H, s), 1.4-1.6 (2H, m), 1.7-1.9 (2H, m), 2.6-2.9 (3H, m), 3.91 (3H, s), 4.2-4.3 (2H, m), 5.45 (2H, d, J = 2 Hz), 6.61 (1H, d, J = 3 Hz), 7.21 (1H, dd, J = 2 Hz, 10 Hz), 7.35 (1H, d, J = 3 Hz), 7.55 (1H, d, J = 8Hz), 7.90 (1H, dd, J = 1 Hz, 8 Hz), 8.24 (1H, s), 8.36 (1H, d, J = 1 Hz).

### [Example 72]

### tert-Butyl 4-[3-fluoro-2-(5-carboxyindol-1-ylmethyl)pyridin-5-yl]piperidine-1-carboxylate

To a solution of tert-butyl 4-[3-fluoro-2-[5-(methoxycarbonyl)indol-1-ylmethyl)pyridin-5-yl]piperidine-1-carboxylate (Example 71) (133 mg, 0.284 mmol) in methanol (2 mL) - water (1 mL) was added lithium hydroxide monohydrate (24 mg, 0.569 mmol). After refluxing for 7 hours, the reaction mixture was cooled to room temperature, added water and 1M hydrogen chloride, and was extracted with ethyl acetate. The organic layer was washed with brine, dried over anhydrous sodium sulfate, and the solvent was removed under reduced pressure, to give the title compound as a pale yellow amorphous (90 mg, yield 70%).
FAB-MS (m/z): 454 (M+1)
¹H NMR (CDCl₃ 400 MHz): δ= 1.47 (9H, s), 1.5-1.7 7 (2H, m), 1.7-1.9 (2H, m), 2.6-2.9 (3H, m), 4.1-4.4 (2H, m), 5.47 (2H, d, J = 2 Hz), 6.63 (1H, d, J = 3 Hz), 7.22 (1H, dd, J = 2 Hz, 10 Hz), 7.37 (1H, d, J = 3 Hz), 7.58 (1H, d, J = 8 Hz), 7.95 (1H, dd, J = 1 Hz, 8 Hz), 8.26 (1H, s), 8.43 (1H, d, J = 1 Hz).

### [Example 73]

### tert-Butyl 3-ethyl-4-[2-(5-methanesulfonylindol-1-ylmethyl)pyridin-5-yl]-3,6-dihydro-2H-pyridine-1-carboxylate

### (1) tert-Butyl 3-ethyl-4-(trifluoromethylsulfonyloxy)-3,6-dihydro-2H-pyridine-1-carboxylate

Under N₂ atmosphere, a solution of 1.0M lithium bis(trimethylsilyl)amide-tetrahydrofuran solution (4.3 mL, 4.3 mmol) in dry tetrahydrofuran (20 mL) was cooled to -78°C. To this was added dropwise a solution of tert-butyl 3-ethyl-4-oxopiperidine-1-carboxylate (881 mg, 3.88 mmol) in dry tetrahydrofuran (5 mL). After stirring at - 78°C for 30 minutes, the reaction mixture was added dropwise a solution of N-phenylbis(trifluorometanesulfonimide) (1.4 g, 3.88 mmol) in dry tetrahydrofuran (5 mL). The reaction mixture was slowly warmed up to 0°C. After stirring for 1 hour, the solvent was removed under reduced pressure. The residue was purified by silica gel column chromatography (hexane/chloroform = 8/1 → 0/100), to give the title compound as a colorless oil (581 mg, yield 42%).
¹H NMR (CDCl₃ 400 MHz): δ= 1.01 (3H, t, J = 7 Hz), 1.48 (9H, s), 1.3-1.6 (1H, m), 1.6-1.8 (1H, m), 2.2-2.5 (1H, m), 3.2-3.5 (1H, m), 3.7-4.0 (2H, m), 4.0-4.5 (1H, m), 5.74 (1H, br s).

### (2) tert-Butyl 3-ethyl-4-(4,4,5,5-tetramethyl-1,3,2-dioxaborolan-2-yl)-3,6-dihydro-2H-pyridine-1-carboxylate

A suspension of tert-butyl 3-ethyl-4-(trifluoromethylsulfonyloxy)-3,6-dihydro-2H-pyridine-1-carboxylate (581 mg, 1.62 mmol), potassium acetate (477 mg, 4.86 mmol), 1,1-bis(diphenylphosphino)ferrocene (54 mg, 0.097 mmol), [1,1-bis(diphenylphosphino)ferrocene]palladium(II) dichloride dichloromethane adduct (79 mg, 0.097 mmol) and bis(pinacolato)diboron (453 mg, 1.78 mmol) in dioxane (8 mL) was stirred at 80°C for 5 hours under N₂. After cooling to room temperature, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 8/1), to give the title compound as a colorless oil (273 mg, yield 76%).
¹H NMR (CDCl₃ 400 MHz): δ= 0.96 (3H, t, J = 7 Hz), 1.26 (12 H, s), 1.46 (9H, s), 1.1-1.6 (2H, m), 2.1-2.4 (1H, m), 2.8-3.1 (1H, m), 3.5-3.8 (1H, m), 3.8-4.0 (1H, m), 4.0-4.4 (1H, m), 6.3-6.5 (1H, m).

### (3) tert-Butyl 3-ethyl-4-[2-(5-methanesulfonylindol-1-ylmethyl)pyridin-5-yl]-3,6-dihydro-2H-pyridine-1-carboxylate

The title compound was prepared from tert-butyl 3-ethyl-4-(4,4,5,5-tetramethyl-1,3,2-dioxaborolan-2-yl)-3,6-dihydro-2H-pyridine-1-carboxylate(30 mg, 0.089 mmol) and 1-(5-bromopyridin-2-ylmethyl)-5-methanesulfonylindole (Example 43 (1)) (33 mg, 0.089 mmol) by the similar manner as described in Example 43 (4) as a pale yellow amorphous (17 mg, yield 39%).
FAB-MS (m/z): 496 (M+1)
¹H NMR (CDCl₃ 400 MHz): δ= 0.91 (3H, t, J = 7 Hz), 1.2-1.5 (2H, m), 1.48 (9H, s), 2.4-2.5 (1H, m), 3.05 (3H, s), 3.0-3.2 (1H, m), 3.7-3.9 (1H, m), 4.1-4.2 (1H, m), 4.2-4.5 (1H, m), 5.46 (2H, s), 5.91 (1H, br s), 6.72 (1H, d,
J = 3 Hz), 6.77 (1H, d, J = 8 Hz), 7.35 (1H, d, J = 3 Hz), 7.43 (1H, d, J = 8 Hz), 7.49 (1H, dd, J = 2 Hz, 8 Hz), 7.70 (1H, m), 8.28 (1H, s), 8.56 (1H, s).

### [Example 74]

### tert-Butyl 4-[2-(5-methanesulfonylindol-1-ylmethyl)pyridin-5-yl]-3,6-dihydro-2H-pyridine-1-carboxylate

The title compound was prepared from tert-butyl 4-(4,4,5,5-tetramethyl-1,3,2-dioxaborolan-2-yl)-3,6-dihydro-2H-pyridine-l-carboxylate(152 mg, 0.492 mmol) and 1-(5-bromopyridin-2-ylmethyl)-5-methanesulfonylindole (Example 43 (1)) (150 mg, 0.411 mmol) by the similar manner as described in Example 43 (4) as a white amorphous (192 mg, yield 100%).
¹H NMR (CDCl₃ 400 MHz): δ= 1.48 (9H, s), 2.4-2.6 (2H, m), 3.06 (3H, s), 3.5-3.7 7 (2H, m), 4.0-4.2 (2H, m), 5.49 (2H, s), 6.0-6.2 (1H, br s), 6.73 (1H, d, J = 3 Hz), 6.76 (1H, d, J = 8 Hz), 7.38 8 (1H, d, J = 3 Hz), 7.43 (1H, d, J = 9 Hz), 7.53 (1H, dd, J = 2 Hz, 8 Hz), 7.70 (1H, dd, J = 1 Hz, 9 Hz), 8.29 (1H, d, J = 1 Hz), 8.62 (1H, d, J = 2 Hz).

### [Example 75]

### tert-Butyl 4-hydroxy-4-[2-(5-methanesulfonylindol-1-ylmethyl)pyridin-5-yllpiperidine-l-carboxylate

To a solution of tert-butyl 4-[2-(5-methanesulfonylindol-1-ylmethyl)pyridin-5-yl]-3,6-dihydro-2H-pyridine-l-carboxylate (Example 74) (50 mg, 0.107 mmol) in isopropanol (7 mL) - dichloromethane (1.4 mL) was added tris(2,2,6,6-tetramethyl-3,5-heptanedionato)manganese(III) (1.3 mg, 0.00214 mmol), and the mixture was stirred at 0°C for 10 minutes. To this was added phenylsilane (26 µL, 0.214 mmol). After stirring for 3 hours, the reaction mixture was added saturated aqueous sodium thiosulfate solution (4 mL). After stirring at room temperature for 2 hours, the reaction mixture was poured into water, and was extracted with ethyl acetate. The organic layer was washed with brine, dried over anhydrous sodium sulfate, and the solvent was removed under reduced pressure. The residue was purified by silica gel column chromatography (chloroform/methanol = 10/1), to give the title compound as a white amorphous (13 mg, yield 25%).
FAB-MS (m/z): 486 (M+1)
¹H NMR (CDCl₃ 400 MHz): δ= 1.47 (9H, s), 1.6-1.8 (2H, m), 1.78(1H, s), 1.8-2.0 (2H, m), 3.06 (3H, s), 3.1-3.3 (2H, m), 3.9-4.2 (2H, m), 5.49 (2H, s), 6.74 (1H, d, J = 3 Hz), 6.77 (1H, d, J = 8 Hz), 7.37 (1H, d, J = 3 Hz), 7.42 (1H, d, J = 8 Hz), 7.6-7.8 8 (2H, m), 8.29(1H, d, J = 1 Hz), 8.71 (1H d, J = 2 Hz).

### [Example 76]

### tert-Butyl 4-fluoro-4-[2-(5-methanesulfonylindol-1-ylmethyl)pyridin-5-yllpiperidine-l-carboxylate

To a solution of tert-butyl 4-hydroxy-4-[2-(5-methanesulfonylindol-1-ylmethyl)pyridin-5-yllpiperidine-1-carboxylate (Example 75) (33 mg, 0.0680 mmol) in dry dichloromethane (4 mL) was added N,N-diethylaminosulfur trifluoride (9.8 µL, 0.0748 mmol) under N₂. After stirring for 3 hours, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/3), to give the title compound as a white amorphous (17 mg, yield 52%).
FAB-MS (m/z): 488 (M+1)
¹H NMR (CDCl₃ 400 MHz): = 1.48 (9H, s), 1.8-2.1 (4H, m), 3.07 (3H, s), 3.0-3.3 (2H, m), 4.0-4.3 (2H, m), 5.51 (2H, s), 6.75 (1H, d, J = 3 Hz), 6.78 (1H, d, J = 8 Hz), 7.38 (1H, d, J = 3 Hz), 7.43 (1H, d, J = 9 Hz), 7.58 (1H, dd, J = 2 Hz, 8 Hz), 7.71 (1H, dd, J = 1 Hz, 9 Hz), 8.30 (1H, d, J = 2 Hz), 8.61 (1H, d, J = 1 Hz).

### [Example 77]

### tert-Butyl 4-[2-(5-methanesulfonylindol-1-ylmethyl)pyridin-5-yl]-4-methoxypiperidine-1-carboxylate

tert-Butyl 4-hydroxy-4-[2-(5-methanesulfonylindol-1-ylmethyl)pyridin-5-yl]piperidine-1-carboxylate (Example 75) (37 mg, 0.0762 mmol) was dissolved in tetrahydrofuran (4 mL). Under N₂ atmosphere, to the mixture was added 60% sodium hydride (9.2 mg, 0.229 mmol), stirred at room temperature for 30 minutes, then added methyl iodide (7.1 µL, 0.114 mmol), and was stirred overnight. To this was added methyl iodide (7.1 µL, 0.114 mmol) and the mixture was stirred for 6 hours. The reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 1/3), to give the title compound as a white amorphous (10 mg, yield 26%).
FAB-MS (m/z): 500 (M+1)
¹H NMR (CDCl₃ 400 MHz): δ= 1.46 (9H, s), 1.7-1.9 (2H, m), 1.9-2.1 (2H, m), 2.98 (3H, s), 3.07 (3H, s), 3.0-3.3 (2H, m), 3.8-4.2 (2H, m), 5.50 (2H, s), 6.75 (1H, d, J = 3 Hz), 6.78 (1H, d, J = 8 Hz), 7.39 (1H, d, J = 3 Hz), 7.44 (1H, d, J = 8 Hz), 7.58 (1H, dd, J = 2 Hz, 8 Hz), 7.71 (1H, dd, J = 2 Hz, 8 Hz), 8.30 (1H, d, J = 2 Hz), 8.61 (1H, d, J = 2 Hz).

### [Example 78]

### tert-Butyl 4-fluoro-4-[2-(5-methanesulfonylindol-1-ylmethyl)pyridin-5-yl]-3-methylpiperidine-1-carboxylate

The title compound was prepared from tert-butyl 4-[2-(5-methanesulfonylindol-1-ylmethyl)pyridin-5-yl]-3-methyl-3,6-dihydro-2H-pyridine-1-carboxylate (Example 43) (95 mg, 0.197 mmol) by the similar manner as described in Example 75 and Example 76 as a colorless oil (6.7 mg, yield 7%).
FAB-MS (m/z): 502 (M+1)
¹H NMR (CDCl₃ 400 MHz) : δ= 0.73 (3H, d, J = 7 Hz), 1.47 (9H, s), 1.7-1.9 (1H, m), 2.1-2.2 (1H, m), 2.2-2.5 (1H, m), 3.07 (3H, s), 3.0-3.3 (1H, m), 3.3-3.5 (1H, m), 3.8-4.2 (2H, m), 5.51 (2H, s), 6.75 (1H, d, J = 3 Hz), 6.78 (1H, d, J = 8 Hz), 7.37 (1H, d, J = 3 Hz), 7.42 (1H, d, J = 9 Hz), 7.56 (1H, d, J = 8 Hz), 7.72 (1H, dd, J = 1 Hz, 9 Hz), 8.30 (1H, d, J = 1 Hz), 8.59 (1H, br s).

### [Example 79]

### tert-Butyl 4-[3-chloro-2-(5-methanesulfonylindol-l-ylmethyl)pyridin-5-yl]-4-fluoropiperidine-1-carboxylate

### (1) tert-Butyl 4-[2-(tert-butyldimethylsilyloxymethyl)-3-chloropyridin-5-yl]-4-hydroxypiperidine-1-carboxylate

A solution of 5-bromo-2-(tert-butyldimethylsilyloxymethyl)-3-chloropyridine (336 mg, 1.05 mmol) in dry tetrahydrofuran (7 mL) was cooled to - 78°C under N₂. To this was added dropwise 1.6M n-butyllithium/n-hexane solution (0.71 mL, 1.14 mmol). After stirring at -78°C for 1 hour, to this was added dropwise a solution of 1-(tert-butoxycarbonyl)-4-piperidone (189 mg, 0.95 mmol) in dry tetrahydrofuran (3.5 mL). The reaction mixture was warmed up to -50°C, stirred at -50°C for 1 hour, and then warmed up to room temperature. After stirring for 1 hour, the reaction mixture was added saturated aqueous ammonium chloride solution, and was extracted with ethyl acetate. The organic layer was washed with brine, dried over anhydrous sodium sulfate, and the solvent was removed under reduced pressure. The residue was purified by silica gel column chromatography (hexane/ethyl acetate = 2/1), to give the title compound as a colorless oil (336 mg, yield 70%).
¹H NMR (CDCl₃ 400 MHz): δ= 0.13 (6H, s), 0.92 (9H, s), 1.49 (9H, s), 1.7-1.8 (3H, m), 1.9-2.1 (2H, m), 3.1-3.3 (2H, m), 4.0-4.2 (2H, m), 4.90 (2H, s), 7.78 (1H, s), 8.57 (1H, s).

### (2) tert-Butyl 4-[2-(tert-butyldimethylsilyloxymethyl)-3-chloropyridin-5-yl]-4-fluoropiperidine-1-carboxylate

Under N₂ atmosphere, tert-butyl 4-[2-(tert-butyldimethylsilyloxymethyl)-3-chloropyridin-5-yl]-4-hydroxypiperidine-1-carboxylate (336 mg, 0.74 mmol) was dissolved in dry dichloromethane (7 mL), and was added dropwise N,N-diethylaminosulfur trifluoride (0.97 mL, 7.4 mmol) at -78°C. After stirring for 30 minutes, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 5/1), to give the title compound as a colorless oil (230 mg, yield 68%).
¹H NMR (CDCl₃ 400 MHz): δ= 0.13 (6H, s), 0.93 (9H, s), 1.49 (9H, s), 1.8-2.1 (4H, m), 3.1-3.3 (2H, m), 4.0-4.3 (2H, m), 4.91 (2H, s), 7.68 (1H, d, J = 2 Hz), 8.47 (1H, d, J = 2 Hz).

### (3) tert-Butyl 4-[3-chloro-2-(hydroxymethyl)pyridin-5-yl]-4-fluoropiperidine-1-carboxylate

To a solution of tert-butyl 4-[2-(tert-butyldimethylsilyloxymethyl)-3-chloropyridin-5-yl]-4-fluoropiperidine-1-carboxylate (230 mg, 0.50 mmol) in dry tetrahydrofuran (7 mL) was added dropwise 1.0 M tetrabutylammonium fluoride-tetrahydrofuran solution (0.6 mL, 0.60 mmol) under stirring. After stirring for 1 hour, the reaction mixture was poured into water, and was extracted with ethyl acetate. The organic layer was washed with brine, dried over anhydrous sodium sulfate, and the solvent was removed under reduced pressure. The residue was purified by silica gel column chromatography (hexane/ethyl acetate = 4/1), to give the title compound as a colorless oil (140 mg, yield 81%).
¹H NMR (CDCl₃ 400 MHz): δ= 1.50 (9H, s), 1.9-2.1 (4H, m), 3.1-3.3 (2H, m), 4.1-4.3 (2H, m), 4.80 (2H, d, J = 4 Hz), 7.72 (1H, d, J = 2 Hz), 8.48 (1H, d, J = 2 Hz).

### (4) tert-Butyl 4-[3-chloro-2-(5-methanesulfonylindol-1-ylmethyl)pyridin-5-yl]-4-fluoropiperidine-l-carboxylate

The title compound was prepared from tert-butyl 4-[(3-chloro-2-hydroxymethyl)pyridin-5-yl]-4-fluoropiperidine-1-carboxylate (35 mg, 0.102 mmol) by the similar manner as described in Example 18 as a colorless oil (18 mg, yield 34%).
FAB-MS (m/z): 522 (M+1)
¹H NMR (CDCl₃ 400 MHz): δ= 1.48 (9H, s), 1.8-2.0 (4H, m), 3.05 (3H, s), 3.0-3.3 (2H, m), 4.0-4.3 (2H, m), 5.59 (2H, s), 6.69 (1H, d, J = 3 Hz), 7.44 (1H, d, J = 3 Hz), 7.64 (1H, d, J = 9 Hz), 7.6-7.8 (2H, m), 8.25 (1H, d, J = 2 Hz), 8.42 (1H, d, J = 2 Hz).

### [Example 80]

### tert-Butyl 4-[3-fluoro-2-(7-fluoro-5-nitroindol-1-ylmethyl)pyridin-5-yl]piperidine-1-carboxylate

The title compound was prepared from 7-fluoro-5-nitroindole (Example 39 (1)) (50 mg, 0.278 mmol) and tert-butyl 4-[3-fluoro-2-(hydroxymethyl)pyridin-5-yl]piperidine-1-carboxylate (Example 13 (2)) (137 mg, 0.441 mmol) by the similar manner as described in Example 18 as a pale yellow oil (70 mg, yield 53%).
¹H NMR (CDCl₃ 400 MHz): δ= 1.47 (9H, s), 1.5-1.7 (2H, m), 1.7-1.9 (2H, m), 2.6-2.9 (3H, m), 4.1-4.4 (2H, m), 5.68 (2H, s), 6.75 (1H, s), 7.24 (1H, s), 7.36 (1H, d, J = 2 Hz), 7.79 (1H, dd, J = 2 Hz, 8 Hz), 8.20 (1H, s), 8.38 (1H, d, J = 2 Hz).

### [Example 81]

### tert-Butyl 4-[2-(5-amino-7-fluoroindol-1-ylmethyl)-3-fluoropyridin-5-yl]piperidine-1-carboxylate

The title compound was prepared from tert-butyl 4-[3-fluoro-2-(7-fluoro-5-nitroindol-1-ylmethyl)pyridin-5-yl]piperidine-1-carboxylate (Example 80) (70 mg, 0.148 mmol) by the similar manner as described in Example 37 as a yellow oil (36 mg, yield 55%).
¹H NMR (CDCl₃ 400 MHz): δ= 1.47 (9H, s), 1.5-1.7 (2H, m), 1.7-1.9 (2H, m), 2.6-2.9 (3H, m), 3.3-3.6 (2H, br s), 4.1-4.3 (2H, m), 5.55 (2H, d, J = 2 Hz), 6.3-6.4 (2H, m), 6.63 (1H, d, J = 2 Hz), 7.10 (1H, d, J = 2 Hz), 7.19 (1H, dd, J = 2 Hz, 8 Hz), 8.21 (1H, s).

### [Example 82]

### tert-Butyl 4-[3-fluoro-2-[7-fluoro-5-(tetrazol-1-yl)indol-1-ylmethyl]pyridin-5-yl]piperidine-1-carboxylate

The title compound was prepared from tert-butyl 4-[2-(5-amino-7-fluoroindol-1-ylmethyl)-3-fluoropyridin-5-yl]piperidine-1-carboxylate (Example 81) (36 mg, 81.3 µmol) by the similar manner as described in Example 38 as a pale yellow oil (25 mg, yield 63%).
¹H NMR (CDCl₃ 400 MHz): δ= 1.47 (9H, s), 1.5-1.7 (2H, m), 1.7-1.9 (2H, m), 2.6-2.9 (3H, m), 4.1-4.3 (2H, m), 5.69 (2H, s), 6.67 (1H, t, J = 2 Hz), 7.2-7.3 (2H, m), 7.36 (1H, d, J = 2 Hz), 7.66 (1H, d, J = 2 Hz), 8.21 (1H, s), 8.93 1H, s).

### [Example 83]

### Isopropyl 4-[3-fluoro-2-[7-fluoro-5-(tetrazol-1-yl)indol-1-ylmethyl]pyridin-5-yl]piperidine-1-carboxylate

The title compound was prepared from tert-butyl 4-[3-fluoro-2-[7-fluoro-5-(tetrazol-1-yl)indol-1-ylmethyl]pyridin-5-yl]piperidine-1-carboxylate (Example 82) (25 mg, 50.4 µmol) by the similar manner as described in Example 54 as a pale yellow oil (17 mg, yield 71%).
FAB-MS (m/z): 482 (M+1)
¹H NMR (CDCl₃ 400 MHz): δ= 1.25 (6H, d, J = 6 Hz), 1.5-1.7 (2H, m), 1.7-1.9 (2H, m), 2.6-2.8 (1H, m), 2.8-2.9 (2H, m), 4.2-4.4 (2H, m), 4.9-5.0 (1H, m), 5.69 (2H, d, J = 2 Hz), 6.67 (1H, t, J = 2 Hz), 7.2-7.3 (2H, m), 7.36 (1H, d, J = 2 Hz), 7.66 (1H, d, J = 2 Hz), 8.21 (1H, s), 8.93 (1H, s).

### [Example 84]

### tert-Butyl 4-[2-(7-methyl-5-nitroindol-1-ylmethyl]pyridin-5-yl]piperidine-1-carboxylate

The title compound was prepared from 7-methyl-5-nitroindole (68 mg, 0.387 mmol) and tert-butyl 4-[2-(hydroxymethyl)pyridin-5-yl]piperidine-1-carboxylate (Example 1 (2)) (326 mg, 1.12 mmol) by the similar manner as described in Example 18 as a yellow oil (19 mg, yield 11%).
¹H NMR (CDCl₃ 400 MHz): δ= 1.47 (9H, s), 1.5-1.7 (2H, m), 1.7-1.9 (2H, m), 2.57 (3H, s), 2.6-2.7 (1H, m), 2.7-2.9 (2H, m), 4.1-4.4 (2H, m), 5.68 (2H, s), 6.47 (1H, d, J = 8 Hz), 6.76 (1H, d, J = 2 Hz), 7.24 (1H, d, J = 2 Hz), 7.38 (1H, dd, J = 2 Hz, 8 Hz), 7.82 (1H, s), 8.45 (1H, d, J = 2 Hz), 8.46 (1H, d, J = 2 Hz).

### [Example 85]

### tert-Butyl 4-[2-(5-amino-7-methylindol-1-ylmethyl]pyridin-5-yl]piperidine-1-carboxylate

The title compound was prepared from tert-butyl 4-[2-(7-methyl-5-nitroindol-1-ylmethyl]pyridin-5-yl]piperidine-1-carboxylate (Example 84) (45 mg, 0.100 mmol) by the similar manner as described in Example 37 as a pale yellow oil (10 mg, yield 25%).
¹H NMR (CDCl₃ 400 MHz): δ= 1.48 (9H, s), 1.5-1.7 (2H, m), 1.7-1.9 (2H, m), 2.39 (3H, s), 2.6-2.7 (1H, m), 2.7-2.9 (2H, m), 4.1-4.4 (2H, m), 5.58 (2H, s), 6.3-6.4 (3H, m), 6.81 (1H, d, J = 2 Hz), 7.02 (1H, d, J = 2 Hz), 7.32 (1H, dd, J = 2 Hz, 8 Hz), 8.44 (1H, d, J = 2 Hz).

### [Example 86]

### tert-Butyl 4-[2-(7-methyl-5-(tetrazol-1-yl)indol-1-ylmethyl]pyridin-5-yl]piperidine-1-carboxylate

The title compound was prepared from tert-butyl 4-[2-(5-amino-7-methylindol-1-ylmethyl]pyridin-5-yl]piperidine-1-carboxylate (Example 85) (20 mg, 47.5 µmol) by the similar manner as described in Example 38 as a pale yellow oil (8 mg, yield 39%).
FAB-MS (m/z): 474 (M+1)
¹H NMR (CDCl₃ 400 MHz): δ= 1.47 (9H, s), 1.5-1.7 (2H, m), 1.7-1.9 (2H, m), 2.58 (3H, s), 2.6-2.7 (1H, m), 2.7-2.9 (2H, m), 4.1-4.3 (2H, m), 5.70 (2H, s), 6.45 (1H, d, J = 8 Hz), 6.70 (1H, d, J = 2 Hz), 7.19 (1H, s), 7.26 (1H, s), 7.38 (1H, dd, J = 2 Hz, 8 Hz), 7.76 (1H, d, J = 2 Hz), 8.47 (1H, d, J = 2 Hz), 8.93 (1H, s).

### [Example 87]

### Pharmacological experiment 1

### (1) Construction of the stable cell line expressing human GPR119

Human GPR119 gene (NM 178471) was purchased from ATCC (ATCC No. 10807349), and is amplyfied according to PCR to form BamHI site at 5' side and Apa I site at 3' side. The primers were tcctggatccatggaatcatctttctcatt (sequence No. 1) and tcctgggcccttagccatcaaactctgagc (sequence No.2). The PCR conditions are described below.

The double-stranded DNA was thermally denatured using a DNA polymerase (KOD-Plus-Ver. 2; TOYOBO #KOD-211) at 98°C for 10 seconds in one cycle. The denatured single-stranded DNA was annealed with the primers at 55°C for 30 seconds. The DNA was subjected to an extension reaction at 68°C for 1 minute and 15 seconds. The above-mentioned steps were repeated in 35 cycles. The PCR product was inserted into pcDNA5/FRT/TO (Invitrogen #V6520-20) plasmid. Flp-in T-Rex-293 cells (Invitorogen #R78007) were transfected with the obtained plasmid. The method of transfection was conducted in accordance with the protocol of the product.

### (2) Measurement of intracellular cAMP

The stable cell line expressing human GPR119 prepared in the above-mentioned method was plated on a 96-well plate at the concentration of 2,500 cells/well using Dulbecco's Modified Eagle Medium (DMEM) containing 10% fetal bovine serum (FBS). Twenty-four hours after plating, tetracyclin (Invitrogen #Q10019) was added at the final concentration of 20 ng/mL to induce hGPR119 gene expression. Twenty-four hours after, the medium was removed, and the cells were stimulated with an assay buffer (0.5 mM IBMX PBS (-)) containing the test compound at 37°C for 30 minutes. The amount of the intracellular cAMP was measured using a commercially available kit (HitHunter™ cAMP XS+ Assay: GE Healthcare #90007503) and a reader (FLUOstar Optima: BMG LABTECH). The test compound was dissolved in 100% DMSO, and added at the final concentration of 1%.

### (3) Experimental result

Examination results are shown in Table 10.

**TABLE 10**

| Test compound | EC₅₀(nM) |
|---|---|
| Example 1 | 21.2 |
| Example 2 | 20.5 |
| Example 5 | 31.6 |
| Example 9 | 420 |
| Example 10 | 353 |
| Example 11 | 21.4 |
| Example 13 | 36.3 |
| Example 14 | 9.5 |
| Example 16 | 49.1 |
| Example 17 | 30.4 |

As is clear from Table 10, the compounds of example mention showed an excellent GPR119 agonist effect.

### [Example 88]

### Pharmacological experiment 2

The examination was performed by the method similar to Example 87 Pharmacological experiment 1-(l), -(2). Those results are shown in Table 11, 12 and 13.

**TABLE 11**

| Test compound | EC₅₀(nM) |
|---|---|
| Example 18 | 25.6 |
| Example 19 | 181 |
| Example 20 | 18.5 |
| Example 21 | 428 |
| Example 22 | 458 |
| Example 23 | 51.3 |
| Example 24 | 19.0 |
| Example 25 | 20.2 |
| Example 26 | 59.3 |
| Example 27 | 13.9 |
| Example 28 | 82.1 |
| Example 29 | 39.0 |
| Example 30 | 11.7 |
| Example 31 | 41.1 |
| Example 32 | 52.0 |
| Example 33 | 15.5 |
| Example 34 | 30.4 |

**TABLE 12**

| Test compound | EC₅₀(nM) |
|---|---|
| Example 38 | 5.4 |
| Example 41 | 3.7 |
| Example 42 | 22.9 |
| Example 43 | 58.7 |
| Example 44 | 13.5 |
| Example 45 | 186 |
| Example 46 | 111 |
| Example 49 | 297 |
| Example 50 | 375 |
| Example 51 | 115 |
| Example 54 | 9.6 |
| Example 55 | 10.7 |
| Example 56 | 26.2 |
| Example 57 | 17.4 |
| Example 58 | 13.1 |
| Example 59 | 104 |
| Example 62 | 65.2 |

**TABLE 13**

| Test compound | EC₅₀(nM) |
|---|---|
| Example 67 | 45.9 |
| Example 68 | 96.8 |
| Example 69 | 17.5 |
| Example 71 | 191 |
| Example 73 | 32.4 |
| Example 76 | 42.6 |
| Example 83 | 10.4 |

As is clear from Tables 11, 12 and 13, the compounds of example mention showed an excellent GPR119 agonist effect.

### [Example 89]

### Pharmacological experiment 3

### Oral glucose tolerance test in normal mice

### (Experimental procedure)

In this experiment, we examined the inhibitory effect of test compound on glycemic excursions after glucose administration in normal mice. The test methods are shown as follows.

Male 9-week-old ICR mice, habituated to the experimental environment for two weeks, were fasted for 18 hours and used to this experiment. Mice were orally administered the test compound or vehicle (polyethylene glycol 400:ethanol:Tween80 = 8:1:1), and after 30 minutes, they were orally given glucose at the dose of 3 g/kg.

Blood was collected at just before the test compound or vehicle administration (-30 min), immediately before glucose challenge (0 min), 20 min, 40 min, 60 min and 120 min after glucose ingestion and then blood glucose levels were determined.

Inhibition rate (%) of the test compound versus vehicle in areas under the glycemic excursion curve between 0 and 120 min after glucose challenge was determined.

### (Experimental result)

**TABLE 14**

| Test compound (3 mg/kg) | Inhibition rate (%) |
|---|---|
| Example 1 | 37.9 |
| Example 2 | 33.7 |

As is clear from Table 14, the compounds of Example 1 and 2 showed an excellent inhibitory effect of glycemic excursions.

### [Example 90]

### Pharmacological experiment 4

Except for the dose of test compounds made 1 mg/kg, the examination was performed by the method similar to Example 89 oral glucose tolerance test in normal mice. Those results are shown in Table 15.

**TABLE 15**

| Test compound (1 mg/kg) | Inhibition rate (%) |
|---|---|
| Example 24 | 37.9 |
| Example 54 | 36.0 |

As is clear from Table 15, the compounds of Example 24 and 54 showed an excellent inhibitory effect of glycemic excursions.

## Claims

1. A compound having the following formula (I) or a pharmaceutically acceptable salt thereof: 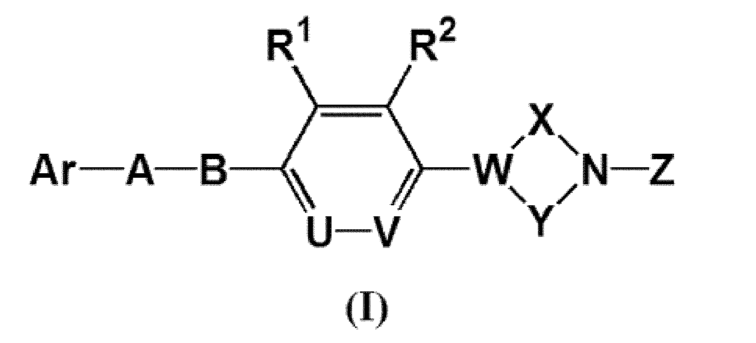 wherein Ar is a bicyclic heterocyclic ring comprising a benzene or pyridine ring condensed with a five-membered or six-membered heterocyclic ring, which optionally has a substituent or substituents selected from the group consisting of a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group having a C₁₋₈ alkoxy group, carboxyl, carbamoyl, an acyl group having a C₁₋₈ alkyl group, an alkylaminocarbonyl group having a C₁₋₈ alkyl group, a dialkylaminocarbonyl group having C₂₋₁₂ alkyl groups, an alkoxycarbonylmethylcarbonyl group having a C₁₋₈ alkoxy group, an alkylsulfonylmethyl group having a C₁₋₈ alkyl group, amino, a C₁₋₈ alkylamino group, a C₂₋₁₂ dialkylamino group, a C₁₋₈ alkylsulfonylamino group, an acylamino group having a C₁₋₈ alkyl group, a C₁₋₈ alkylsulfinyl group, a C₁₋₈ alkylsulfonyl group, sulfamoyl, a C₁₋₈ alkylaminosulfonyl group, a C₂₋₁₂ dialkylaminosulfonyl group, phenylsulfonyl, and a five-membered or six-membered heteroaryl group;
A is (CH₂)ₘ, C(O), S, O, NR³, or a bond, wherein m is an integer of 1 to 3, and R³ is hydrogen or a C₁₋₈ alkyl group;
B is (C(R4)H)n, S, O, CH=CH, NR⁵, or a bond, wherein n is an integer of 1 to 3, and each of R⁴ and R⁵ independently is hydrogen or a C₁₋₈ alkyl group, provided that B is neither S, O, nor NR⁵ when A is S, O, or NR³;
one of U and V is N and the other is CR⁶, or each of U and V is N, wherein R⁶ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
W is C or CR⁷, wherein R⁷ is hydrogen, a halogen atom, hydroxyl, a C₁₋₈ alkyl, a C₁₋₈ alkoxy group, a C₁₋₈ alkyl group having one to three halogen atoms, or a C₁₋₈ alkoxy group having one to three halogen atoms;
X is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms;
when W is C, X may combine to W with a double bond;
Y is a C₁₋₃ alkylene group, which optionally has a substituent or substituents selected from the group consisting of a halogen atom, hydroxyl, a C₁₋₈ alkyl group, a C₁₋₈ alkoxy group, a C₁₋₈ alkyl group having one to three halogen atoms, and a C₁₋₈ alkoxy group having one to three halogen atoms;
Z is C(O)OR⁸, C(O)R⁹, SO₂R¹⁰, C(O)NR¹¹R¹², CH₂C(O)N(R¹³) (R¹⁴), or a five-membered or six-membered heteroaryl group comprising carbon and nitrogen atoms, said carbon atom combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R⁸, R⁹, R¹⁰, R¹¹, R¹², R¹³, and R¹⁴ independently is a C₁₋₈ alkyl group, a C₂₋₈ alkenyl group, a C₃₋₈ cycloalkyl group, phenyl, or a C₁₋₈ alkyl group having phenyl; and
each of R¹ and R² independently is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms.

2. A compound or a pharmaceutically acceptable salt thereof defined in claim 1, wherein Ar is indole, indoline, indazole, pyrrolopyridine, benzofuran, benzothiophene, benzo[b]thiophene-1,1-dioxide, or tetrahydroquinoline, which optionally has a substituent or substituents selected from the group consisting of a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group having a C₁₋₈ alkoxy group, carboxyl, carbamoyl, an acyl group having a C₁₋₈ alkyl group, an alkylaminocarbonyl group having a C₁₋₈ alkyl group, a dialkylaminocarbonyl group having C₂₋₁₂ alkyl groups, an alkoxycarbonylmethylcarbonyl group having a C₁₋₈ alkoxy group, an alkylsulfonylmethyl group having a C₁₋₈ alkyl group, amino, a C₁₋₈ alkylamino group, a C₂₋₁₂ dialkylamino group, a C₁₋₈ alkylsulfonylamino group, an acylamino group having a C₁₋₈ alkyl group, a C₁₋₈ alkylsulfinyl group, a C₁₋₈ alkylsulfonyl group, sulfamoyl, a C₁₋₈ alkylaminosulfonyl group, a C₂₋₁₂ dialkylaminosulfonyl group, phenylsulfonyl, and a five-membered or six-membered heteroaryl group.

3. A compound or a pharmaceutically acceptable salt thereof defined in claim 1, wherein Ar is indole, indoline, indazole, pyrrolopyridine, benzofuran, benzothiophene, benzo[b]thiophene-1,1-dioxide, or tetrahydroquinoline, which has one substituent selected from the group consisting of cyano, an alkoxycarbonyl group having a C₁₋₈ alkoxy group, a C₁₋₈ alkylsulfonyl group, sulfamoyl, phenylsulfonyl, and a five-membered or six-membered heteroaryl group.

4. A compound or a pharmaceutically acceptable salt thereof defined in claim 1, wherein Ar is indole, indoline, indazole, pyrrolopyridine, benzofuran, benzothiophene, benzo[b]thiophene-1,1-dioxide, or tetrahydroquinoline having a substituent or substituents, said substituent being a C₁₋₈ alkylsulfonyl group.

5. A compound or a pharmaceutically acceptable salt thereof defined in claim 1, wherein Ar is indole, indoline, indazole, pyrrolopyridine, benzofuran, benzothiophene, benzo[b]thiophene-1,1-dioxide, or tetrahydroquinoline having two substituents, one of said substituents being a C₁₋₈ alkylsulfonyl group, and the other being a C₁₋₈ alkyl group or a halogen atom.

6. A compound or a pharmaceutically acceptable salt thereof defined in claim 1, wherein Ar is indole, indoline, indazole, pyrrolopyridine, benzofuran, benzothiophene, benzo[b]thiophene-1,1-dioxide, or tetrahydroquinoline having a substituent or substituents, said substituent being 1-tetrazolyl.

7. A compound or a pharmaceutically acceptable salt thereof defined in claim 1, wherein Ar is indole, indoline, indazole, pyrrolopyridine, benzofuran, benzothiophene, benzo[b]thiophene-1,1-dioxide, or tetrahydroquinoline having two substituents, one of said substituents being 1-tetrazolyl, and the other being a C₁₋₈ alkyl group or a halogen atom.

8. A compound or a pharmaceutically acceptable salt thereof defined in one of claims 1 to 7, wherein A is CH₂, and B is a bond.

9. A compound or a pharmaceutically acceptable salt thereof defined in one of claims 1 to 7, wherein A is O, and B is CH₂.

10. A compound or a pharmaceutically acceptable salt thereof defined in one of claims 1 to 9, wherein one of U and V is N, and the other is CH.

11. A compound or a pharmaceutically acceptable salt thereof defined in one of claims 1 to 9, wherein U is N, and V is CH.

12. A compound or a pharmaceutically acceptable salt thereof defined in one of claims 1 to 11, wherein each of X and Y is ethylene.

13. A compound or a pharmaceutically acceptable salt thereof defined in one of claims 1 to 12, wherein Z is C(O)_{OR}⁸_{.}

14. A compound or a pharmaceutically acceptable salt thereof defined in claim 13, wherein R⁸ is a C₁₋₈ alkyl group.

15. A compound or a pharmaceutically acceptable salt thereof defined in claim 13, wherein R⁸ is a C₃₋₅ alkyl group.

16. A compound or a pharmaceutically acceptable salt thereof defined in one of claims 1 to 12, wherein Z is 3-C₁₋₈ alkyl-1,2,4-oxadiazol-5-yl or 5-C₁₋₈ alkyl-1,2,4-oxadiazol-3-yl.

17. A compound or a pharmaceutically acceptable salt thereof defined in one of claims 1 to 12, wherein Z is 5-C₁₋₈ alkylpyrimidin-2-yl.

18. A compound or a pharmaceutically acceptable salt thereof defined in one of claims 1 to 17, wherein each of R¹ and R² is hydrogen.

19. A compound or a pharmaceutically acceptable salt thereof defined in one of claims 1 to 17, wherein one of R¹ and R² is a C₁₋₈ alkyl group, and the other is hydrogen.

20. A compound or a pharmaceutically acceptable salt thereof defined in one of claims 1 to 17, wherein one of R¹ and R² is a halogen atom, and the other is hydrogen.

21. An agent for treating diabetes containing a compound or a pharmaceutically acceptable salt thereof defined in one of claims 1 to 20 as an active ingredient.

22. A GPR119 agonist containing a compound or a pharmaceutically acceptable salt thereof defined in one of claims 1 to 20 as an active ingredient.

23. A compound having the following formula (II) or a pharmaceutically acceptable salt thereof: 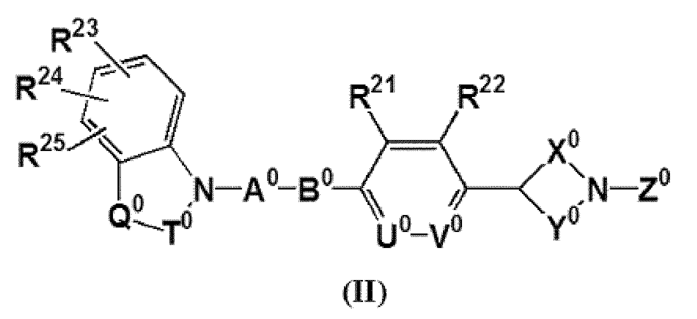 wherein each of R²³, R²⁴, and R²⁵ independently is hydrogen, a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group having a C₁₋₈ alkoxy group, carboxyl, carbamoyl, an acyl group having a C₁₋₈ alkyl group, an alkylaminocarbonyl group having a C₁₋₈ alkyl group, a dialkylaminocarbonyl group having C₂₋₁₂ alkyl groups, an alkoxycarbonylmethylcarbonyl group having a C₁₋₈ alkoxy group, an alkylsulfonylmethyl group having a C₁₋₈ alkyl group, amino, a C₁₋₈ alkylamino group, a C₂₋₁₂ dialkylamino group, a C₁₋₈ alkylsulfonylamino group, an acylamino group having a C₁₋₈ alkyl group, a C₁₋₈ alkylsulfinyl group, a C₁₋₈ alkylsulfonyl group, sulfamoyl, a C₁₋₈ alkylaminosulfonyl group, a C₂-₁₂ dialkylaminosulfonyl group, phenylsulfonyl, or a five-membered or six-membered heteroaryl group;
each of Q⁰ and T⁰ independently is C(R²⁶) (R²⁷), or Q⁰ and T⁰ are combined to form CR²⁸=CR²⁹ or CR³⁰=N, wherein each of R²⁶ and R²⁷ independently is hydrogen or a C₁₋₈ alkyl group, each of R²⁸ and R²⁹ independently is hydrogen or a C₁₋₈ alkyl group, and R³⁰ is hydrogen or a C₁₋₈ alkyl group;
A⁰ is (CH₂)p, C(O), or a bond, wherein p is an integer of 1 to 3;
B⁰ is (C(R³¹)H)_{q}, S, O, CH=CH, NR³², or a bond, wherein q is an integer of 1 to 3, and each of R³¹ and R³² is hydrogen or a C₁₋₈ alkyl group, provided that B⁰ is neither S, O, nor NR³² when A⁰ is CH₂ or a bond;
one of U⁰ and V⁰ is N and the other is CR³³, or each of U⁰ and V⁰ is N, wherein R³³ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
each of X⁰ and Y⁰ independently is a C₁₋₃ alkylene group, which optionally has a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms;
Z⁰ is C(O)OR³⁴, C(O)R³⁵, SO₂R³⁶, C(O)NR³⁷R³⁸, CH₂C (O)N (R³⁹) (R⁴⁰) or a five-membered or six-membered heteroaryl group comprising carbon and nitrogen atoms, said carbon atom combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R³⁴, R³⁵, R³⁶, R³⁷, R³⁸, R³⁹, and R⁴⁰ independently is a C₁₋₈ alkyl group, a C₂₋₈ alkenyl group, a C₃₋₈ cycloalkyl group, phenyl, or a C₁₋₈ alkyl group having phenyl; and
each of R²¹ and R²² independently is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms.

24. A compound or a pharmaceutically acceptable salt thereof defined in claim 23, wherein each of R²³, R²⁴, and R²⁵ independently is hydrogen, a halogen atom, cyano,
a C₁₋₈ alkyl group, an alkoxycarbonyl group having a C₁₋₈ alkoxy group, a C₁₋₈ alkylsulfonyl group, sulfamoyl, phenylsulfonyl, or a five-membered or six-membered heteroaryl group.

25. A compound or a pharmaceutically acceptable salt thereof defined in claim 23, wherein one of R²³, R²⁴, and R²⁵ is a C₁₋₈ alkylsulfonyl group.

26. A compound or a pharmaceutically acceptable salt thereof defined in claim 23, wherein one of R²³, R²⁴, and R²⁵ is a C₁₋₈ alkylsulfonyl group, and another is a C₁₋₈ alkyl group or a halogen atom.

27. A compound or a pharmaceutically acceptable salt thereof defined in claim 23, wherein one of R²³, R²⁴, and R²⁵ is 1-tetrazolyl.

28. A compound or a pharmaceutically acceptable salt thereof defined in claim 23, wherein one of R²³, R²⁴, and R²⁵ is 1-tetrazolyl, and another is a C₁₋₈ alkyl group or a halogen atom.

29. A compound or a pharmaceutically acceptable salt thereof defined in one of claims 23 to 28, wherein Q⁰ and T⁰ are combined to form CH=CH.

30. A compound or a pharmaceutically acceptable salt thereof defined in one of claims 23 to 28, wherein each of Q⁰ and T⁰ is CH₂.

31. A compound or a pharmaceutically acceptable salt thereof defined in one of claims 23 to 30, wherein A⁰ is CH₂, and B⁰ is a bond.

32. A compound or a pharmaceutically acceptable salt thereof defined in one of claims 23 to 31, wherein one of U⁰ and V⁰ is N, and the other is CH.

33. A compound or a pharmaceutically acceptable salt thereof defined in one of claims 23 to 31, wherein U⁰ is N, and V⁰ is CH.

34. A compound or a pharmaceutically acceptable salt thereof defined in one of claims 23 to 33, wherein each of X⁰ and Y⁰ is ethylene.

35. A compound or a pharmaceutically acceptable salt thereof defined in one of claims 23 to 34, wherein Z⁰ is C(O)OR³⁴.

36. A compound or a pharmaceutically acceptable salt thereof defined in claim 35, wherein R³⁴ is a C₁₋₈ alkyl group.

37. A compound or a pharmaceutically acceptable salt thereof defined in claim 35, wherein R³⁴ is a C₃₋₅ alkyl group.

38. A compound or a pharmaceutically acceptable salt thereof defined in one of claims 23 to 34, wherein Z⁰ is 3-C₁₋₈ alkyl-1,2,4-oxadiazol-5-yl or 5-C₁₋₈ alkyl-1,2,4-oxadiazol-3-yl.

39. A compound or a pharmaceutically acceptable salt thereof defined in one of claims 23 to 34, wherein Z⁰ is 5-C₁₋₈ alkylpyrimidin-2-yl.

40. A compound or a pharmaceutically acceptable salt thereof defined in one of claims 23 to 39, wherein each of R²¹ and R²² is hydrogen.

41. A compound or a pharmaceutically acceptable salt thereof defined in one of claims 23 to 39, wherein one of R²¹ and R²² is a C₁₋₈ alkyl group, and the other is hydrogen.

42. A compound or a pharmaceutically acceptable salt thereof defined in one of claims 23 to 39, wherein one of R²¹ and R²² is a halogen atom, and the other is hydrogen.

43. A compound having the following formula (III) or a pharmaceutically acceptable salt thereof: 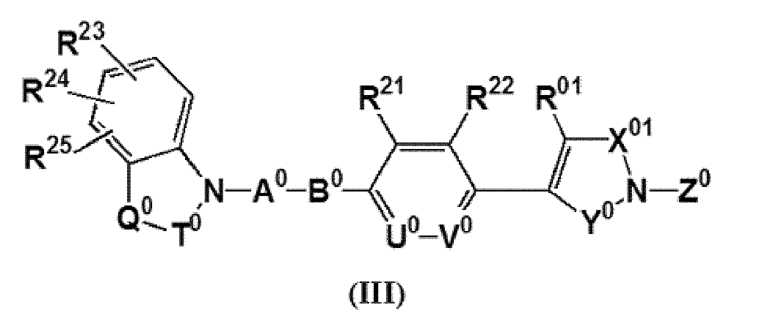 wherein each of R²³, R²⁴, and R²⁵ independently is hydrogen, a halogen atom, nitro, cyano, hydroxyl, a C₁₋₈ alkyl group, a C₁₋₈ alkoxy group, a C₁₋₈ alkyl group having one to three halogen atoms, a C₁₋₈ alkoxy group having one to three halogen atoms, phenoxy, an alkoxycarbonyl group having a C₁₋₈ alkoxy group, carboxyl, carbamoyl, an acyl group having a C₁₋₈ alkyl group, an alkylaminocarbonyl group having a C₁₋₈ alkyl group, a dialkylaminocarbonyl group having C₂₋₁₂ alkyl groups, an alkoxycarbonylmethylcarbonyl group having a C₁₋₈ alkoxy group, an alkylsulfonylmethyl group having a C₁₋₈ alkyl group, amino, a C₁₋₈ alkylamino group, a C₂₋₁₂ dialkylamino group, a C₁₋₈ alkylsulfonylamino group, an acylamino group having a C₁₋₈ alkyl group, a C₁₋₈ alkylsulfinyl group, a C₁₋₈ alkylsulfonyl group, sulfamoyl, a C₁₋₈ alkylaminosulfonyl group, a C₂-₁₂ dialkylaminosulfonyl group, phenylsulfonyl, or a five-membered or six-membered heteroaryl group;
each of Q⁰ and T⁰ independently is C(R²⁶) (R²⁷), or Q⁰ and T⁰ are combined to form CR²⁸=CR²⁹ or CR³⁰=N, wherein each of R²⁶ and R²⁷ independently is hydrogen or a C₁₋₈ alkyl group, each of R²⁸ and R²⁹ independently is hydrogen or a C₁₋₈ alkyl group, and R³⁰ is hydrogen or a C₁₋₈ alkyl group;
A⁰ is (CH₂)ₚ, C(O), or a bond, wherein p is an integer of 1 to 3;
B⁰ is (C(R³¹)H)_{q}, S, O, CH=CH, NR³², or a bond, wherein q is an integer of 1 to 3, and each of R³¹ and R³² is hydrogen or a C₁₋₈ alkyl group, provided that B⁰ is neither S, O, nor NR³² when A⁰ is CH₂ or a bond;
one of U⁰ and V⁰ is N and the other is CR³³, or each of U⁰ and V⁰ is N, wherein R³³ is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms;
X⁰¹ is methylene or ethylene, which optionally has a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms;
R⁰¹ is hydrogen, a C₁₋₈ alkyl group, or a C₁₋₈ alkyl group having one to three halogen atoms;
Y⁰ is a C₁₋₃ alkylene group, which optionally has a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms;
Z⁰ is C(O)OR³⁴, C(O)R³⁵, SO₂R³⁶, C(O)NR³⁷R³⁸, CH₂C(O)N(R³⁹) (R⁴⁰) or a five-membered or six-membered heteroaryl group comprising carbon and nitrogen atoms, said carbon atom combining to the nitrogen atom of the neighboring cyclic amine, and said heteroaryl group optionally having a substituent or substituents selected from the group consisting of a halogen atom, a C₁₋₈ alkyl group, a C₁₋₈ alkoxy group, a C₁₋₈ alkyl group having one to three halogen atoms, and a C₁₋₈ alkoxy group having one to three halogen atoms, wherein each of R³⁴, R³⁵, R³⁶, R³⁷, R³⁸, R³⁹, and R⁴⁰ independently is a C₁₋₈ alkyl group, a C₂-₈ alkenyl group, a C₃₋₈ cycloalkyl group, phenyl, or a C₁₋₈ alkyl group having phenyl; and
each of R²¹ and R²² independently is hydrogen, a halogen atom, hydroxyl, a C₁₋₈ alkyl group, a C₁₋₈ alkoxy group, a C₁₋₈ alkyl group having one to three halogen atoms, or a C₁₋₈ alkoxy group having one to three halogen atoms.

44. An agent for treating diabetes containing a compound or a pharmaceutically acceptable salt thereof defined in one of claims 23 to 43 as an active ingredient.

45. A GPR119 agonist containing a compound or a pharmaceutically acceptable salt thereof defined in one of claims 23 to 43 as an active ingredient.


SEQUENCE LISTING

<110>  Nippon Chemiphar Co., Ltd.

<120>  GPR119 agonist

<130>  NJ-57

<150>  JP 2009-200626
<151>  2009-08-31

<150>  JP 2010-019857
<151>  2010-02-01

<160>  2

<170>  PatentIn version 3.5

<210>  1
<211>  30
<212>  DNA
<213>  Artificial Sequence

<220>
<223>  primer

<400>  1
tcctggatcc atggaatcat ctttctcatt                                        30


<210>  2
<211>  30
<212>  DNA
<213>  Artificial Sequence

<220>
<223>  primer

<400>  2
tcctgggccc ttagccatca aactctgagc                                        30